# EUROPEAN PATENT APPLICATION

(11) **EP 4 840 946 A1**
(43) Date of publication of application: **18.11.2026**
(21) Application number: 25738791.0
(22) Date of filing: 08.01.2025
(51) Int. Cl.: C12N 5/10, A61K 35/17, A61K 38/17, A61P 35/00, C12N 5/0783, C12N 15/12

(54) **T CELLS WITH ENHANCED GLUCOSE UPTAKE ABILITY ON-DEMAND, CELL POPULATION, AND PHARMACEUTICAL COMPOSITION CONTAINING SAME**

(30) Priority: 09.01.2024 JP 2024001533
(71) Applicant: National Cancer Center, Tokyo 104-0045 (JP); Arc Therapies Inc., Tokyo, 160-0018 (JP)
(72) Inventor: NISHIKAWA, Hiroyoshi, Tokyo 104-0045 (JP); WATANABE, Keisuke, Tokyo 104-0045 (JP)
(74) Representative: Engelhard, Markus
(86) International application number: PCT/JP2025/000413
(87) International publication number: WO 2025/150523

(57) **Abstract**

The present disclosure provides cells that are competitive with cancer cells without becoming starved or exhausted even in a glucose-depleted environment. More specifically, the present disclosure provides T cells with enhanced glucose uptake ability, in which the T cells are modified so that expression of a glucose transporter is regulated in the T cells in response to a change in tumor microenvironment conditions, and/or include a nucleic acid sequence encoding a glucose transporter, the nucleic acid sequence being operably linked to a tumor microenvironment-sensing element that senses a change in tumor microenvironment conditions.

## Description

### Technical Field

The present disclosure relates to T cells with enhanced glucose uptake ability on demand, a cell population, and a pharmaceutical composition containing such cells.

### Background Art

Cancer cells reprogram their metabolic systems to favor their own cell proliferation, actively use the glycolytic pathway, which has low efficiency in ATP production, even in the presence of oxygen, and increase glucose uptake and lactic acid production (Warburg effect).

In the tumor microenvironment, many factors that suppress metabolic activity and functions of antitumor T cells are present, and in addition to immunosuppressive factors, metabolic competition between tumors and T cells is involved in formation of an immunosuppressive environment. When T cells receive stimulation through TCR, the intracellular concentration of Ca²⁺ increases, and calcineurin is activated. NFAT, a transcription factor dephosphorylated by activation of calcineurin, translocates into the nucleus, and promotes transcription of IL-2 gene and the like through interaction with other transcription factors, leading to activation.

On the other hand, because cancer cells consume glucose in large amounts, thereby causing glucose depletion, T_{eff}-type tumor-specific T cells, which require glucose as an energy source, have a decreased cellular Ca²⁺ concentration and reduced nuclear translocation of NFAT even when they receive a signal through TCR upon infiltrating into a tumor. Therefore, the T cells become dysfunctional, and cell proliferation and cytokine production are suppressed.

In addition to glucose, when nutrients such as amino acids and fatty acids are also consumed in large amounts by cancer cells, T cells competing for those nutrients become dysfunctional. As described above, in the tumor microenvironment, the antitumor effector action of tumor-specific T cells is inhibited by a metabolic mechanism that supports active proliferation of cancer cells.

Therefore, cells that are competitive with cancer cells without becoming starved or exhausted even under such an environment are desired.

### Summary of Invention

### Solution to Problem

The present disclosure provides T cells with enhanced glucose uptake ability that function without becoming starved or exhausted even in a tumor environment, a cell population containing such cells, and a pharmaceutical composition containing such cells. In particular, in the present disclosure, a representative feature is that the glucose uptake ability is enhanced in response to demand (on demand).

Accordingly, the present disclosure provides the following.
[Item 1] A T cell with enhanced glucose uptake ability, in which the T cell is modified to express a glucose transporter in response to demand (on demand), and/or has enhanced expression of a glucose transporter in response to demand (on demand).
[Item 2] The T cell according to the above item, in which the T cell includes a chimeric antigen receptor (CAR) or a T cell receptor (TCR).
[Item 2A] The T cell according to any one of the above items, in which the T cell includes a chimeric antigen receptor (CAR).
[Item 3] The T cell according to any one of the above items, in which the T cell has an effector function.
[Item 4] The T cell according to any one of the above items, in which the T cell is a precursor cell of an effector T cell (T_{eff}).
[Item 5] The T cell according to any one of the above items, in which the on-demand expression and/or enhancement is achieved specifically in a tumor environment.
[Item 6] The T cell according to any one of the above items, in which the CAR is expressed in the T cell.
[Item 7] The T cell according to any one of the above items, in which the glucose transporter includes GLUT1, GLUT2, GLUT3, and GLUT4.
[Item 8] The T cell according to any one of the above items, in which the glucose transporter is GLUT3.
[Item 9] The T cell according to any one of the above items, in which the T cell is a human T cell.
[Item 10] A cell population containing T cells with enhanced glucose uptake ability in response to demand (on demand).
[Item 11] The cell population according to any one of the above items, in which the T cells include cells having a property of exhibiting an effector function when introduced into a body.
[Item 12] The cell population according to any one of the above items, in which the T cells include a chimeric antigen receptor (CAR) or a T cell receptor (TCR).
[Item 12A] The cell population according to any one of the above items, in which the T cells include a chimeric antigen receptor (CAR).
[Item 13] The cell population according to any one of the above items, in which the T cells are modified to express a glucose transporter in response to demand (on demand), and/or have enhanced expression of a glucose transporter in response to demand (on demand).
[Item 14] The cell population according to any one of the above items, in which the T cells have an effector function.
[Item 15] The cell population according to any one of the above items, in which the cell population contains T_{eff}.
[Item 16] The cell population according to any one of the above items, in which the cell population includes precursor cells of T_{eff}.
[Item 16A] The cell population according to any one of the above items, in which the cell population (or precursor cells of T_{eff}) includes at least one type of cell selected from the group consisting of Tnv, Tscm, Tcm, and Temra.
[Item 17] The cell population according to any one of the above items, in which the CAR is expressed in the T cells.
[Item 18] The cell population according to any one of the above items, in which the glucose transporter includes GLUT1, GLUT2, GLUT3, and GLUT4.
[Item 19] The cell population according to any one of the above items, in which the glucose transporter is GLUT3.
[Item 20] The cell population according to any one of the above items, in which the T cells are human T cells.
[Item 21] A pharmaceutical composition containing the T cell according to any one of the above items or the cell population according to any one of the above items.
[Item 22] The pharmaceutical composition according to any one of the above items, in which the pharmaceutical composition is for treatment or prevention of cancer, an autoimmune disease, allergy, or an infectious disease.
[Item 22A] The pharmaceutical composition according to any one of the above items, in which the pharmaceutical composition is for prevention or treatment of cancer.
[Item 23] The pharmaceutical composition according to any one of the above items, in which the pharmaceutical composition is for curing cancer.
[Item 24] The pharmaceutical composition according to any one of the above items, in which the pharmaceutical composition is for prevention of cancer metastasis or prevention of cancer recurrence.
[Item 25] The pharmaceutical composition according to any one of the above items, in which the pharmaceutical composition is for preventing or treating cancer so that the cancer does not recur.
[Item 26] A pharmaceutical composition for prevention or treatment of a disease in a subject, in which the pharmaceutical composition is characterized by:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item 26A] The pharmaceutical composition according to any one of the above items, in which the T cells include a chimeric antigen receptor (CAR) or a T cell receptor (TCR).
[Item 26B] The pharmaceutical composition according to any one of the above items, in which the T cells include a chimeric antigen receptor (CAR).
[Item 26C] The pharmaceutical composition according to any one of the above items, in which the disease includes cancer, an autoimmune disease, allergy, or an infectious disease.
[Item 26D] The pharmaceutical composition according to any one of the above items, in which the disease includes cancer.
[Item 27] The pharmaceutical composition according to any one of the above items, in which the T cells with enhanced glucose uptake ability include T_{eff} that is not modified to express a glucose transporter in response to demand (on demand) and does not have enhanced expression of a glucose transporter in response to demand (on demand), and cells that are modified to express a glucose transporter in response to demand (on demand) and/or have enhanced expression of a glucose transporter in response to demand (on demand).
[Item 28] The composition according to any one of the above items, in which the glucose transporter is GLUT3.
[Item 29] The T cell or cell population according to any one of the above items, in which the T cell or cell population is for use as a medicament.
[Item 30] The cell or cell population according to any one of the above items, in which the cell or cell population is for treatment or prevention of cancer, an autoimmune disease, allergy, or an infectious disease.
[Item 30A] The cell or cell population according to any one of the above items, in which the cell or cell population is for prevention or treatment of cancer.
[Item 31] The cell or cell population according to any one of the above items, in which the cell or cell population is for curing cancer.
[Item 32] The cell or cell population according to any one of the above items, in which the cell or cell population is for prevention of cancer metastasis or prevention of cancer recurrence.
[Item 33] The cell or cell population according to any one of the above items, in which the cell or cell population is for preventing or treating cancer so that the cancer does not recur.
[Item 34] A cell or cell population for prevention or treatment of a disease in a subject, in which the cell or cell population is characterized by:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item 34A] The cell or cell population according to any one of the above items, in which the T cell includes a chimeric antigen receptor (CAR) or a T cell receptor (TCR).
[Item 34B] The cell or cell population according to any one of the above items, in which the T cell includes a chimeric antigen receptor (CAR).
[Item 34C] The cell or cell population according to any one of the above items, in which the disease includes cancer, an autoimmune disease, allergy, or an infectious disease.
[Item 34D] The cell or cell population according to any one of the above items, in which the disease includes cancer.
[Item 35] The cell or cell population according to any one of the above items, in which the T cells with enhanced glucose uptake ability include T_{eff} that is not modified to express a glucose transporter in response to demand (on demand) and does not have enhanced expression of a glucose transporter in response to demand (on demand), and cells that are modified to express a glucose transporter in response to demand (on demand) and/or have enhanced expression of a glucose transporter in response to demand (on demand).
[Item 36] The cell or cell population according to any one of the above items, in which the glucose transporter is GLUT3.
[Item 37] A method for treatment or prevention in a subject, the method including a step of administering an effective amount of the T cell according to any one of the above items or the cell population according to any one of the above items to the subject in need thereof.
[Item 38] The method according to any one of the above items, in which the treatment or prevention of the subject is for treatment or prevention of cancer, an autoimmune disease, allergy, or an infectious disease.
[Item 38A] The method according to any one of the above items, in which the treatment or prevention of the subject includes prevention or treatment of cancer.
[Item 39] The method according to any one of the above items, in which the treatment or prevention of the subject includes curing cancer.
[Item 40] The method according to any one of the above items, in which the treatment or prevention of the subject includes prevention of cancer metastasis or prevention of cancer recurrence.
[Item 41] The method according to any one of the above items, in which the treatment or prevention of the subject includes preventing or treating cancer so that the cancer does not recur.
[Item 42] A method for prevention or treatment of a disease in a subject, the method including:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item 42A] The method according to any one of the above items, in which the T cells include a chimeric antigen receptor (CAR) or a T cell receptor (TCR).
[Item 42B] The method according to any one of the above items, in which the T cells include a chimeric antigen receptor (CAR).
[Item 42C] The method according to any one of the above items, in which the disease includes cancer, an autoimmune disease, allergy, or an infectious disease.
[Item 42D] The method according to any one of the above items, in which the disease includes cancer.
[Item 43] The method according to any one of the above items, in which the T cells with enhanced glucose uptake ability include T_{eff} that is not modified to express a glucose transporter in response to demand (on demand) and does not have enhanced expression of a glucose transporter in response to demand (on demand), and cells that are modified to express a glucose transporter in response to demand (on demand) and/or have enhanced expression of a glucose transporter in response to demand (on demand).
[Item 44] The method according to any one of the above items, in which the glucose transporter is GLUT3.
[Item 45] Use of the T cell according to any one of the above items or the cell population according to any one of the above items for producing a medicament containing the cell or cell population.
[Item 46] The use according to any one of the above items, in which the medicament is for treatment or prevention of cancer, an autoimmune disease, allergy, or an infectious disease.
[Item 46A] The use according to any one of the above items, in which the medicament is for prevention or treatment of cancer.
[Item 47] The use according to any one of the above items, in which the medicament is for curing cancer.
[Item 48] The use according to any one of the above items, in which the medicament is for prevention of cancer metastasis or prevention of cancer recurrence.
[Item 49] The use according to any one of the above items, in which the medicament is for preventing or treating cancer so that the cancer does not recur.
[Item 50] The use according to any one of the above items, in which the medicament is for prevention or treatment of a disease in a subject, and the medicament is characterized by:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item 50A] The use according to any one of the above items, in which the T cells include a chimeric antigen receptor (CAR) or a T cell receptor (TCR).
[Item 50B] The use according to any one of the above items, in which the T cells include a chimeric antigen receptor (CAR).
[Item 50C] The use according to any one of the above items, in which the disease includes cancer, an autoimmune disease, allergy, or an infectious disease.
[Item 50D] The use according to any one of the above items, in which the disease includes cancer.
[Item 51] The use according to any one of the above items, in which the T cells with enhanced glucose uptake ability include T_{eff} that is not modified to express a glucose transporter in response to demand (on demand) and does not have enhanced expression of a glucose transporter in response to demand (on demand), and cells that are modified to express a glucose transporter in response to demand (on demand) and/or have enhanced expression of a glucose transporter in response to demand (on demand).
[Item 52] The use according to any one of the above items, in which the glucose transporter is GLUT3.

The present disclosure also provides the following.
[Item A1] A T cell with enhanced glucose uptake ability, in which expression of a glucose transporter is regulated in the T cell in response to a change in tumor microenvironment conditions.
[Item A1A] A T cell with enhanced glucose uptake ability, the T cell being modified so that expression of a glucose transporter is regulated in the T cell in response to a change in tumor microenvironment conditions.
[Item A2] The T cell according to any one of the above items, in which the tumor microenvironment conditions include a degree of transmission of a TCR-related signal.
[Item A3] The T cell according to any one of the above Items A, in which the TCR-related signal is mediated by a molecule selected from the group consisting of a TCR signal or a part thereof, and a chimeric signal of a TCR signal and another antigen (also referred to as a CAR signal).
[Item A4] The T cell according to any one of the above items, in which the TCR-related signal is mediated by a molecule including a TCR signal domain.
[Item A5] The T cell according to any one of the above Items A, in which the change in tumor microenvironment conditions includes presentation of a tumor antigen and/or a costimulatory ligand, and/or a change in cytokine conditions.
[Item A6] The T cell according to any one of the above items, in which the degree of transmission of the TCR-related signal includes a degree of TCR stimulation.
[Item A7] The T cell according to any one of the above items, in which the degree of transmission of the TCR-related signal includes at least one selected from the group consisting of a degree of TCR stimulation, TCR co-stimulation, and a humoral factor.
[Item A8] The T cell according to any one of the above items, in which the degree of transmission of the TCR-related signal includes a degree of TCR stimulation, and at least one selected from the group consisting of TCR co-stimulation and a humoral factor.
[Item A9] The T cell according to any one of the above items, in which the degree of transmission of the TCR-related signal includes a degree of TCR stimulation, TCR co-stimulation, and a humoral factor.
[Item A10] The T cell according to any one of the above Items A, in which the TCR stimulation is selected from the group consisting of NFAT, NF-κB, and the STAT transcription factor family, which translocate into the nucleus in association with activation of T cells.
[Item A11] The T cell according to any one of the above items, in which the tumor microenvironment conditions include a degree of oxygen concentration.
[Item A12] A T cell including a nucleic acid sequence encoding a glucose transporter, the nucleic acid sequence being operably linked to a tumor microenvironment-sensing element that senses a change in tumor microenvironment conditions.
[Item A13] The T cell according to any one of the above claims, in which the tumor microenvironment-sensing element includes:
   (i) an inducible activation motif that is activated in response to a change in tumor microenvironment conditions; and
   (ii) a nucleic acid sequence encoding a degradation factor for the glucose transporter that is inactivated in response to the change in the tumor microenvironment conditions.
[Item A14] The T cell according to any one of the above claims, in which the tumor microenvironment-sensing element includes:
   (i) an inducible activation motif to which a nuclear translocation factor that translocates into the nucleus in response to activation of T cells binds;
   (ii) a nuclear translocation factor-binding inducible activation motif to which a nuclear translocation factor that translocates into the nucleus in response to a decrease in oxygen concentration binds; and
   (iii) a nucleic acid sequence encoding a glucose transporter-degrading enzyme whose activity of degrading a glucose transporter is inactivated in response to the decrease in oxygen concentration.
[Item A15] The T cell according to any one of the above items, wherein the nucleic acid sequence encoding the glucose transporter includes a construct operably linked to an inducible activation motif that is activated in response to a change in tumor microenvironment conditions and/or a nucleic acid sequence encoding a degradation factor for the glucose transporter that is inactivated in response to the change in the tumor microenvironment conditions.
[Item A16] The T cell according to any one of the above items, wherein the nucleic acid sequence encoding the glucose transporter includes a construct operably linked to a nuclear translocation factor-binding inducible activation motif to which a nuclear translocation factor that translocates into the nucleus in response to activation of T cells binds.
[Item A17] The T cell according to any one of the above items, in which the nuclear translocation factor is selected from the group consisting of NFAT, NF-κB, and the STAT transcription factor family.
[Item A18] The T cell according to any one of the above items, in which the nuclear translocation factor-binding inducible activation motif is selected from the group consisting of an NFAT-binding domain, a κB motif (GGGACTTTCC) (SEQ ID NO: 1), a STAT-binding sequence (TTCNNNGAA), and modified sequences thereof.
[Item A19] The T cell according to any one of the above items, in which a combination of the nuclear translocation factor and the nuclear translocation factor-binding inducible activation motif includes:
   (1) a combination of NFAT and an NFAT-binding domain;
   (2) a combination of NF-κB and a κB motif (GGGACTTTCC (SEQ ID NO: 1); or
   (3) a combination of the STAT transcription factor family and a STAT-binding sequence (TTCNNNGAA).
[Item A20] The T cell according to any one of the above items, wherein the nucleic acid sequence encoding the glucose transporter includes a construct operably linked to a nuclear translocation factor-binding inducible activation motif to which a nuclear translocation factor that translocates into the nucleus in response to a decrease in oxygen concentration binds.
[Item A21] The T cell according to any one of the above items, in which the nuclear translocation factor is selected from the group consisting of HIF-1.
[Item A22] The T cell according to any one of the above items, in which the nuclear translocation factor-binding inducible activation motif is selected from the group consisting of HIF-RE.
[Item A23] The T cell according to any one of the above items, in which a combination of the nuclear translocation factor and the nuclear translocation factor-binding inducible activation motif is selected from the group consisting of:
   (1) a combination of HIF-1 and HIF-RE.
[Item A24] The T cell according to any one of the above items, wherein the nucleic acid sequence encoding the glucose transporter includes a construct operably linked to a nucleic acid sequence encoding a factor whose activity of degrading the glucose transporter is inactivated in response to a decrease in oxygen concentration.
[Item A25] The T cell according to any one of the above items, in which the factor to be inactivated is selected from the group consisting of an oxygen-dependent degradation factor (ODD).
[Item A26] The T cell according to any one of the above items, in which the T cell includes a chimeric antigen receptor (CAR).
[Item A27] The T cell according to any one of the above items, in which the T cell has an effector function.
[Item A28] The T cell according to any one of the above items, in which the T cell is a precursor cell of an effector T cell (T_{eff}).
[Item A29] The T cell according to any one of the above items, in which memory function of the precursor cell of the effector T cell is prolonged.
[Item A30] The T cell according to any one of the above items, in which a memory T cell phenotype of the precursor cell of the effector T cell is maintained.
[Item A31] The T cell according to any one of the above items, in which the effector T cell is activated or activity of the effector T cell is maintained, and memory function of the precursor cell of the effector T cell is prolonged or a memory T cell phenotype of the precursor cell of the effector T cell is maintained.
[Item A32] The T cell according to any one of the above items, in which the expression and/or enhancement is achieved specifically in a tumor environment.
[Item A33] The T cell according to any one of the above items, in which the CAR is expressed in the T cell.
[Item A34] The T cell according to any one of the above items, in which the glucose transporter includes GLUT1, GLUT2, GLUT3, and GLUT4.
[Item A35] The T cell according to any one of the above items, in which the glucose transporter is GLUT3.
[Item A36] The T cell according to any one of the above items, in which the T cell is a human T cell.
[Item A37] A cell population containing T cells with enhanced glucose uptake ability in response to demand (on demand).
[Item A38] A cell population containing the T cell according to any one of the above items.
[Item A39] The cell population according to any one of the above items, in which the T cells include cells having a property of exhibiting an effector function when introduced into a body.
[Item A40] The cell population according to any one of the above items, in which the T cells include a chimeric antigen receptor (CAR).
[Item A41] The cell population according to any one of the above items, in which the T cells are modified so that expression of a glucose transporter is regulated in the T cells in response to a change in tumor microenvironment conditions, and/or include a nucleic acid sequence encoding a glucose transporter, the nucleic acid sequence being operably linked to a tumor microenvironment-sensing element that senses a change in tumor microenvironment conditions.
[Item A42] The cell population according to any one of the above items, in which the T cells have an effector function.
[Item A43] The cell population according to any one of the above items, in which the cell population contains T_{eff}. [Item A44] The cell population according to any one of the above items, in which the cell population includes precursor cells of T_{eff}.
[Item A45] The cell population according to any one of the above items, in which the CAR is expressed in the T cells. [Item A46] The cell population according to any one of the above items, in which the glucose transporter includes GLUT1, GLUT2, GLUT3, and GLUT4.
[Item A47] The cell population according to any one of the above items, in which the glucose transporter is GLUT3. [Item A48] The cell population according to any one of the above items, in which the T cells are human T cells.
[Item A49] A pharmaceutical composition containing the T cell according to any one of the above items or the cell population according to any one of the above items.
[Item A50] The pharmaceutical composition according to any one of the above items, in which the pharmaceutical composition is for treatment or prevention of cancer, an autoimmune disease, allergy, or an infectious disease.
[Item A51] The pharmaceutical composition according to any one of the above items, in which the pharmaceutical composition is for prevention or treatment of cancer.
[Item A52] The pharmaceutical composition according to any one of the above items, in which the pharmaceutical composition is for curing cancer.
[Item A53] The pharmaceutical composition according to any one of the above items, in which the pharmaceutical composition is for prevention of cancer metastasis or prevention of cancer recurrence.
[Item A54] The pharmaceutical composition according to any one of the above items, in which the pharmaceutical composition is for preventing or treating cancer so that the cancer does not recur.
[Item A55] A pharmaceutical composition for prevention or treatment of a disease in a subject, in which the pharmaceutical composition is characterized by:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item A56] The pharmaceutical composition according to any one of the above items, in which the T cells include a chimeric antigen receptor (CAR) or a T cell receptor (TCR).
[Item A57] The pharmaceutical composition according to any one of the above items, in which the T cells include a chimeric antigen receptor (CAR).
[Item A58] The pharmaceutical composition according to any one of the above items, in which the disease includes cancer, an autoimmune disease, allergy, or an infectious disease.
[Item A59] The pharmaceutical composition according to any one of the above items, in which the disease includes cancer.
[Item A60] The pharmaceutical composition according to any one of the above items, in which the T cells with enhanced glucose uptake ability include T_{eff} that is not modified so that expression of a glucose transporter is regulated in response to a change in tumor microenvironment conditions and is not configured so that expression of a glucose transporter is regulated in response to a change in tumor microenvironment conditions, and cells that are modified so that expression of a glucose transporter is regulated in response to a change in tumor microenvironment conditions and/or are configured so that expression of a glucose transporter is regulated in response to a change in tumor microenvironment conditions.
[Item A60A] The pharmaceutical composition according to any one of the above items, in which the T cells with enhanced glucose uptake ability are not modified to include a nucleic acid sequence encoding a glucose transporter, the nucleic acid sequence being operably linked to a tumor microenvironment-sensing element that senses a change in tumor microenvironment conditions, and include T_{eff} that does not include a nucleic acid sequence encoding a glucose transporter, the nucleic acid sequence being operably linked to a tumor microenvironment-sensing element that senses a change in tumor microenvironment conditions, and cells that are modified to include a nucleic acid sequence encoding a glucose transporter, the nucleic acid sequence being operably linked to a tumor microenvironment-sensing element that senses a change in tumor microenvironment conditions, and/or cells that include a nucleic acid sequence encoding a glucose transporter, the nucleic acid sequence being operably linked to a tumor microenvironment-sensing element that senses a change in tumor microenvironment conditions.
[Item A61] The composition according to any one of the above items, in which the glucose transporter is GLUT3.
[Item A62] The T cell or cell population according to any one of the above items, in which the T cell or cell population is for use as a medicament.
[Item A63] The cell or cell population according to any one of the above items, in which the cell or cell population is for treatment or prevention of cancer, an autoimmune disease, allergy, or an infectious disease.
[Item A64] The cell or cell population according to any one of the above items, in which the cell or cell population is for prevention or treatment of cancer.
[Item A65] The cell or cell population according to any one of the above items, in which the cell or cell population is for curing cancer.
[Item A66] The cell or cell population according to any one of the above items, in which the cell or cell population is for prevention of cancer metastasis or prevention of cancer recurrence.
[Item A67] The cell or cell population according to any one of the above items, in which the cell or cell population is for preventing or treating cancer so that the cancer does not recur.
[Item A68] A cell or cell population for prevention or treatment of a disease in a subject, in which the cell or cell population is characterized by:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item A69] The cell or cell population according to any one of the above items, in which the T cell includes a chimeric antigen receptor (CAR) or a T cell receptor (TCR).
[Item A70] The cell or cell population according to any one of the above items, in which the T cell includes a chimeric antigen receptor (CAR).
[Item A71] The cell or cell population according to any one of the above items, in which the disease includes cancer, an autoimmune disease, allergy, or an infectious disease.
[Item A72] The cell or cell population according to any one of the above items, in which the disease includes cancer.
[Item A73] The cell or cell population according to any one of the above items, in which the T cells with enhanced glucose uptake ability include T_{eff} that is not modified so that expression of a glucose transporter is regulated in response to a change in tumor microenvironment conditions and is not configured so that expression of a glucose transporter is regulated in response to a change in tumor microenvironment conditions, and cells that are modified so that expression of a glucose transporter is regulated in response to a change in tumor microenvironment conditions and/or are configured so that expression of a glucose transporter is regulated in response to a change in tumor microenvironment conditions.
[Item A73A] The cell or cell population according to any one of the above items, in which the T cells with enhanced glucose uptake ability do not include a nucleic acid sequence encoding a glucose transporter, the nucleic acid sequence being operably linked to a tumor microenvironment-sensing element that senses a change in tumor microenvironment conditions, and include T_{eff} that does not include a nucleic acid sequence encoding a glucose transporter, the nucleic acid sequence being operably linked to a tumor microenvironment-sensing element that senses a change in tumor microenvironment conditions, and cells that are modified to include a nucleic acid sequence encoding a glucose transporter, the nucleic acid sequence being operably linked to a tumor microenvironment-sensing element that senses a change in tumor microenvironment conditions, and/or cells that include a nucleic acid sequence encoding a glucose transporter, the nucleic acid sequence being operably linked to a tumor microenvironment-sensing element that senses a change in tumor microenvironment conditions.
[Item A74] The cell or cell population according to any one of the above items, in which the glucose transporter is GLUT3.
[Item A75] A method for treatment or prevention in a subject, the method including a step of administering an effective amount of the T cell according to any one of the above items or the cell population according to any one of the above items to the subject in need thereof.
[Item A76] The method according to any one of the above items, in which the treatment or prevention of the subject is for treatment or prevention of cancer, an autoimmune disease, allergy, or an infectious disease.
[Item A77] The method according to any one of the above items, in which the treatment or prevention of the subject includes prevention or treatment of cancer.
[Item A78] The method according to any one of the above items, in which the treatment or prevention of the subject includes curing cancer.
[Item A79] The method according to any one of the above items, in which the treatment or prevention of the subject includes prevention of cancer metastasis or prevention of cancer recurrence.
[Item A80] The method according to any one of the above items, in which the treatment or prevention of the subject includes preventing or treating cancer so that the cancer does not recur.
[Item A81] A method for prevention or treatment of a disease in a subject, the method including:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item A82] The method according to any one of the above items, in which the T cells include a chimeric antigen receptor (CAR) or a T cell receptor (TCR).
[Item A83] The method according to any one of the above items, in which the T cells include a chimeric antigen receptor (CAR).
[Item A84] The method according to any one of the above items, in which the disease includes cancer, an autoimmune disease, allergy, or an infectious disease.
[Item A85] The method according to any one of the above items, in which the disease includes cancer.
[Item A86] The method according to any one of the above items, in which the T cells with enhanced glucose uptake ability include T_{eff} that is not modified so that expression of a glucose transporter is regulated in response to a change in tumor microenvironment conditions and is not configured so that expression of a glucose transporter is regulated in response to a change in tumor microenvironment conditions, and cells that are modified so that expression of a glucose transporter is regulated in response to a change in tumor microenvironment conditions and/or are configured so that expression of a glucose transporter is regulated in response to a change in tumor microenvironment conditions.
[Item A86A] The method according to any one of the above items, in which the T cells with enhanced glucose uptake ability do not include a nucleic acid sequence encoding a glucose transporter, the nucleic acid sequence being operably linked to a tumor microenvironment-sensing element that senses a change in tumor microenvironment conditions, and include T_{eff} that does not include a nucleic acid sequence encoding a glucose transporter, the nucleic acid sequence being operably linked to a tumor microenvironment-sensing element that senses a change in tumor microenvironment conditions, and cells that are modified to include a nucleic acid sequence encoding a glucose transporter, the nucleic acid sequence being operably linked to a tumor microenvironment-sensing element that senses a change in tumor microenvironment conditions, and/or cells that include a nucleic acid sequence encoding a glucose transporter, the nucleic acid sequence being operably linked to a tumor microenvironment-sensing element that senses a change in tumor microenvironment conditions.
[Item A87] The method according to any one of the above items, in which the glucose transporter is GLUT3.
[Item A88] Use of the T cell according to any one of the above items or the cell population according to any one of the above items for producing a medicament containing the cell or cell population.
[Item A89] The use according to any one of the above items, in which the medicament is for treatment or prevention of cancer, an autoimmune disease, allergy, or an infectious disease.
[Item A90] The use according to any one of the above items, in which the medicament is for prevention or treatment of cancer.
[Item A91] The use according to any one of the above items, in which the medicament is for curing cancer.
[Item A92] The use according to any one of the above items, in which the medicament is for prevention of cancer metastasis or prevention of cancer recurrence.
[Item A93] The use according to any one of the above items, in which the medicament is for preventing or treating cancer so that the cancer does not recur.
[Item A94] The use according to any one of the above items, in which the medicament is for prevention or treatment of a disease in a subject, and the medicament is characterized by:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item A95] The use according to any one of the above items, in which the T cells include a chimeric antigen receptor (CAR) or a T cell receptor (TCR).
[Item A96] The use according to any one of the above items, in which the T cells include a chimeric antigen receptor (CAR).
[Item A97] The use according to any one of the above items, in which the disease includes cancer, an autoimmune disease, allergy, or an infectious disease.
[Item A98] The use according to any one of the above items, in which the disease includes cancer.
[Item A99] The use according to any one of the above items, in which the T cells with enhanced glucose uptake ability include T_{eff} that is not modified so that expression of a glucose transporter is regulated in response to a change in tumor microenvironment conditions and is not configured so that expression of a glucose transporter is regulated in response to a change in tumor microenvironment conditions, and cells that are modified so that expression of a glucose transporter is regulated in response to a change in tumor microenvironment conditions and/or are configured so that expression of a glucose transporter is regulated in response to a change in tumor microenvironment conditions.
[Item A99A] The use according to any one of the above items, in which the T cells with enhanced glucose uptake ability do not include a nucleic acid sequence encoding a glucose transporter, the nucleic acid sequence being operably linked to a tumor microenvironment-sensing element that senses a change in tumor microenvironment conditions, and include T_{eff} that does not include a nucleic acid sequence encoding a glucose transporter, the nucleic acid sequence being operably linked to a tumor microenvironment-sensing element that senses a change in tumor microenvironment conditions, and cells that are modified to include a nucleic acid sequence encoding a glucose transporter, the nucleic acid sequence being operably linked to a tumor microenvironment-sensing element that senses a change in tumor microenvironment conditions, and/or cells that include a nucleic acid sequence encoding a glucose transporter, the nucleic acid sequence being operably linked to a tumor microenvironment-sensing element that senses a change in tumor microenvironment conditions.
[Item A100] The use according to any one of the above items, in which the glucose transporter is GLUT3.

In the present disclosure, it is intended that one or more of the above features can be provided in further combinations in addition to the specified combination. Note that still further embodiments and advantages of the present disclosure will be appreciated by those skilled in the art upon reading and understanding the following detailed description, if necessary.

Note that features and remarkable operations and effects of the present disclosure other than those described above will be apparent to those skilled in the art with reference to the following embodiments and drawings of the invention.

### Advantageous Effects of Invention

According to the present disclosure, effector T cells (T_{eff}) with enhanced glucose uptake ability can be provided, and by using such cells, chimeric antigen receptor (CAR) T cells that function without becoming starved or exhausted even in a tumor environment can be provided.

The cells of the present disclosure can be referred to as metabolism-enhanced T cells, in which a mechanism by which tumor cells survive in a tumor environment is applied to T cells. According to the cells of the present disclosure, effective cytotoxic activity and cytokine production ability under a low-glucose environment such as a tumor microenvironment, which could not be achieved by conventional immune checkpoint inhibitor therapy, can be maintained, and a high antitumor effect in vivo can be exerted. In addition, by using an on-demand glucose transporter expression system in which a glucose transporter is expressed in accordance with an increase in glucose demand, over-differentiation and exhaustion of T cells due to excessive activation can be avoided, the effect can be maintained for a long period, and memory phenotype T cells that also exhibit a significant effect on prevention of recurrence can be induced. Such an on-demand type is achieved by a property in which expression of a glucose transporter is regulated in the T cells in response to a change in tumor microenvironment conditions, or is achieved by including a nucleic acid sequence encoding a glucose transporter, the nucleic acid sequence being operably linked to a tumor microenvironment-sensing element that senses a change in tumor microenvironment conditions.

### Brief Description of Drawings

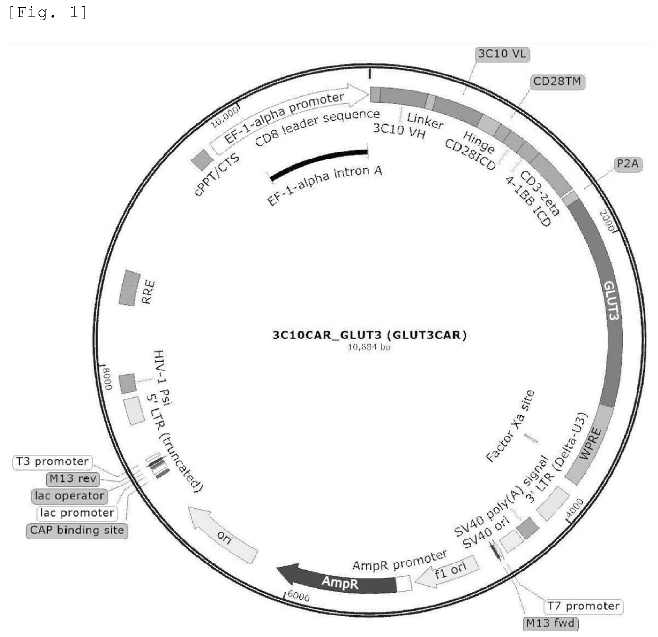

[FIG. 1] FIG. 1 shows an example structure of a metabolically modified anti-EGFRvIII CAR (3C10 CAR_GLUT3) lentiviral vector plasmid. A single-chain antibody (scFv) derived from a mouse-derived antibody (clone 3C10) targeting EGFRvIII expressed in glioblastoma (GBM) and the like was constructed, and the single-chain antibody was linked to CD8 hinge, a CD28 transmembrane portion (CD28TM), a CD28 intracellular domain (CD28ICD), 4-1BBICD, and CD3z to construct a CAR. A high-affinity glucose transporter (GLUT3) was encoded downstream of the CAR via a P2A sequence. The drawing shows an example in which the 3C10 CARGLUT3 was incorporated into a third-generation lentiviral vector plasmid having an EF-1a promoter.

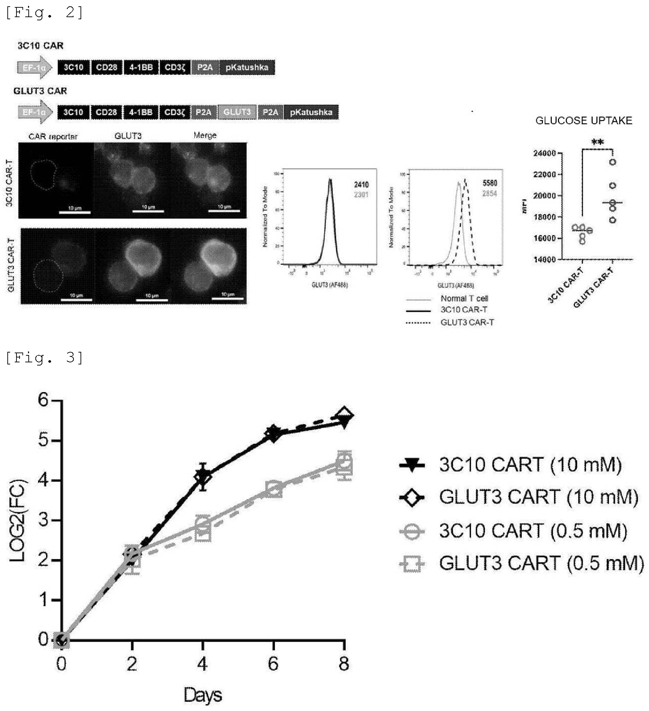

[FIG. 2] FIG. 2 shows T-cell surface GLUT3 expression and enhanced glucose uptake. The upper panel shows structures of a conventional CAR (3C10 CAR) and a metabolically modified CAR (GLUT3 CAR). The lower left shows fluorescence microscopy images of CAR and GLUT3 expression on T cells for 3C10 CAR and GLUT3 CAR. The lower center shows GLUT3 expression quantified by fluorescence intensity. The lower right shows quantification results of the amount of glucose uptake into cells by the 2-NBDG method. A conventional CAR (3C10 CAR) and a metabolically modified CAR (GLUT3 CAR) were introduced into healthy donor-derived T cells by gene transfer to obtain CAR-T cells. Uniform GLUT3 expression was observed on the membrane of GLUT3 CAR-T cells by fluorescence microscopy. In addition, in the GLUT3 CAR-T cells, glucose uptake was enhanced compared with T cells and conventional CAR-T cells (3C10 CAR-T cells).
[FIG. 3] FIG. 3 shows results indicating T-cell expansion efficiency equivalent to that of normal CAR-T cells. The X-axis shows the number of days after introduction, and the Y-axis shows LOG2(FC). Peripheral blood mononuclear cells (PBMCs) containing T cells were stimulated with anti-CD3/28 beads, and then CAR was genetically introduced to analyze T-cell proliferation efficiency. Under both normal glucose conditions (10 mM) and low glucose conditions (0.5 mM), GLUT3 CAR-T cells had establishment efficiency equivalent to that of conventional CAR-T cells (3C10 CAR-T cells).

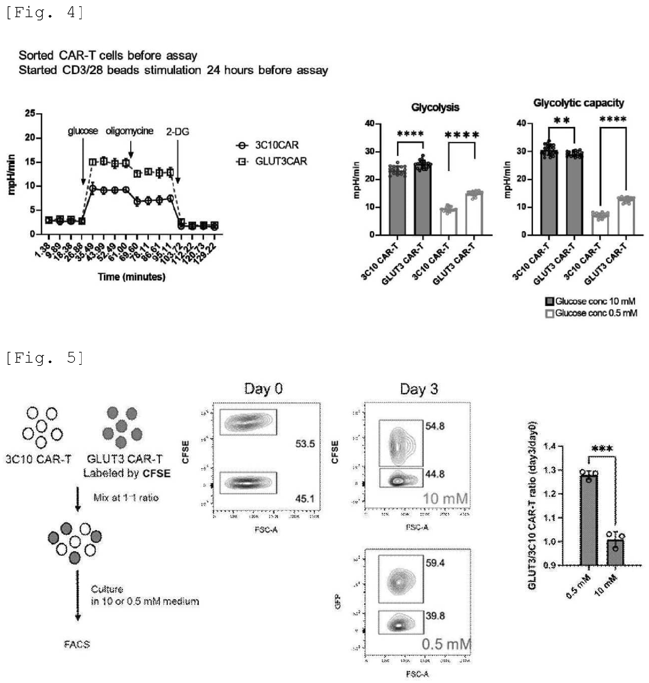

[FIG. 4] FIG. 4 shows results indicating enhancement of T-cell glycolytic ability by GLUT3 expression. The graph on the left shows the glycolytic ability of 3C10 CAR-T cells and GLUT3 CAR-T cells under normal glucose conditions (Glu conc 10 mM) and low glucose conditions (Glu conc 0.5 mM). The lower graph shows the glycolytic ability of 3C10 CAR-T cells and GLUT3 CAR-T cells under normal glucose conditions (Glu conc 10 mM). The left side of the right panel shows values of glycolysis observed after addition of glucose, and the right side shows values of glycolytic capacity observed after addition of Oligomycine. The metabolism of GLUT3 CAR-T cells and 3C10 CAR-T cells was analyzed using a Fluxanalyzer. In both a low-glucose environment and a normal-glucose environment, GLUT3 CAR had enhanced glycolytic ability compared with 3C10 CAR.
[FIG. 5] FIG. 5 shows results indicating that GLUT3 CAR-T cells have a competitive advantage under low glucose conditions. The left panel shows the experimental procedure. The middle panel shows the cell number proportions of 3C10 CAR-T cells and GLUT3 CAR-T cells on day 0 and day 3. The right panel shows the ratio of 3C10 CAR-T cells to GLUT3 CAR-T cells on day 3. 3C10 CAR-T cells (unlabeled) and GLUT3 CAR-T cells (CSFE-labeled) were co-cultured under normal glucose conditions and low glucose conditions. Under normal glucose conditions, survival of both cells was observed to a similar extent, whereas under low glucose conditions, significant survival of GLUT3 CAR-T cells was observed.

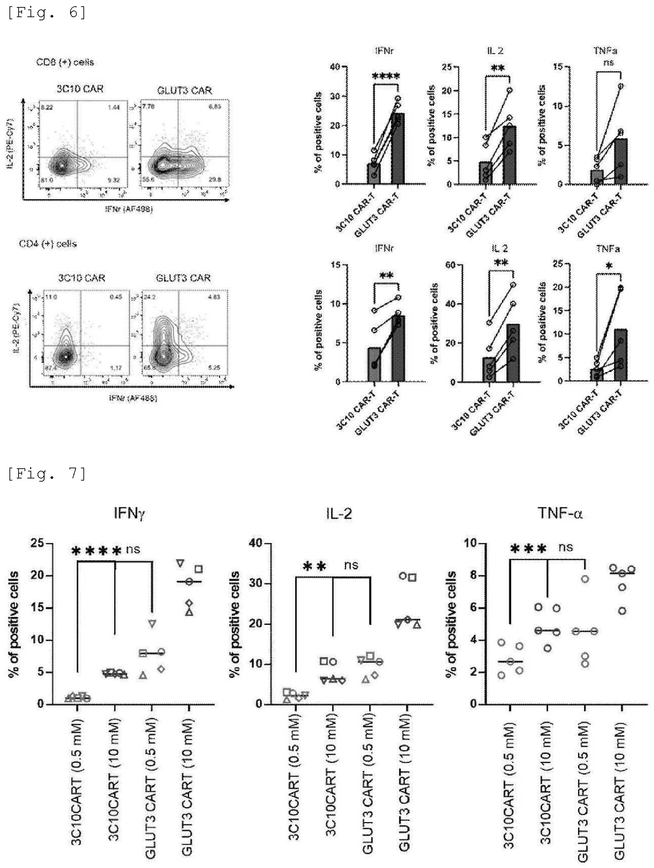

[FIG. 6] FIG. 6 shows results indicating enhanced cytokine production ability of GLUT3 CAR-T cells. The left panel shows intracellular IFNg and IL-2 analysis for CD8-positive cells at the top, and intracellular IFNg and IL-2 amounts for CD4-positive cells at the bottom. The right panel shows the proportions of positive cells for IFNγ, IL-2, and TNF-α. After antigen stimulation of 3C10 CAR-T cells and GLUT3 CAR-T cells, the proportion of cytokine-producing cells was analyzed by FCM. A significant increase in expression of cytokines (IFN-g, IL-2, TNF-a), which are important for T cell maintenance and activation and exertion of antitumor activity, was observed.
[FIG. 7] FIG. 7 shows results indicating the function of GLUT3 CAR-T cells that is maintained even under low glucose conditions. The panels from left to right show the proportions of IFNγ-, IL-2-, and TNF-α-producing cells, respectively, under normal glucose conditions (10 mM) and low glucose conditions (0.5 mM). Cytokine production ability under normal glucose conditions and low glucose conditions was compared. In conventional CAR-T cells (3C10 CAR-T cells), an extreme decrease in function under low glucose conditions was observed. On the other hand, in GLUT3 CAR-T cells, high cytokine production ability was observed under normal glucose conditions, and even under low glucose conditions, GLUT3 CAR-T cells exerted a function comparable to the cytokine production ability of 3C10 CAR-T cells under normal glucose conditions.

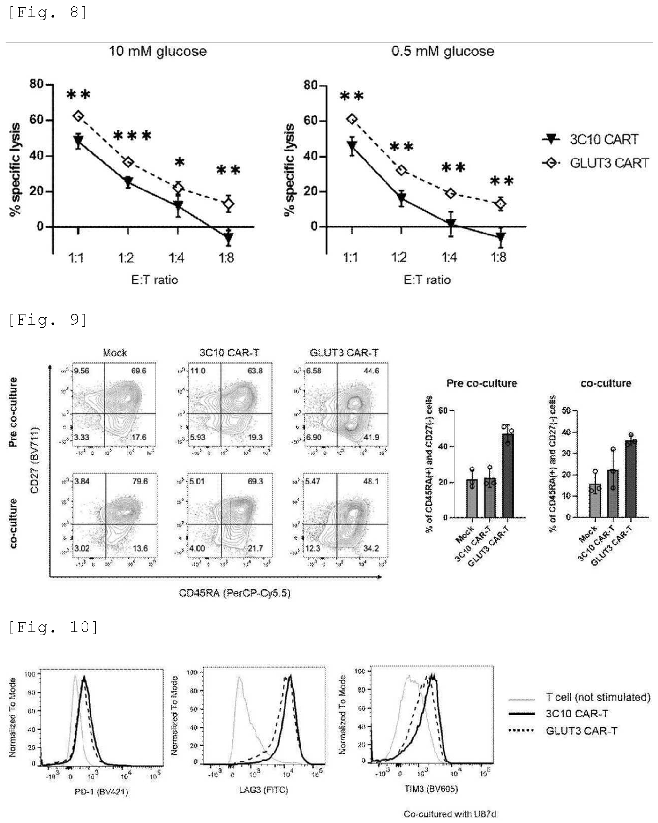

[FIG. 8] FIG. 8 shows results indicating enhanced cytotoxic activity of GLUT3 CAR-T cells. The left panel shows results of cytotoxic activity of 3C10 CAR-T cells and GLUT3 CAR-T cells against the U87d cell line at a glucose concentration of 10 mM, and the right panel shows results of cytotoxic activity of 3C10 CAR-T cells and GLUT3 CAR-T cells against the U87d cell line at a glucose concentration of 0.5 mM. Cytotoxic activity was analyzed using an EGFRvIII-expressing cell line as a target. Cytotoxic activity was analyzed under low glucose conditions and normal glucose conditions, and GLUT3 CAR-T cells showed higher cytotoxic activity than 3C10 CAR-T cells under both environments.
[FIG. 9] FIG. 9 shows results indicating properties imparted by GLUT3 CAR to the T-cell memory phenotype. The left panel shows expression of CD45 and CD27 before co-culture with the U87d tumor cell line (Preco-culture) and after co-culture (co-culture), and the right panel shows, from the left, the proportions of the CD45RA-positive/CD27-negative fraction before co-culture and after co-culture. Memory phenotypes of 3C10 CAR-T cells and GLUT3 CAR-T cells were compared. In GLUT3 CAR-T cells, a stronger tendency toward differentiation into effector T cells was observed compared with 3C10 CAR.
[FIG. 10] FIG. 10 shows suppression of expression of inhibitory molecules in GLUT3 CAR-T cells. Data for PD-1, LAG3, and TIM3 are shown from the left. Expression of inhibitory molecules under stimulation with EGFRvIII antigenpositive cells was compared. In GLUT3 CAR-T cells, suppression of expression of PD-1, LAG3, and TIM3 was observed.

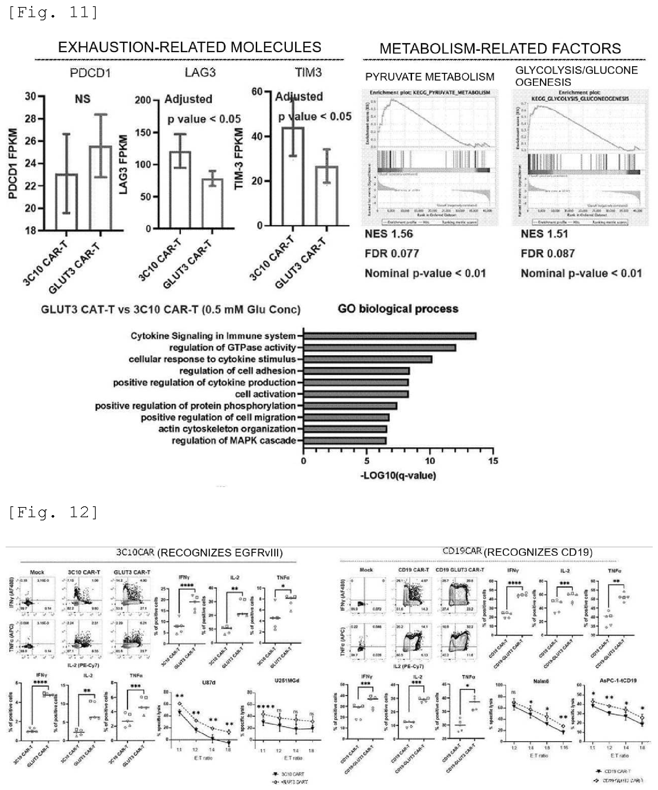

[FIG. 11] FIG. 11 shows mRNA analysis of GLUT3 CAR-T cells (evaluation of metabolism, exhaustion, activation, and differentiation). The upper left panel shows, from the left, PDCD1 expression, LAG3 expression, and TIM3 expression, and the upper right panel shows, from the left, an enrichment plot related to lactic acid metabolism and an enrichment plot related to glucose metabolism. The lower panel shows factors with increased expression based on Gene Ontology. Details of 3C10 CAR-T and GLUT3 CAR-T cells were analyzed based on mRNA expression. In GLUT3 CAR-T cells, as with FCM, a decrease in exhaustion-related molecules and increases in lactate metabolism- and glycolysis-related factors were observed. These factors are factors associated with enhanced T-cell effector functions such as activation and cytokine production.
[FIG. 12] FIG. 12 shows demonstration of universality, that is, that similar effects are obtained not only with 3C10 CAR, but also with CD19 CAR. The left panel shows an experiment using 3C10 CAR, and the right panel shows an experiment using CD19 CAR. In each panel, the upper left shows intracellular IL-2, IFNg, and TNF-a analysis of Mock-introduced T cells, 3C10 CAR-T cells, and GLUT3 CAR-T cells, the upper right shows, from the left, the proportions of IFNg-, IL-2-, and TNFa-positive cells among 3C10 CAR-T cells and GLUT3 CAR-T cells under normal glucose conditions (10 mM), and the lower left shows, from the left, the proportions of IFNg-, IL-2-, and TNFa-positive cells among 3C10 CAR-T cells and GLUT3 CAR-T cells under low glucose conditions (0.5 mM). The lower right shows, from the left, cytotoxic activity of 3C10 CAR-T cells and GLUT3 CAR-T cells against the U87d cell line and the U251MGd cell line under low glucose conditions (0.5 mM). It was confirmed that effects similar to those of 3C10 CAR were also obtained when GLUT3 was incorporated into CD19 CAR. It was also shown to be effective against pancreatic cancer cells obtained by causing CD19 to be expressed in a pancreatic cancer cell line.

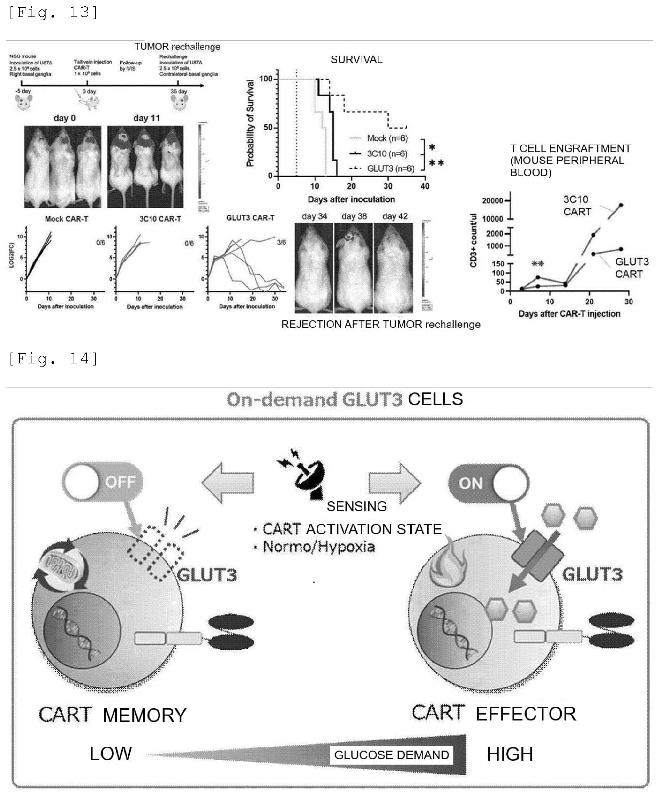

[FIG. 13] FIG. 13 shows that rapid tumor eradication is achieved in an intracranial xenograft model. The upper left panel shows a brief scheme of the experiment. Briefly, this is a model in which 2.5 x 10⁴ cells of the U87d tumor cell line are transplanted into the cranium of NSG mice, which are immunodeficient mice, 1 x 10⁶ CAR-T cells are administered via the tail vein 5 days later, and tumor burden is quantified over time by bioluminescence imaging (BLI). The middle left shows tumor images on day 0 and day 11. The graph at the lower left shows luminescence intensity over time (reflecting tumor burden). The middle-center panel shows Kaplan-Meier curves of each treatment group. Below the middle panel, tumor images on day 34, day 38, and day 42 in tumor rechallenge mice are shown. The right column shows peripheral blood CD3⁺ T-cell counts over time. Antitumor activity was observed in a U87Δ intracranial xenograft model. In the GLUT3 CAR-T cell treatment group, more rapid tumor eradication, improved survival, and superior T-cell expansion were observed compared with the 3C10 CAR-T cell treatment group. In addition, tumor rejection was confirmed by tumor rechallenge in mice cured in the GLUT3 CAR-T cell group.
[FIG. 14] FIG. 14 shows an outline of CAR-T cells with on-demand GLUT3 expression. The drawing schematically shows a system in which, in a steady state, only CAR is expressed and GLUT3 is not expressed, and GLUT3 is expressed when glucose demand increases, such as upon activation of CAR-T cells or in a tumor environment.

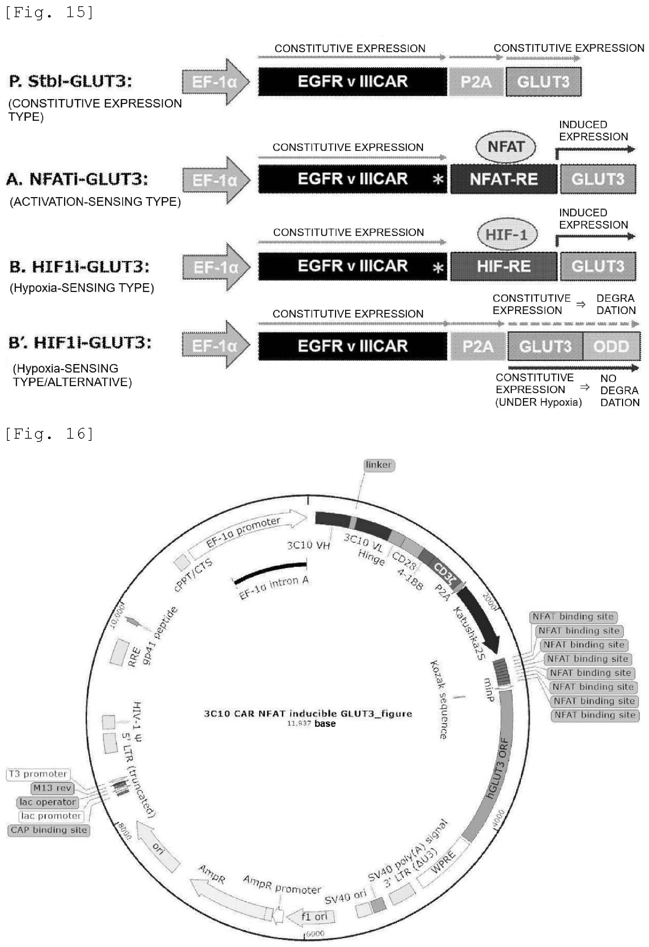

[FIG. 15] FIG. 15 shows a design of an inducible GLUT3 expression system. P indicates a constitutive expression type, A indicates NFATi-GLUT3, B indicates HIF1i-GLUT3, and B' indicates a hypoxia-sensing alternative version of HIF1i-GLUT3. The symbols in the schematic diagram respectively indicate an EF-1 promoter (EF-1a), an anti-EGFRvIII CAR (EGFRvIII CAR=3C10 CAR), a P2A sequence (P2A), an NFAT response element (NFAT-RE), an HIF1a response element (HIF-RE), a high-affinity glucose transporter (GLUT3), an oxygen-dependent degradation domain (ODD), and an asterisk (*) indicates a stop codon. CAR constructs that express GLUT3 specifically in a tumor environment or specifically upon activation were designed. GLUT3 expression in response to an increase in NFAT due to activation (A.) and tumor hypoxia (B. and B') is enabled. CAR constructs that express GLUT3 specifically in a tumor environment or specifically upon activation were designed. GLUT3 expression in response to an increase in NFAT due to activation (A.) and tumor hypoxia (B.) is enabled. In A., independently of CAR (EGFRvIII CAR as an example), which is driven by the EF-1a promoter and constitutively expressed, GLUT3 is encoded via a minimal promoter (MP) under an eight-repeat NFAT-RE to which NFAT, which translocates into the nucleus upon T-cell activation, binds and induces transcription. In B., similarly independently of CAR, GLUT3 is encoded via a minimal promoter under an eight-repeat HIF-RE to which HIF-1a, which translocates into the nucleus in a hypoxic state of T cells, binds and induces transcription. In B', a GLUT3-ODD element in which GLUT3 is degraded in the presence of oxygen is encoded via a P2A sequence immediately downstream of the CAR driven by the EF-1a promoter.
[FIG. 16] FIG. 16 shows a structure when the above NFATi-GLUT3 CAR is incorporated into a lentiviral vector plasmid as an example. Details of the structure are described in the Example section. This is an all-in-one lentiviral vector plasmid in which 3C10 CAR, which is constitutively driven by an EF-1a promoter, and GLUT3, which is driven in an NFAT nuclear translocation-dependent manner by an eight-repeat NFAT binding site and a minimum promoter, are incorporated into one plasmid.

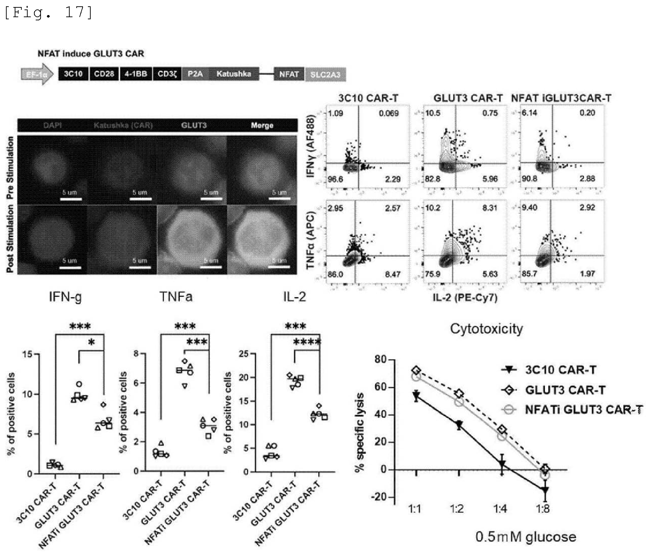

[FIG. 17] FIG. 17 shows the operation and basic functions of an Inducible GLUT3 CAR system. The upper left shows the structure of NFAT-inducible GLUT3 CAR. The middle left panel shows fluorescence microscopy images of CAR and GLUT3 expression in T cells under non-stimulation (Pre stimulation) and under stimulation (Post stimulation). The lower left panel shows, from the right, IFN-γ, TNFα, and IL-2 after stimulation with the U87d cell line. The middle right panel shows intracellular expression intensities of IFN-γ, IL-2, and TNFα in each CAR-T cell under stimulation with the U87d cell line by flow plots. The lower right panel shows a graph indicating that, under glucose conditions (0.5 mM), the cells exhibit cytotoxic activity stronger than that of 3C10 CAR-T cells and equivalent to that of GLUT3 CAR-T cells. A system that expresses GLUT3 in association with NFAT nuclear translocation was constructed (NFATi GLUT3 CAR-T), and its operation was confirmed. The cytotoxic activity of NFATiGLUT3 CAR-T cells (NFATi GLUT3 CAR-T) was equivalent to that of GLUT3 CAR-T cells with constitutive expression (GLUT3 CAR-T), and cytokine production was at an intermediate level between 3C10 CAR-T (without GLUT3) and GLUT3 CAR-T cells with constitutive expression (GLUT3 CAR-T).

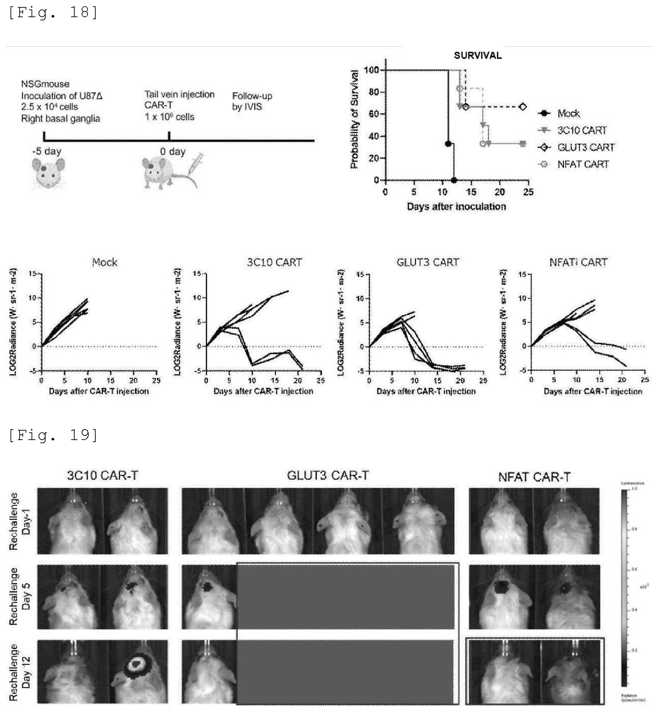

[FIG. 18] FIG. 18 shows evaluation of antitumor effects of Mock T cells, 3C10 CAR-T cells, GLUT3 CAR-T cells, and NFATiGLUT3 CAR-T cells in a mouse model similar to that in FIG. 15. The upper left shows an experimental schematic diagram. The upper right shows a Kaplan-Meier survival curve. The lower panel shows changes over time in tumor burden quantified by bioluminescence imaging (BLI). CR was obtained in four mice treated with GLUT3 CAR-T and in two mice treated with NFATi GLUT3 CAR-t.
[FIG. 19] FIG. 19 shows a tumor challenge and tumor rechallenge experiment (sudden death of mice in the GLUT3 CAR-T cell group). The left panel shows tumor burden measured by bioluminescence imaging indicating survival on day 1 after challenge, day 5 after rechallenge, and day 12 after rechallenge in mice treated with 3C10 CAR-T, GLUT3 CAR-T, and NFAT CAR-T, shown in this order. Portions lacking BLI images indicate death of mice. Tumor (U87d) was re-transplanted into cured mice from the experiment in the previous figure, and rejection ability was evaluated ("surrogate for memory formation"). 1 x 10⁵ tumor cells were re-transplanted, and tumor rejection was not achieved with 3C10 CAR-T, whereas rejection was achieved with GLUT3 CAR-T and NFATiCAR-T. However, in the GLUT3 CAR-T group, three out of four mice died (death unrelated to tumor growth).

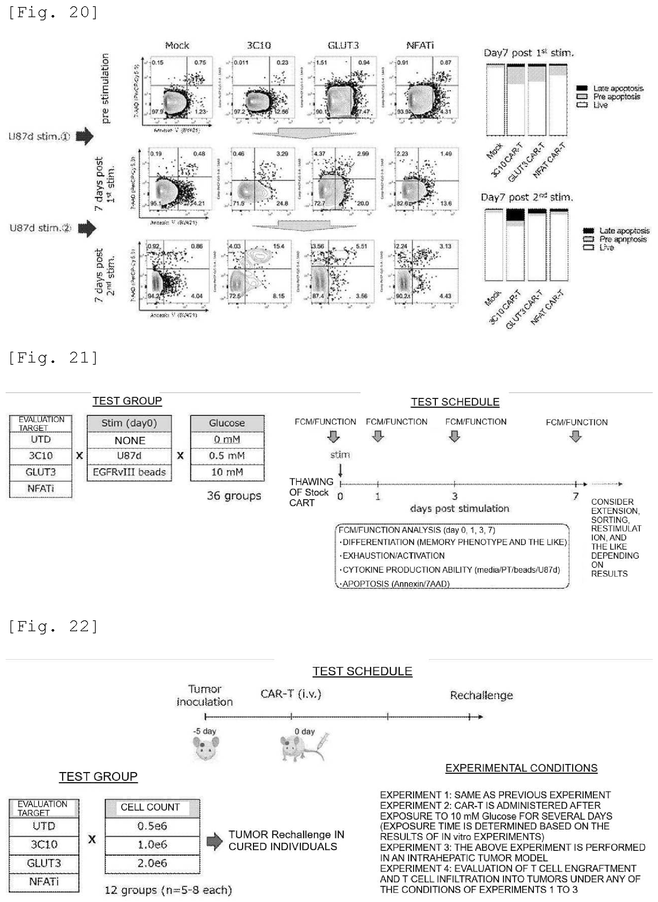

[FIG. 20] FIG. 20 shows suppression of apoptosis after stimulation by NFATiGLUT3. The left panel shows staining for 7-AAD and Annexin V as cell death and apoptosis markers in Mock T cells, 3C10 CAR-T cells, GLUT3 CAR-T cells, and NFATi CAR-T cells in an unstimulated state (upper row of the left panel), 7 days after the first stimulation with the U87d cell line (middle row of the same panel), and 7 days after the second stimulation with the U87d cell line (lower row of the same panel). The graphs in the right panel show, from the top, the proportions of late apoptotic cells, pre-apoptotic cells, and viable cells in each CAR-T cell 7 days after the first stimulation and 7 days after the second stimulation. CAR-T cells were repeatedly stimulated with U87d, and CAR-T cell apoptosis was observed using Annexin V and 7-ADD. GLUT3 has an apoptosis-suppressing effect (considered to be the sum of activation-induced cell death and cell death as a result of terminal differentiation). It is suggested that NFAT CAR-T has a higher apoptosis-suppressing effect than GLUT3 CAR-T.
[FIG. 21] FIG. 21 shows enhancement of long-term survival after stimulation. Each CAR-T cell is observed over time under the above conditions. This drawing shows an in vitro (CAR) T-cell stimulation and culture test plan. Untransduced T cells (UTD), 3C10 CAR-T cells, GLUT3 CAR-T cells, and NFATi CAR-T cells are used as evaluation targets, and an unstimulated group and stimulated groups (stimulation with the U87d cell line or stimulation with EGFRvIII magnetic beads) are provided. Furthermore, each group is cultured under glucose-free conditions (0 mM), low glucose conditions (0.5 mM), and normal glucose conditions (10 mM) (36 groups in total). For each group, the analyses shown in the schedule on the right are performed over time. Under low glucose, conventional CAR-T (3C10) does not exhibit effective cytokine production, whereas GLUT3 CAR-T and NFATi GLUT3 CAR-T exhibit effective cytokine production ability. However, due to excessive glucose uptake, GLUT3 CAR-T differentiates over time (day 3 or day 7) into CCR7-negative Tem or Temra, undergoes terminal differentiation, loses stemness, and undergoes apoptosis. On the other hand, in NFATi GLUT3 CAR-T cells, which are on-demand-type cells, apoptosis is suppressed, and CCR7-positive Tcm and Tscm (memory cells, cells having stemness) are maintained. When CAR-T cells (established under normal glucose conditions) are suddenly (day 0) placed under low-glucose conditions of 0 mM or 0.5 mM, GLUT3 CAR-T cells, which are highly glucose-dependent, are prone to apoptosis (⇔ NFATi GLUT-3 CAR-T cells show resistance to low glucose).
[FIG. 22] FIG. 22 shows enhancement of long-term survival after stimulation. The above antitumor effect, survival, and adverse events are examined in a system similar to that of the mice described above. This drawing shows enhancement of long-term survival in vivo. The above antitumor effect, survival, and adverse events are examined in a system similar to that of the mice described above. The U87d tumor cell line is transplanted intracranially into the cranium of NSG mice, which are immunodeficient mice, and 5 days later, Mock-introduced T cells (UTD), 3C10 CAR-T cells, GLUT3 CAR-T cells, or NFATiCAR-T cells are administered via the tail vein, and quantification of antitumor effects by bioluminescence imaging and survival are analyzed. In Experiment 1, GLUT3 CAR-T and NFATi GLUT3 CAR-T show equivalent antitumor activity. In Experiment 2, apoptosis and over-differentiation occur due to GLUT3 CAR-T overactivation caused by glucose exposure, and antitumor activity is lost. On the other hand, in NFATiCAR-T cells, excessive activation is suppressed, and therefore an effective antitumor effect is exerted. Also in rechallenge, NFATiCAR-T cells reject tumors. In Experiment 4, in proportion to the tumor effect, superior CAR-T cell engraftment and T-cell infiltration into tumors are observed in NFATiGLUT3. Experiment 3 is an exploratory experiment in a liver tumor model suggested to exhibit immunosuppression due to a low-glucose, high-lactate environment, conducted with the expectation that results similar to those in Experiment 2 would be obtained.

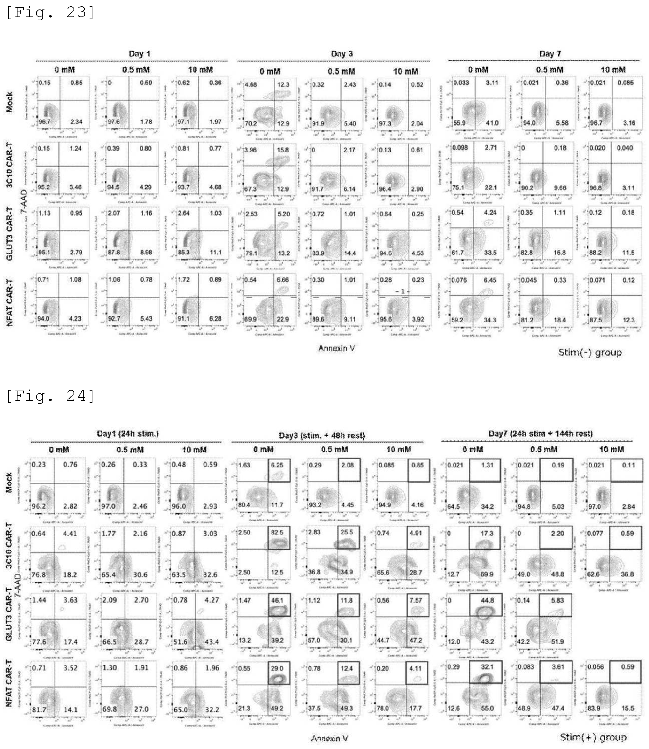

[FIG. 23] FIG. 23 shows data indicating enhancement of long-term survival after stimulation (in vitro data). The three panels on the left show results on day 1, the three panels in the middle show results on day 3, and the three panels on the right show results on day 7. In each panel, 0 mM, 0.5 mM, and 10 mM are shown from the left, and results for Mock, 3C10 CAR-T, and GLUT3 CAR-T are shown from the top. At each time point, staining data for 7-AAD and Annexin V, which reflect cell death and apoptosis, are shown. Cell death in each CAR-T cell was examined using Annexin V and 7-AAD. In an unstimulated state, differences depending on glucose concentration were observed, but no substantial differences were observed among the respective CAR-T cells.
[FIG. 24] FIG. 24 shows in vitro data indicating enhancement of long-term survival after stimulation. The three panels on the left show results on day 1, the three panels in the middle show results on day 3, and the three panels on the right show results on day 7. In each panel, 0 mM, 0.5 mM, and 10 mM are shown from the left, and results for Mock, 3C10 CAR-T, and GLUT3 CAR-T are shown from the top. At each time point, staining data for 7-AAD and Annexin V, which reflect cell death and apoptosis, are shown. Survival and cell death of each CAR-T cell after stimulation were evaluated in the same manner as in the previous figure. Compared with 3C10 CAR-T cells and GLUT3 CAR-T cells, survival was enhanced (cell death was suppressed) in NFATi GLUT3 CAR-T cells.

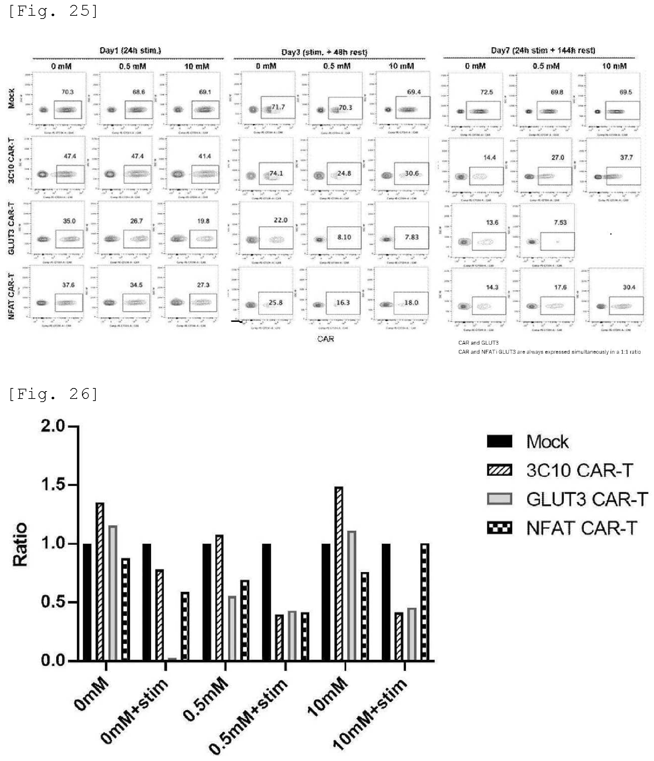

[FIG. 25] FIG. 25 shows in vitro data indicating enhancement of long-term survival after stimulation. The three panels on the left show results on day 1, the three panels in the middle show results on day 3, and the three panels on the right show results on day 7. In each panel, glucose concentrations of 0 mM, 0.5 mM, and 10 mM are shown from the left, and results for Mock, 3C10 CAR-T, and GLUT3 CAR-T are shown from the top. The proportions of CAR-expressing cells at each time point are shown. The proportion of CAR-positive cells was analyzed for each CAR-T cell population after stimulation. After stimulation, the proportion of CAR-positive cells decreased, particularly in GLUT3 CARCAR-T cells with constitutive expression, and it was considered that this fraction had undergone cell death.
[FIG. 26] FIG. 26 shows enhancement of long-term survival after stimulation. T-cell counts on day 7 are shown. As a model reflecting post-administration dynamics in a patient body (dynamics in which, after administration, cells infiltrate from blood having a sufficient glucose concentration into a tumor environment, which is a low- to no-glucose environment), cells were established and cultured under normal glucose conditions (glucose 10 mM), stimulated with EGFRvIII-binding beads, then cultured under no-glucose (0 mM), low-glucose (0.5 mM), or normal-glucose (10 mM) conditions, and the viable cell count after 7 days was evaluated. The graph shows the relative cell number, with the Mock T-cell count set to 1. In GLUT3 CAR-T, almost all cells had died under 0 mM. On the other hand, in NFATi CAR-T, many cells were maintained. It was suggested that cell death may have resulted from overactivation caused by constitutive GLUT3 expression and metabolism highly dependent on glucose.

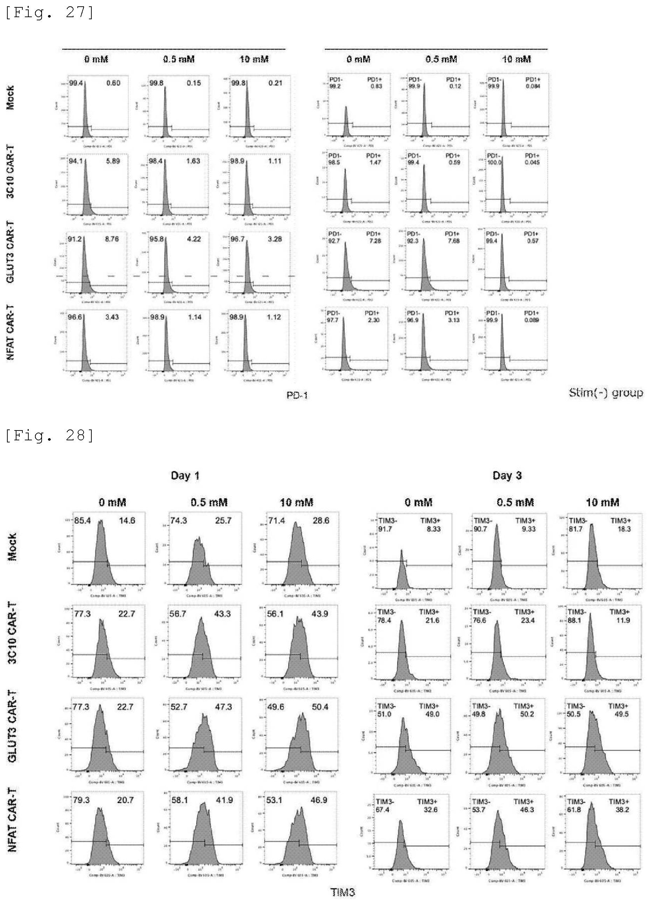

[FIG. 27] FIG. 27 shows expression of an inhibitory molecule (PD-1). The three left panels show results of PD-1 expression on day 1 of culture under no-glucose (0 mM), low-glucose (0.5 mM), or normal-glucose (10 mM) conditions, and the three right panels show results of PD-1 expression on day 3 of culture. In each panel, 0 mM, 0.5 mM, and 10 mM are shown from the left, and results for Mock, 3C10 CAR-T, and GLUT3 CAR-T are shown from the top. In NFATi GLUT3 CAR-T cells, expression of the inhibitory molecule (PD-1) was lower than that in GLUT3 CAR-T cells.
[FIG. 28] FIG. 28 shows suppressed expression of an inhibitory molecule (TIM3). The three left panels show results of TIM3 expression on day 1 of culture under no-glucose (0 mM), low-glucose (0.5 mM), or normal-glucose (10 mM) conditions, and the three right panels show results of TIM3 expression on day 3 of culture. In each panel, 0 mM, 0.5 mM, and 10 mM are shown from the left, and results for Mock, 3C10 CAR-T, and GLUT3 CAR-T are shown from the top. In NFATi GLUT3 CAR-T cells, expression of the inhibitory molecule (TIM3) was lower than that in GLUT3 CAR-T cells.

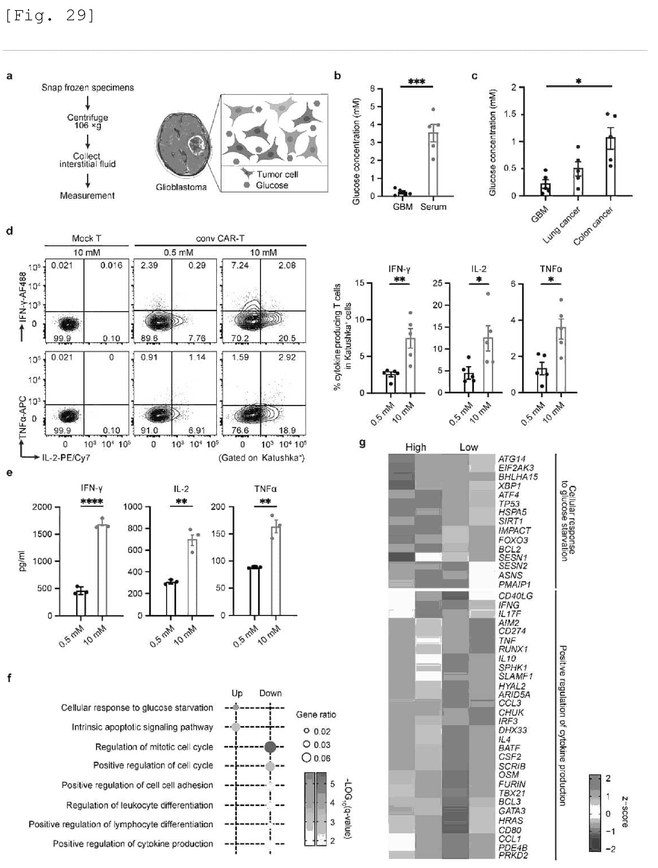

[FIG. 29] FIG. 29 is a drawing showing that a low-glucose state impairs the function of CAR-T cells. (a-c) Interstitial fluid was collected by a low-speed centrifugation method from surgical specimens or biopsy specimens of patients (n=5 each) with glioblastoma (GBM), non-small cell lung cancer (Lung cancer), or colorectal cancer (Colon cancer), and glucose concentrations were examined. (a) Experimental schema (b) Glucose concentrations in the interstitial fluid of GBM specimens and in the paired serum specimens Data are shown as mean ±SEM. Statistical analysis by Student's t-test; ***, P<0.001 (c) Glucose concentrations in interstitial fluid of GBM, non-small cell lung cancer (lung cancer), and colorectal cancer (colorectal cancer) specimens Data are shown as mean ±SEM. Statistical analysis by one-way analysis of variance; *, P<0.05 (d,e) Mock-introduced T cells (Mock T cells) and conventional EGFRvIII CAR-T cells were prepared from PBMCs of healthy subjects (n=5) (d) Cytokine production by Mock-transduced T cells (Mock T) and conv EGFRvIII CAR-T cells (conv CAR-T) when stimulated with EGFRvIII-expressing tumor cells (U-87mGΔ) under low-glucose (0.5 mM) or high-glucose (10 mM) conditions was examined by intracellular cytokine staining. Representative contour plots (left) and a summary of frequencies of cytokine (IFN-γ, IL-2, TNFα)-producing cells (right) are shown. Data are shown as mean ±SEM. Statistical analysis by Student's t-test; *, P<0.05; **, P<0.01 (e) Cytokine (IFN-γ, IL-2, TNFα) production by conv EGFRvIII CAR-T cells co-cultured with U-87mGΔ cells for 16 hours under low-glucose (0.5 mM) or high-glucose (10 mM) conditions was measured by ELISA. Data are shown as mean ±SEM. Statistical analysis by Student's t-test; ***, P<0.001; ****, P<0.0001 (f) Gene expression of EGFRvIII CAR-T cells 24 hours after EGFRvIII bead stimulation under low-glucose (0.5 mM) or high-glucose (10 mM) conditions. Pathways enriched under low-glucose conditions compared with high-glucose conditions are shown according to the Molecular Signatures Database. (g) Gene expression of selected genes is shown in a heat map. Gene expression was normalized by row. All experiments were performed at least twice.

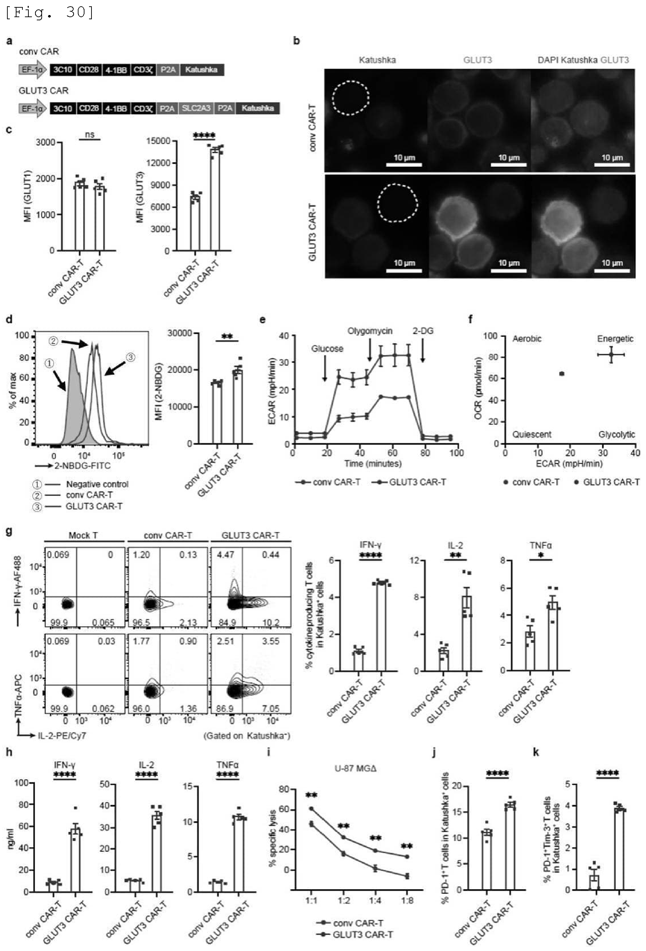

[FIG. 30] FIG. 30 is a drawing showing that overexpression of GLUT3 enhances metabolic fitness of CAR-T cells and increases cytotoxicity and cytokine production under low-glucose conditions. (a) Constructs of a conventional EGFRvIII CAR (conv CAR, upper) and an EGFRvIII CAR stably expressing GLUT3 (GLUT3 CAR, lower) (b) Immunofluorescence staining of conv EGFRvIII CAR-T cells (conv CAR-T) and GLUT3 EGFRvIII CAR-T cells (GLUT3 CAR-T) White dotted circles indicate T cells that were not transduced with CAR. Katushka (red), 4,6-diamino-2-phenylindole (DAPI: blue), GLUT3 (green) (c) Expression levels of GLUT1 (left) and GLUT3 (right) in CAR-T cells A summary of mean fluorescence intensity (MFI) is shown. Data are shown as mean ±SEM. Statistical analysis by Student's t-test; ns, not significant; ****, P<0.0001 (d) Glucose uptake by CAR-T cells as measured by a glucose analog 2-NBDG uptake assay Representative histograms (left) and a summary of mean fluorescence intensity (MFI) (right) are shown. Data are shown as mean ±SEM. Statistical analysis by Student's t-test; **, P<0.01 (e,f) Glycolytic ability (e) and metabolic phenotype (f) of CAR-T cells under low-glucose (0.5 mM) conditions as analyzed by a flux analyzer Data are shown as mean ±SD. (g,h) CAR-T cells were prepared from PBMCs of healthy subjects (n=5) (g) Cytokine production upon stimulation with EGFRvIII-expressing tumor cells (U-87mGΔ) under low-glucose (0.5 mM) conditions was examined by intracellular cytokine staining. Representative contour plots (left) and a summary of frequencies of cytokine (IFN-γ, IL-2, TNFα)-producing cells (right) are shown. Data are shown as mean ±SEM. Statistical analysis by Student's t-test; *, P<0.05; **, P<0.01; ****, P<0.0001 (h) Cytokine (IFN-γ, IL-2, TNFα) production by CAR-T cells co-cultured with U-87mGΔ cells for 16 hours under low-glucose (0.5 mM) conditions was measured by ELISA. Data are shown as mean ±SEM. Statistical analysis by Student's t-test; ****, P<0.0001 (i) Cytotoxicity of CAR-T cells under low-glucose (0.5 mM) conditions was measured by a luciferase-based assay. CAR-T cells and luciferase-expressing target cells (U-87mGΔ-Luc) were co-cultured at the indicated effector-to-target (E : T) ratios for 24 hours, and specific lysis was calculated based on luciferase activity. Data are shown as mean ±SEM. Statistical analysis by Student's t-test; **, P<0.01 (j,k) Expression of exhaustion markers (PD-1 and Tim-3) by CAR-T cells A summary of the frequencies of PD-1⁺ CAR-T cell (j) and PD-1⁺Tim-3⁺ CAR-T cell (k) populations is shown. Data are shown as mean ±SEM. Statistical analysis by Student's t-test; ****, P<0.0001

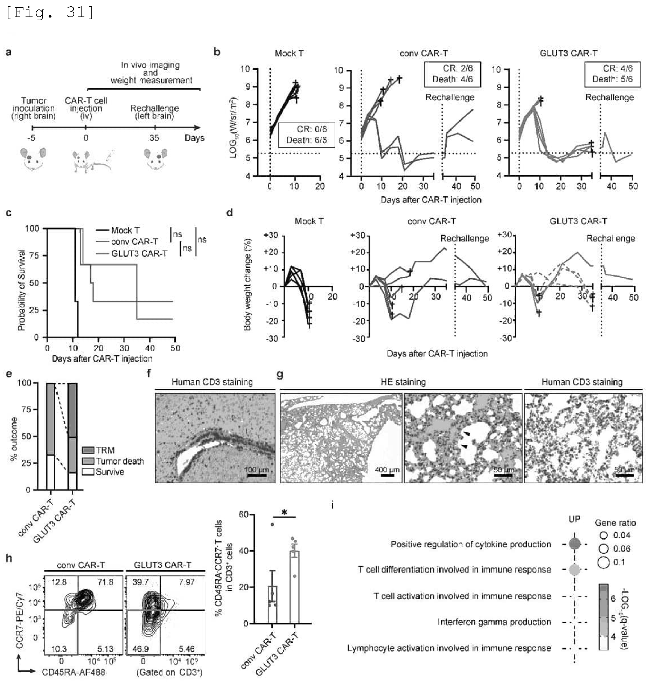

[FIG. 31] FIG. 31 is a drawing showing experimental results in an intracranial human GBM cell xenograft model. In this model, stable GLUT3 expression in CAR-T cells did not improve survival because of severe toxicity. (a) Experimental schema NSG mice (n=6) intracranially inoculated with U-87mGΔ-Luc cells were intravenously administered, on day 5 after tumor inoculation, either mock T cells (Mock T), conventional EGFRvIII CAR-T cells (conv CAR-T), or GLUT3 EGFRvIII CAR-T cells (GLUT3 CAR-T). Mice in which U-87mGΔ-Luc tumors were eradicated by treatment with conventional EGFRvIII CAR-T cells or GLUT3 EGFRvIII CAR-T cells were rechallenged with the same tumor on day 35. Tumor burden was monitored by BLI. (b) Tumor burden measured by BLI Each line indicates an individual mouse treated with the indicated CAR-T cells. Crosses indicate cause of death. The numbers in the upper right panel indicate the number of mice that achieved a complete response (CR) by day 35 and the number of mice that died. The horizontal dotted line indicates the background BLI signal level obtained from mice that were not inoculated with tumor. (c) Kaplan-Meier curves Statistical analysis by a log-rank test for the Kaplan-Meier curves (d) Changes in body weight of each mouse during CAR-T-cell treatment Crosses indicate any cause of death, and colored dotted lines indicate mice with treatment-related mortality (TRM). (e) Stacked bar graph showing the results at the end of the observation period. TRM: treatment-related mortality (f,g) Pathological analysis of brain and lung tissues of mice in which severe weight loss was observed on day 14. (f) IHC staining of human CD3⁺ cells in brain tissue (g) Hematoxylin and eosin (HE) staining of lung tissue (left: low magnification, middle: high magnification) and human CD3 staining (right) Arrows indicate hyaline membranes. (h) Phenotype of circulating human T cells (CAR-T cells) on day 14 after CAR-T-cell administration Representative contour plots (left) and a summary of the frequency of effector memory (CD45RA CCR7⁻⁻) T cells (right) are shown. Data are shown as mean ±SEM. Statistical analysis by Student's t-test; *, P<0.05 (i) Comparison of gene expression between conventional EGFRvIII CAR-T cells and GLUT3 EGFRvIII CAR-T cells on day 7 after stimulation with EGFRvIII beads under low-glucose (0.5 mM) conditions Pathways enriched in GLUT3 EGFRvIII CAR-T cells compared with conventional EGFRvIII CAR-T cells are shown according to the Molecular Signatures Database.

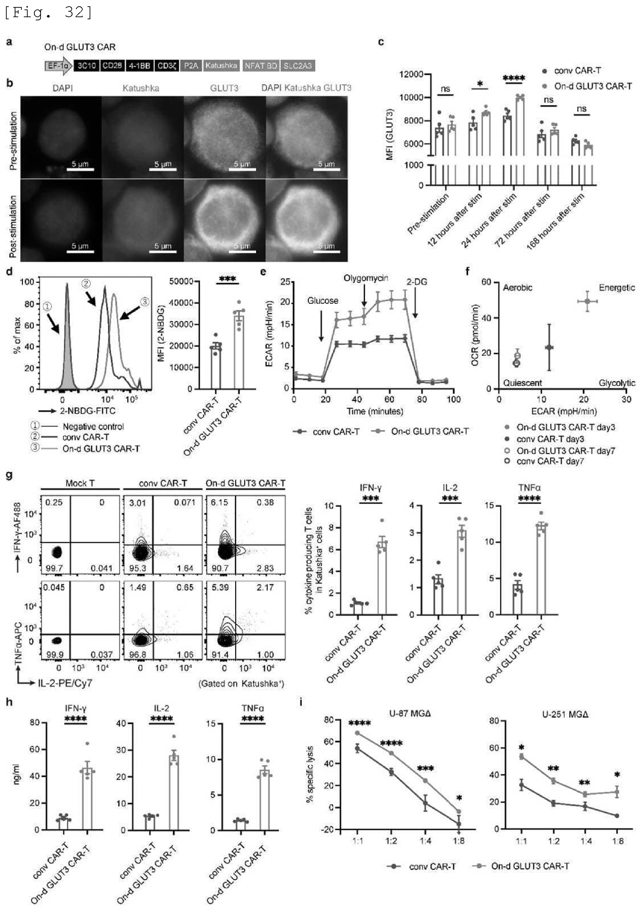

[FIG. 32] FIG. 32 is a drawing showing that on-demand expression of GLUT3 imparts excellent effector activity to CAR-T cells. (a) A construct in which GLUT3 expression is driven by NFAT binding to an NFAT-binding domain (NFAT-BD), enabling on-demand GLUT3 expression on CAR-T cells (on-demand GLUT3 CAR) BD: binding domain (b) Immunofluorescence imaging of on-demand GLUT3 EGFRvIII CAR-T cells for Katushka (red), which is a CAR introduction marker, 4,6-diamino-2-phenylindole (DAPI: blue), and GLUT3 (green) (c) Time course of GLUT3 expression in conv EGFRvIII CAR-T cells (conv CAR-T) and on-demand GLUT3 EGFRvIII CAR-T cells (on-demand GLUT3 CAR-T) Data are shown as mean ±SEM. Statistical analysis by Student's t-test; ns, not significant; *, P<0.05; t****, P<0.0001 MFI: mean fluorescence intensity (d) Glucose uptake ability as measured by a glucose analog 2-NBDG uptake assay Representative histograms (left) and an MFI summary (right) are shown. Data are shown as mean ±SEM. Statistical analysis by Student's t-test; ***, P<0.001 (e,f) Glycolytic ability (e) and metabolic phenotype (f) of CAR-T cells cultured under low-glucose (0.5 mM) conditions were examined by a flux analyzer on day 3 and day 7 as indicated after stimulation with EGFRvIII beads. Data are shown as mean ±SD. (g,h) CAR-T cells were prepared from PBMCs of healthy subjects (n=5) (g) Cytokine production associated with stimulation with EGFRvIII-expressing tumor cells (U-87mGΔ) under low-glucose (0.5 mM) conditions by intracellular cytokine staining Representative contour plots (left) and a summary of frequencies of cytokine (IFN-γ, IL-2, TNFα)-producing cells (right) are shown. Data are shown as mean ±SEM. Statistical analysis by Student's t-test; ***, P<0.001; ****, P<0.0001 (h) Cytokine (IFN-γ, IL-2, TNFα) production by CAR-T cells co-cultured with U-87mGΔ cells for 16 hours under low-glucose (0.5 mM) conditions was measured by ELISA. Data are shown as mean ±SEM. Statistical analysis by Student's t-test; ****, P<0.0001 (i) Cytotoxicity of CAR-T cells against U-87mGΔ and U-251mGΔ cells under low-glucose (0.5 mM) conditions was measured by a luciferase-based assay. CAR-T cells and luciferase-expressing target cells (U-87mGΔ-Luc or U-251mGΔ-Luc) were co-cultured at the indicated effector-to-target (E : T) ratios for 24 hours under low-glucose (0.5 mM) conditions, and specific lysis was calculated based on luciferase activity. Data are shown as mean ±SEM. Statistical analysis by Student's t-test; *, P<0.05; **, P<0.01; ***, P<0.001; ****, P<0.0001

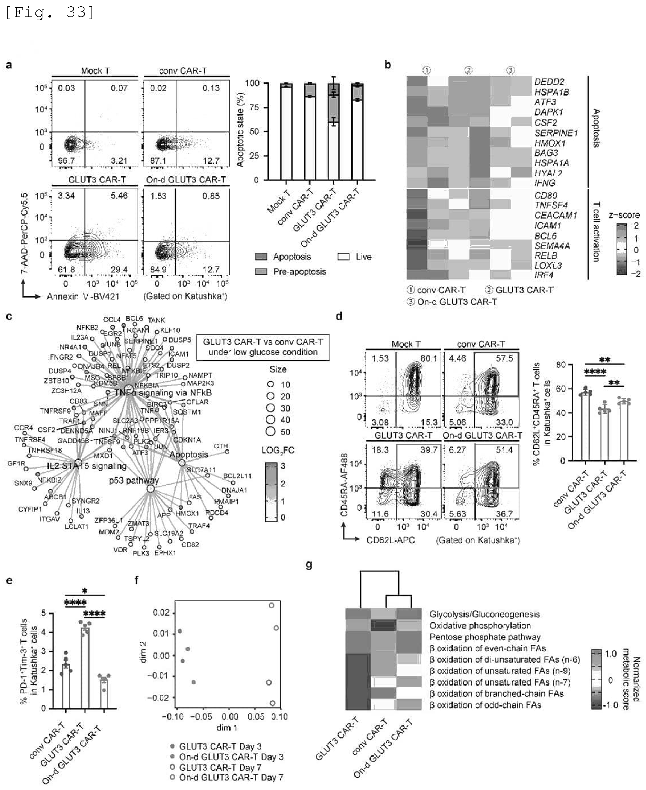

[FIG. 33] FIG. 33 is a drawing showing that on-demand expression of GLUT3 results in a reduction in differentiated T cells and improves survival of T cells by avoiding overactivation. (a) Apoptotic status measured by Annexin V and 7-AAD staining of the indicated CAR-T cell populations [mock T cells (Mock T), conv EGFRvIII CAR-T cells (conv CAR-T), GLUT3 EGFRvIII CAR-T cells (GLUT3 CAR-T), or on-demand GLUT3 EGFRvIII CAR-T cells (on-demand GLUT3 CAR-T)] 72 hours after antigen stimulation under low-glucose (0.5 mM) conditions Representative contour plots (left) and a summary of the frequencies of live (Live) (Annexin V⁻ 7-AAD⁻), pre-apoptotic (Pre-apoptosis) (Annexin V⁺ 7-AAD⁻), and apoptotic (Apoptosis) (Annexin V⁺ 7-AAD⁺) cell populations (right) are shown. (b) Heat map of gene expression related to apoptosis and T-cell activation in CAR-T cells 72 hours after stimulation with EGFRvIII beads under low-glucose (0.5 mM) conditions Gene expression was normalized by row. (c) Comparison of gene expression between conv EGFRvIII CAR-T cells (conv CAR-T) and GLUT3 EGFRvIII CAR-T cells (GLUT3 CAR-T) 72 hours after stimulation with EGFRvIII beads under low-glucose (0.5 mM) conditions Pathways enriched in GLUT3 EGFRvIII CAR-T cells as compared with conv EGFRvIII CAR-T cells are shown, together with their interrelationships. (d,e) CAR-T cells were prepared from PBMCs of healthy subjects (n=5) and were stimulated with EGFRvIII-expressing tumor cells (U-87mGΔ) under low-glucose (0.5 mM) conditions. (d) T-cell phenotypes of the indicated CAR-T cell populations (CD62L⁺ CD45RA⁺: naive/stem cell memory; CD62L⁺ CD45RA⁻: central memory; CD62L⁻CD45RA⁻: effector memory; and CD62L⁻ CD45RA⁺: terminally differentiated effector memory). Representative contour plots (left) and a summary of the frequency of the naive/stem cell memory T-cell (CD62L⁺CD45RA⁺) population (right) are shown. Data are shown as mean ±SEM. Statistical analysis by Student's t-test; ***, P<0.01; ****, P<0.0001 (e) Expression of exhaustion markers (PD-1 and Tim-3) by CAR-T cells A summary of the frequency of the PD-1⁺Tim-3⁺CAR-T cell population is shown. Data are shown as mean ±SEM. Statistical analysis by Student's t-test; *, P<0.05; ****, P<0.0001 (f) Comparison of gene expression between GLUT3 EGFRvIII CAR-T cells (GLUT3 CAR-T) and on-demand GLUT3 EGFRvIII CAR-T cells (on-demand GLUT3 CAR-T) after stimulation with EGFRvIII beads under low-glucose (0.5 mM) conditions Principal component analysis of RNA-seq transcriptome profiles of GLUT3 EGFRvIII CAR-T cells and on-demand GLUT3 EGFRvIII CAR-T cells on day 3 or day 7 after stimulation (g) Heat map of metabolic scores of CAR-T cells analyzed by METAFlux using RNA-seq data on day 7 after stimulation. Gene expression was normalized by row. FA: fatty acid

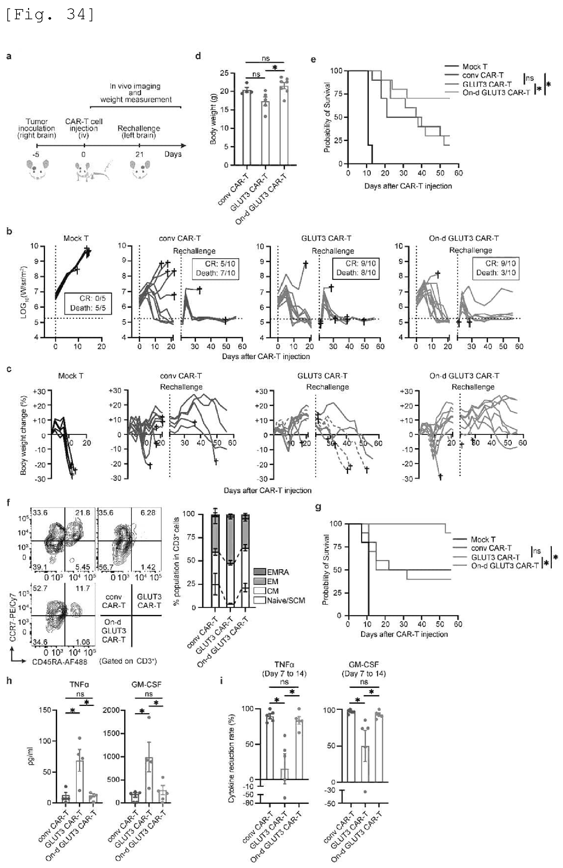

[FIG. 34] FIG. 34 is a drawing showing on-demand GLUT3 expression by CAR-T cells. On-demand GLUT3 expression by CAR-T cells induces a sustained antitumor effect with a superior survival benefit in an intracranial human GBM cell xenograft model (a) Experimental schema NSG mice (n=10, except for the mock T-cell group; n=5) intracranially inoculated with U-87mGΔ-Luc cells were intravenously administered, on day 5 after tumor inoculation, either mock T cells (Mock T), conv EGFRvIII CAR-T cells (conv CAR-T), GLUT3 EGFRvIII CAR-T cells (GLUT3 CAR-T), or on-demand GLUT3 EGFRvIII CAR-T cells (on-demand GLUT3 CAR-T). Mice in which U-87mGΔ-Luc tumors were eradicated by treatment with conv EGFRvIII CAR-T cells, GLUT3 EGFRvIII CAR-T cells, or on-demand GLUT3 EGFRvIII CAR-T cells were rechallenged with the same tumor on day 21. Tumor burden was monitored by BLI. (b) Tumor burden measured by BLI Each line indicates an individual mouse treated with the indicated CAR-T cells. Crosses indicate cause of death. The numbers in the upper right panel indicate the number of mice that achieved a complete response (CR) by day 21 and the number of mice that died. The horizontal dotted line indicates the background BLI signal level obtained from mice that were not inoculated with tumor. (c) Changes in body weight of each mouse during CAR-T-cell treatment Crosses indicate any cause of death, and colored dotted lines indicate mice with treatment-related mortality (TRM). (d) Body weight of mice on day 42 after CAR-T-cell infusion Data are shown as mean ±SEM. Statistical analysis by Student's t-test; ns, not significant; *, P<0.05 (e) Kaplan-Meier curves of survival of U-87mGΔ tumor-bearing mice treated with the indicated CAR-T cells (n=10, except for the mock T-cell group; n=5) Statistical analysis by a log-rank test for the Kaplan-Meier curves; ns, not significant; *, P<0.05 (f) Phenotypes of circulating human T cells on day 14 after CAR-T-cell administration Representative contour plots (left) and summaries of the frequencies of each T-cell population [CD45RA CCR7⁺⁺: naive/stem cell memory (SCM), CD45RA CCR7⁻⁺: central memory (CM), CD45RA CCR7⁻⁻: effector memory (EM), and CD45RA CCR7⁺⁻: terminally differentiated effector memory (EMRA)] are shown. (g) Kaplan-Meier curves of survival of mice bearing U-251mGΔ cells treated with the indicated CAR-T cells (n=10, except for the mock T-cell group; n=5) Statistical analysis by a log-rank test for the Kaplan-Meier curves; ns, not significant; *, P<0.05 (h) Serum cytokine (TNFα and GM-CSF) concentrations on day 14 after CAR-T-cell administration Data are shown as mean ±SEM. Statistical analysis by Student's t-test; ns, not significant; *, P<0.05 (i) Rates of decrease in serum TNFα and GM-CSF on day 14 relative to day 7 after CAR-T-cell administration Data are shown as mean ±SEM. Statistical analysis by Student's t-test; ns, not significant; *, P<0.05 From the results of FIG. 34, it can be said that constitutively expressed GLUT3 enhances the antitumor effect (that is, effector function) of EGFRvIII CAR-T cells. It can also be said that on-demand GLUT3 expression not only enhances the effector function of EGFRvIII CAR-T cells, but also enhances prolongation of memory function and maintenance of the memory T-cell phenotype. Furthermore, it can be concluded that adverse events such as weight loss and treatment-related death induced by constitutively expressed GLUT3 were prevented, and as a result, a remarkable improvement in survival, including cure, was achieved.

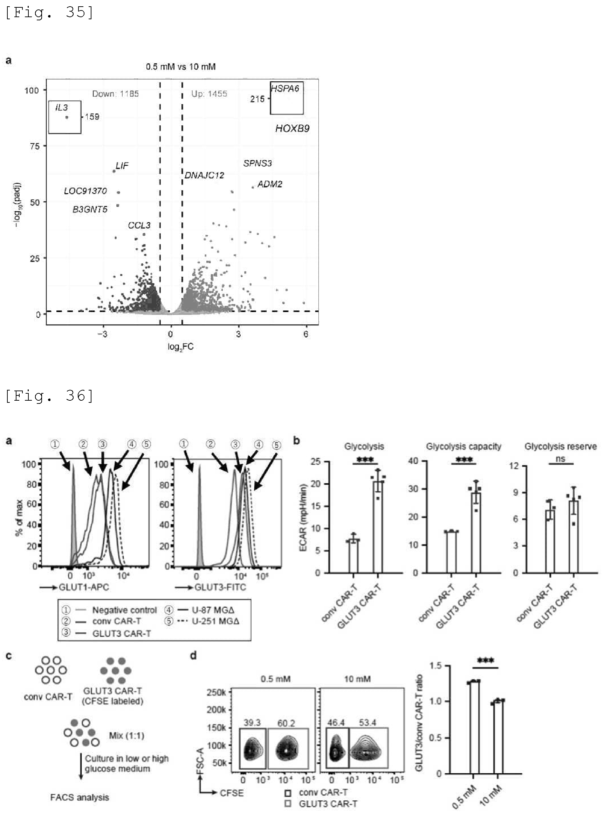

[FIG. 35] FIG. 35 is a drawing showing that a low-glucose state impairs the function of CAR-T cells. (a) Volcano plot showing genes differentially expressed in conv EGFRvIII CAR-T cells cultured under low-glucose (0.5 mM) conditions compared with high-glucose (10 mM) conditions The X-axis represents log₂ (fold change) (log₂ FC), and the Y-axis represents -log₁₀ (adjusted p value) [(-log₁₀(padj))]. The dotted lines represent the cutoff value for log₂ (fold change), x=±1.4, and the cutoff value for -log₁₀ (adjusted p value), y=0.1. The top 5 genes and bottom 5 genes are highlighted in green and purple, respectively.
[FIG. 36] FIG. 36 is a drawing showing that overexpression of GLUT3 enhances metabolic fitness of CAR-T cells. (a) Expression of GLUT1 (left) and GLUT3 (right) in conv EGFRvIII CAR-T cells (conv CAR-T), GLUT3 EGFRvIII CAR-T cells (GLUT3 CAR-T), U-87mGΔ cells, and U-251mGΔ cells Representative histograms are shown. (b) Glycolytic parameters of CAR-T cells cultured under low-glucose (0.5 mM) conditions as measured by a glycolysis stress test Data are shown as mean ±SD. Statistical analysis by Student's t-test; ns, not significant; ***, P<0.001 (c,d) Competition assay (72-hour culture) of CAR-T cells under low-glucose (0.5 mM) or high-glucose (10 mM) conditions (c) Experimental schema (n=3) (d) Representative contour plots (left) and a summary of the proportions of each CAR-T-cell population (right) Data are shown as mean ±SEM. Statistical analysis by Student's t-test; ***, P<0.001

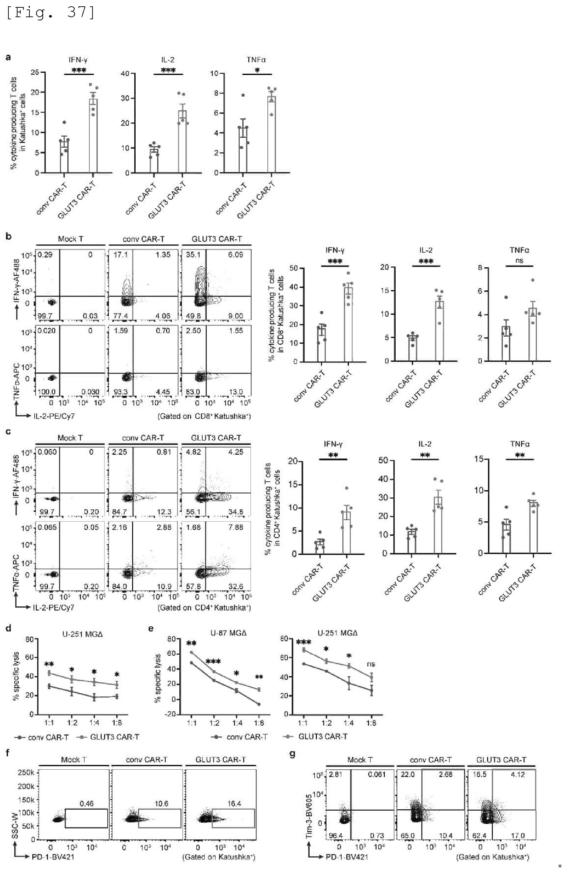

[FIG. 37] FIG. 37 is a drawing showing that stable expression of GLUT3 improves the function of CAR-T cells under low-glucose conditions. (a-c) Cytokine production by mock T cells (Mock T), conv EGFRvIII CAR-T cells (conv CAR-T), and GLUT3 EGFRvIII CAR-T cells (GLUT3 CAR-T) (n=5) when stimulated with EGFRvIII-expressing tumor cells (U-87mGΔ) under high-glucose (10 mM) conditions was examined by intracellular staining. (a) Summary of the frequencies of cytokine (IFN-γ, IL-2, TNFα)-producing CD3⁺ CAR-T cells Data are shown as mean ±SEM. Statistical analysis by Student's t-test; *, P<0.05; ***, P<0.001 (b) Representative contour plots (left) and a summary of the frequencies of cytokine (IFN-γ, IL-2, TNFα)-producing CD8⁺ CAR-T cells (right) Data are shown as mean ±SEM. Statistical analysis by Student's t-test; ns, not significant; ***, P<0.001 (c) Representative contour plots (left) and a summary of the frequencies of cytokine (IFN-γ, IL-2, TNFα)-producing CD4⁺ CAR-T cells (right) Data are shown as mean ±SEM. Statistical analysis by Student's t-test; **, P<0.01 (d,e) Cytotoxicity of CAR-T cells against U-87mGΔ cells and U-251mGΔ cells under low-glucose (0.5 mM) or high-glucose (10 mM) conditions was determined by a luciferase-based assay. CAR-T cells and luciferase-expressing target cells (U-87mGΔ-Luc and U-251mGΔ-Luc) were co-cultured at the indicated effector-to-target (E : T) ratios for 24 hours, and specific lysis was calculated based on luciferase activity. (d) Cytotoxicity of CAR-T cells against U-251mGΔ-Luc cells under low-glucose (0.5 mM) conditions Data are shown as mean ±SEM. Statistical analysis by Student's t-test; *, P<0.05; **, P<0.01
   (e) Cytotoxicity of CAR-T cells against U-87mGΔ-Luc cells or U-251mGΔ-Luc cells under high-glucose (10 mM) conditions Data are shown as mean ±SEM. Statistical analysis by Student's t-test; ns, not significant; *, P<0.05; **, P<0.01; ***, P<0.001
   (F,G) Expression of exhaustion markers (PD-1, f; PD-1 and Tim-3, g) by the indicated CAR-T cell populations Representative contour plots are shown.

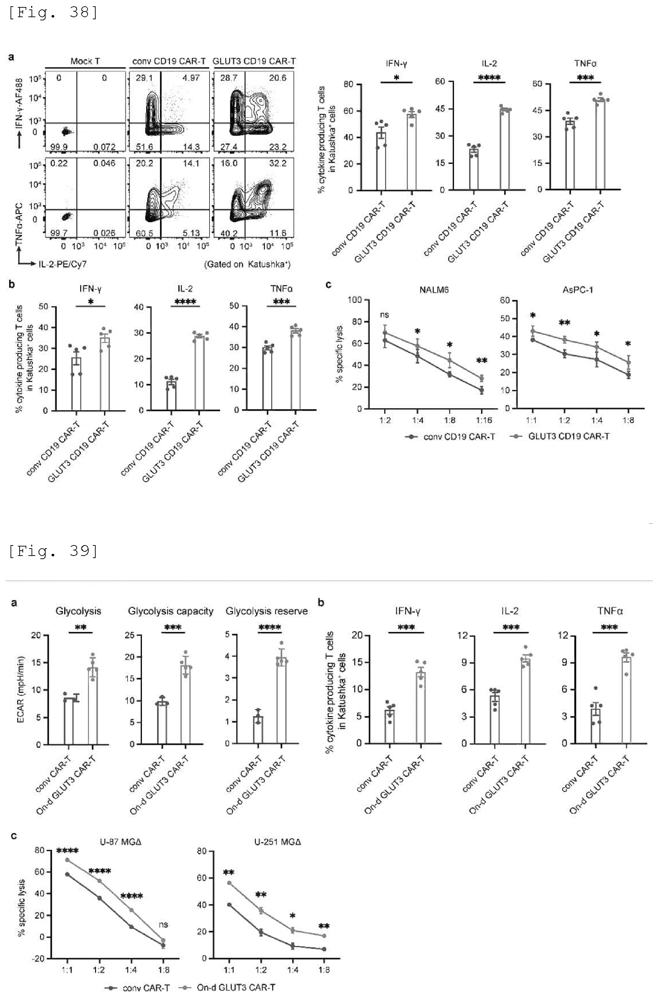

[FIG. 38] FIG. 38 is a drawing showing that improvement in CAR-T cell function by stable expression of GLUT3 does not depend on the type of scFv. (a,b) Mock T cells (Mock T), conv CD19 CAR-T cells (conv CD19 CAR-T), and GLUT3 CD19 CAR-T cells (GLUT3 CD19 CAR-T) were prepared from PBMCs of healthy subjects (n=5). (a) Cytokine production by the indicated CAR-T cells upon stimulation with CD19-expressing tumor cells (NALM6) under low-glucose (0.5 mM) conditions, as determined by intracellular cytokine staining Representative contour plots (left) and a summary of frequencies of cytokine (IFN-γ, IL-2, TNFα)-producing cells (right) are shown. Data are shown as mean ±SEM. Statistical analysis by Student's t-test; *, P<0.05; ***, P<0.001; ****, P<0.0001
   (b) Cytokine (IFN-γ, IL-2, TNFα) production by conv CD19 CAR-T cells and GLUT3 CD19 CAR-T cells co-cultured with NALM6 for 16 hours under low-glucose (0.5 mM) conditions was measured by ELISA. Data are shown as mean ±SEM. Statistical analysis by Student's t-test; *, P<0.05; ***, P<0.001; ****, P<0.0001
   (c) Cytotoxicity of conv CD19 CAR-T cells and GLUT3 CD19 CAR-T cells against NALM6 and CD19-expressing AsPC-1 cells under low-glucose (0.5 mM) conditions was determined by a luciferase-based assay. CAR-T cells and luciferase-expressing target cells (NALM6-Luc and CD19-expressing AsPC-1-Luc) were co-cultured at the indicated effector-to-target (E : T) ratios for 24 hours, and the specific lysis rate was evaluated based on luciferase activity. Data are shown as mean ±SEM. Statistical analysis by Student's t-test; ns, not significant; *, P<0.05; **, P<0.01
[FIG. 39] FIG. 39 is a drawing showing that on-demand GLUT3 expression enhances metabolic fitness and improves cytokine production and cytotoxicity of CAR-T cells. (a) Glycolytic parameters of conv EGFRvIII CAR-T cells (conv CAR-T) and on-demand GLUT3 EGFRvIII CAR-T cells (on-demand GLUT3 CAR-T) cultured under low-glucose (0.5 mM) conditions (measured by a glycolysis stress test) are shown. Data are shown as mean ±SD. Statistical analysis by Student's t-test; **, P<0.01; ***, P<0.001; ****, P<0.0001 (b,c) Conv EGFRvIII CAR-T cells (conv CAR-T) and on-demand GLUT3 EGFRvIII CAR-T cells (on-demand GLUT3 CAR-T) were prepared from PBMCs of healthy subjects (n=5). (b) Cytokine production by the indicated CAR-T cells upon stimulation with EGFRvIII-expressing tumor cells (U-87mGΔ) under high-glucose (10 mM) conditions was examined by intracellular cytokine staining. A summary of the frequencies of cytokine (IFN-γ, IL-2, TNFα)-producing cells is shown. Data are shown as mean ±SEM. Statistical analysis by Student's t-test; ***, P<0.001 (c) Cytotoxicity of CAR-T cells against U-87mGΔ cells and U-251mGΔ cells under high-glucose (10 mM) conditions was determined by a luciferase-based assay. CAR-T cells and luciferase-expressing target cells (U-87mGΔ-Luc or U-251mGΔ-Luc) were co-cultured at the indicated effector-to-target (E : T) ratios for 24 hours under high-glucose (10 mM) conditions, and specific lysis was calculated based on luciferase activity. Data are shown as mean ±SEM. Statistical analysis by Student's t-test; ns, not significant; *, P<0.05; **, P<0.01; ****,<0.0001.

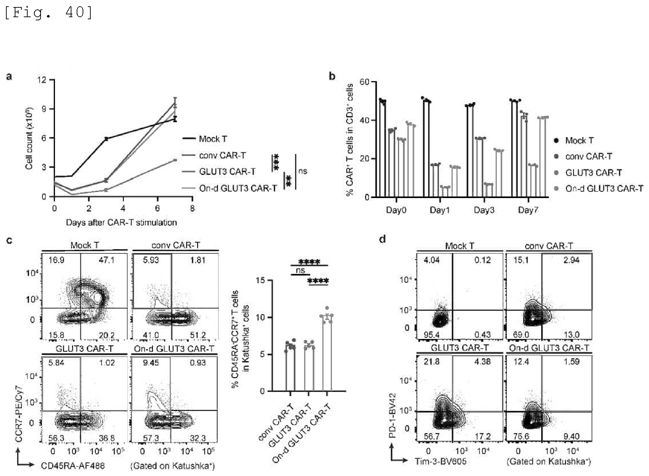

[FIG. 40] FIG. 40 is a drawing showing that on-demand expression of GLUT3 improves the survival of CAR-T cells. (a,b) T-cell proliferation among CD3⁺ cells and the frequency of CAR⁺ T cells at 24 hours after stimulation with anti-EGFRvIII beads under low-glucose (0.5 mM) conditions Mock T cells (Mock T), conv EGFRvIII CAR-T cells (conv CAR-T), GLUT3 EGFRvIII CAR-T cells (GLUT3 CAR-T), and on-demand GLUT3 EGFRvIII CAR-T cells (on-demand GLUT3 CAR-T) were prepared from PBMCs of healthy subjects (n=3). (c) Phenotype of CAR-T cells on day 7 after stimulation with U-87mGΔ cells Representative contour plots (left) and a summary of the frequency of central memory (CD45RA CCR7⁻⁺) T cells (right) are shown. Data are shown as mean ±SEM. Statistical analysis by Student's t-test; ns, not significant; ****, P<0.0001 (d) Expression of bacterial shedding markers (PD-1 and Tim-3) by the indicated CAR-T-cell populations at 72 hours after stimulation with EGFRvIII-expressing tumor cells (U-87mGΔ) Representative contour plots are shown.

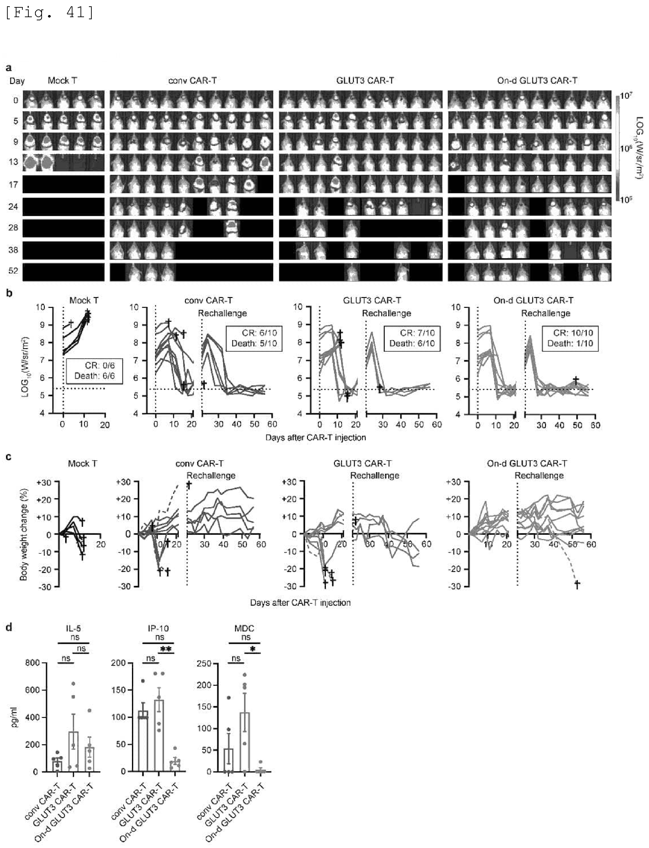

[FIG. 41] FIG. 41 is a drawing showing on-demand GLUT3 expression by CAR-T cells. On-demand GLUT3 expression by CAR-T cells increases survival while maintaining a sustained antitumor effect in an intracranial human GBM cell xenograft model. (a) BLI of mice having U-87mGΔ-Luc tumors (b,c) NSG mice (n=10, except for the mock CAR-T cell group; n=5) intracranially inoculated with U-251mGΔ-Luc cells were intravenously administered, on day 5 after tumor inoculation, either mock T cells (Mock T), conv EGFRvIII CAR-T cells (conv CAR-T), GLUT3 EGFRvIII CAR-T cells (GLUT3 CAR-T), or on-demand GLUT3 EGFRvIII CAR-T cells (on-demand GLUT3 CAR-T). Mice in which U-87mGΔ-Luc tumors were eradicated by EGFRvIII CAR-T cells, GLUT3 EGFRvIII CAR-T cells, or on-demand GLUT3 EGFRvIII CAR-T cells were rechallenged with the same tumor on day 21. Tumor burden was monitored by BLI. (b) Tumor burden measured by BLI Each colored line indicates an individual mouse. Crosses indicate cause of death. The numbers in the upper right indicate the number of mice that showed a complete response (CR) by day 21 and the number of mice that died. The horizontal dotted line indicates the baseline BLI signal level obtained from mice that were not inoculated with tumor.
   (c) Changes in body weight of mice during CAR-T-cell treatment Crosses indicate any cause of death, and colored dotted lines indicate mice that experienced treatment-related death (TRM). (d) Serum cytokine and chemokine concentrations (IL-5, IP-10, and MDC) on day 14 after CAR-T-cell administration were examined by ELISA. Data are shown as mean ±SEM. Statistical analysis by Student's t-test; ns, not significant; *, P<0.05; **, P<0.01

### Description of Embodiments

Hereinafter, the present disclosure will be described by way of the best mode. Throughout the present specification, the expression of singular forms is to be understood as including the concept of plural forms unless otherwise stated. Therefore, it should be understood that singular articles (for example, "a", "an", "the", and the like in English) also include the concept of their plural forms unless specifically stated otherwise. In addition, the terms used in the present specification are to be understood as being used in the sense commonly used in the art unless otherwise stated. Therefore, unless otherwise defined, all technical and scientific terms used in the present specification have the same meanings as generally understood by those skilled in the art to which the present disclosure belongs. In the case of conflict, the present specification (including definitions) will control.

Hereinafter, definitions and/or basic technical contents of terms particularly used in the present specification will be described as appropriate.

In the present specification, "about" means ±10% of the numerical value that follows. For example, "about 20" is intended to include "18 to 22". A numerical range includes all numerical values between both endpoints and the numerical values of both endpoints. "About" regarding a range applies to both endpoints of the range. Therefore, for example, "about 20 to 30" is intended to include "18 to 33".

In the present specification, the term "T cell" is used in a broad sense used in the art, and refers to lymphocytes generated in bone marrow that migrate to the thymus and mature. T cells may be CD45-positive and CD3-positive cells among normal fractions of peripheral blood and bone marrow-derived mononuclear cells. As the T cells to be used, T cells isolated from a donor, particularly a human donor, may be used, but are not limited thereto. Examples of T cells and cells derived therefrom include isolated T cells that have not been passaged in culture, T cells that have been passaged and maintained under cell culture conditions without being immortalized, and T cells that have been immortalized and can be maintained indefinitely under cell culture conditions. It is known that multiple types of T cells exist depending on their functions, and examples thereof include effector memory T cells (T_{eff}), naive T cells (Tnv), stem cell memory T cells (Tscm), central memory T cells (Tcm), terminally differentiated RA-positive T cells (Temra), and the like. In the present specification, the term "peripheral T cell" refers to a T cell present outside the thymus, and can be obtained from peripheral blood, lymph nodes, or other tissues. In the present specification, when referring to "peripheral T cells", it is sufficient that peripheral T cells are contained in a cell group, and the T cells do not need to be isolated. A cell fraction containing various lymphocytes in addition to T cells, such as peripheral blood mononuclear cells (PBMCs), may be used.

In the present specification, the term "cell population" refers to a population containing two or more cells, and may be, for example, a state in which cells are gathered together in a planar manner or a cell mass formed by adhesion of cells to each other three-dimensionally. In addition, the "cell population" may be formed of a single type of cell or may contain a plurality of types of cells. When referring to a cell population of T cells of the present disclosure, it is sufficient that the cell population contains at least one cell, such as Tnv, Tscm, Tcm, or Temra, that can have an effector function at a place where the cell is to function (typically in vivo).

In the present specification, the term "flow cytometry" refers to a technique for measuring the particle number and individual physical, chemical, and biological properties of cells, organisms, and other biological particles suspended in a liquid. An apparatus using this technique is referred to as a "flow cytometer". In the present disclosure, "positive" and "negative" for cell markers (for example, FoxP3, CTLA4, Helios, CD103, and the like) are determined by flow cytometry, as commonly used in the art. More specifically, in flow cytometry, cells are aligned and flowed in a single line, and the number of cells is counted by a spectroscopic technique. For example, the number of target cells is counted by irradiating cells labeled with fluorescence or a luminescent enzyme with laser light, and detecting fluorescence or luminescence signals emitted from the cells by a detector such as a photodiode. In addition, detection results from the detector can be imported into a computer to generate and display a twodimensional plot. This makes it possible to easily grasp the presence or absence of target cells, the number thereof, and the like.

In the present specification, the term "effector function" refers, in T cells, to biological activities such as cytotoxic activity, cytokine production, and division/proliferation, which are caused by signals via TCR or CAR, and refers, in other immune cells, to biological activities caused by the Fc region of an antibody. Examples of effector functions include C1q binding and complementdependent cytotoxicity (CDC), Fc receptor binding, antibody-dependent cellular cytotoxicity (ADCC), phagocytosis, downregulation of cell surface receptors (for example, B cell receptors), and activation of B cells. In the present disclosure, whether T cells have an effector function can be determined by confirming that the T cells have cytotoxic activity, cytokine production, and division/proliferation using methods exemplified in Examples and the like.

In the present specification, the term "effector cell" or "effector T cell" means an immune cell that is a T cell having an effector function and that exerts a cytotoxic action on a target cell, and may also be referred to as T_{eff}. In the present specification, whether a cell is an "effector" cell can be determined by determination methods such as a cytotoxicity assay, surface antigen analysis by flow cytometry, and intracellular cytokine staining. Effector cells are immune cells that execute effector functions, and mediate antibody-dependent cellular cytotoxicity (ADCC) and the like. Effector cells include, for example, peripheral blood mononuclear cells (PBMCs), natural killer (NK) cells, monocytes, macrophages, cytotoxic T cells, neutrophils, and the like, and can be isolated from natural tissues such as blood.

In the present specification, the term "precursor cells of effector T cells (T_{eff})" (also referred to as "Effector T cell precursors") refers to cells that are not effector cells, but acquire an effector function by being transferred to a place where they are to function (for example, in vivo) or by other stimulation. Such cells include lymphoid precursor cells, Tnv, Tscm, Tcm, Temra, and the like. Precursor cells of effector T cells are activated at an early stage of an immune response and mature into effector T cells through proliferation and differentiation, whereas some precursor cells have an ability to differentiate into memory T cells. These precursor cells have specific gene expression patterns and metabolic states, which contribute to prolongation of memory function. Several molecular and metabolic properties are involved in this prolongation of memory function. First, precursor cells of effector T cells have specific characteristics in their metabolic programs. In these cells, oxidative phosphorylation, rather than the glycolytic pathway (glycolysis) characteristic of effector T cells, is predominant, and this metabolic pathway has high energy efficiency and enables long-term survival of the cells. This metabolic property is an important factor supporting long-term maintenance of function as memory T cells. Next, the role of transcription factors is also important. In precursor cells of effector T cells, transcription factors such as T-bet and Eomes are expressed, and these promote differentiation into memory T cells while enabling their long-term survival and function. In addition, these precursor cells expressing surface markers such as CD62L and CCR7 have the ability to migrate to secondary lymphoid tissues. Through this homing function, the precursor cells receive survival signals necessary in secondary lymphoid tissues and contribute to maintenance of memory function. Furthermore, suppression of antigen stimulation is also involved in prolongation of memory function. Some precursor cells are released from antigen stimulation, whereby complete differentiation into effector T cells is suppressed, and instead, properties as memory T cells are retained. By this mechanism, an excessive effector response is suppressed, and at the same time, memory function is retained. In addition, the cytokine environment around the cells also plays an important role. In particular, cytokines such as IL-7 and IL-15 provide survival signals necessary for maintenance of memory T cells, and this greatly contributes to prolongation of memory function. Due to these factors, precursor cells of effector T cells survive for a long period of time compared with ordinary effector T cells and retain memory function that enables a rapid and strong secondary immune response. This property is considered to play an important role not only in immune defense against infectious diseases but also in cancer immunotherapy and vaccine development. Thus, understanding the properties of precursor cells of effector T cells has great significance in both basic immunology research and clinical applications.

In precursor cells of effector T cells, the phrase "a memory T cell phenotype is maintained" refers to a phenomenon in which, in the process of differentiation into effector T cells, some precursor cells exist while retaining phenotypes characteristic of memory T cells (for example: surface markers, metabolic states, expression of transcription factors, and the like). Such precursor cells survive even after completion of an immune response and have the ability to provide immune memory over a long period of time. This phenomenon is considered to be an important mechanism for achieving both the process in which T cells exert an effector function and the memory function that enables long-term survival and a rapid secondary immune response. The characteristics of this phenomenon will be described. First, precursor cells of effector T cells maintain surface markers characteristic of memory T cells. These include homing molecules such as CD62L and CCR7, and by expression of these molecules, the precursor cells retain the ability to migrate to secondary lymphoid tissues. Next, these precursor cells are also characterized by maintenance of their metabolic state. Specifically, oxidative phosphorylation is predominant over the glycolytic pathway, and long-term survival is enabled by maintaining a metabolic program with high energy efficiency. This metabolic property is an important factor for maintaining function as memory T cells. In addition, the precursor cells also maintain a characteristic balance in expression of transcription factors. Appropriate expression of transcription factors such as T-bet and Eomes plays a role in making differentiation into effector T cells compatible with maintenance of the memory T cell phenotype. Furthermore, the precursor cells have responsiveness to cytokine signals and can receive survival signals such as IL-7 and IL-15. This responsiveness enables the precursor cells to survive for a long period of time while retaining the properties of memory T cells. Finally, these precursor cells can be maintained in an antigen-independent manner. That is, precursor cells of memory T cells have the ability to retain their phenotype even in the absence of an antigen and maintain a state of readiness for a rapid immune response. Due to the above properties, maintenance of the memory T cell phenotype by precursor cells of effector T cells plays an extremely important role in the flexibility of the immune system and establishment of long-term immune memory. These properties are considered to have great significance in defense against infectious diseases, cancer immunotherapy, and vaccine design.

In the present specification, the term "antigen receptor" refers to any molecule that specifically binds to a target antigen and transmits a signal into a cell expressing the receptor. In the present disclosure, the antigen receptor can be introduced into T cells, and may be natural or an artificial molecule. For example, in addition to a T cell receptor (TCR) and a B cell receptor (BCR), a chimeric antigen receptor (CAR) is also encompassed.

In the present specification, the term "chimeric antigen receptor (CAR)" refers to a modified receptor that can impart antigen specificity to cells (for example, immune cells). CAR is also known as an artificial T cell receptor, a chimeric T cell receptor, or a chimeric immune receptor. CAR is a modified receptor that transfers antigen specificity to immune system cells (for example, T cells such as naive T cells, central memory T cells, effector memory T cells, or combinations thereof, NK cells, macrophages, and the like), and may include, for example, an antigen-specific targeting region, an extracellular domain, a transmembrane domain, a costimulatory domain, and/or an intracellular signaling domain. In addition, CAR may also include a CAR using a plurality of antigen-specific targeting regions (often two types) (bispecific CAR). Preferably, the CAR of the present disclosure includes at least one extracellular domain capable of binding to an antigen, at least one transmembrane domain, and at least one intracellular domain.

In the present specification, the term "T cell receptor (TCR)" refers to a receptor present in T cells. TCR is a heterodimeric receptor molecule consisting of two TCR polypeptide chains, and includes αβ-type TCR expressed by ordinary T cells and γδ-type TCR having special functions. The α- and β-chain TCR molecules form a complex with a plurality of CD3 molecules (CD3ζ chain, CD3ε chain, CD3γ chain, and CD3δ chain), transmit intracellular signals after antigen recognition, and initiate various immune responses. Endogenous antigens, such as viral antigens that have proliferated intracellularly in association with viral infection and cancer antigens derived from cancer cells, are presented as antigen peptides on MHC class I molecules. In addition, antigens derived from foreign microorganisms are taken up by antigen-presenting cells by endocytosis, and after being processed, are presented on MHC class II molecules. These antigens are recognized by TCRs expressed by CD8+ T cells or CD4+ T cells, respectively. It is also known that costimulatory molecules such as CD28, ICOS, and OX40 molecules are important for stimulation mediated by TCR molecules. With respect to the αβ-type TCR, which is one of the main focuses in the present specification, the respective gene products of α and β are considered to express specificity through unique combinations.

In the present specification, the term "glucose uptake ability" refers to the ability to take up glucose into cells per unit time. Uptake of glucose into cells can be evaluated by a method such as a 2-NBDG uptake assay.

In the present specification, the term "enhancement of glucose uptake ability" refers to improvement in glucose uptake ability by any method, and improvement in glucose uptake ability can be achieved by modifying immune cells (for example, T cells) so as to express one or more types of glucose transporters and the like, and/or modifying the immune cells so that expression of a glucose transporter is enhanced. Such genetically modified immune cells are expected to show high glucose uptake, for example, in a low-glucose environment (for example, in a tumor microenvironment). Therefore, immune cells co-expressing one or more types of glucose transporters and a chimeric receptor polypeptide may exhibit excellent physiological activity (for example, under a tumor microenvironment such as low-glucose conditions, optionally in the presence of a therapeutic antibody), such as cell proliferation, activation (for example, cytokine production, for example, increased production of IL-2 or IFNγ), cytotoxicity, and/or in vivo antitumor activity.

In the present specification, the term "glucose transporter" (GLUT) refers to a transporter that performs facilitated diffusion-type transport of glucose (dextrose). For GLUT, molecular species of GLUT1 to GLUT14 have been reported. Among them, for example, GLUT1 is expressed in a wide range of tissues and is responsible for constitutive baseline glucose transport, GLUT2 operates when sugar concentration increases and enhances glucose uptake, GLUT3 plays a central role in the central nervous system and exhibits a transport function even under low glucose concentrations, and GLUT4 operates in response to insulin stimulation.

In the present specification, the term "tumor microenvironment" is used interchangeably with "tumor environment", and the tumor microenvironment (TME) refers to a local biological environment surrounding tumor tissue and is a concept including various factors involved in tumor progression, proliferation, metastasis, drug resistance, and the like. This environment includes not only tumor cells but also non-tumor cells such as immune cells (for example, T cells and macrophages), fibroblasts, vascular endothelial cells, and adipocytes. In addition, humoral factors such as extracellular matrix (ECM), cytokines, chemokines, and growth factors are also important components. Furthermore, the tumor microenvironment is also affected by physical factors such as oxygen concentration, pH, and nutritional state. Intercellular interactions and changes in physical factors in the tumor microenvironment are considered to play an important role in tumor malignancy and therapeutic responsiveness. In particular, an immunosuppressive microenvironment has attracted attention as a factor that reduces the effects of immunotherapy. In the present invention, the term "tumor microenvironment" refers to a collection of biological and physical factors including these elements and interactions thereof, and is an important target in tumor therapy and diagnosis.

In the present specification, the term "tumor microenvironment conditions" means a tumor microenvironment or conditions equivalent thereto. Therefore, the "tumor microenvironment conditions" mean a broad concept including, in addition to conditions constituting a tumor microenvironment, similar or equivalent environmental conditions under which a tumor is placed in vivo. Specifically, the tumor microenvironment conditions include not only the local biological environment surrounding tumor tissue but also endogenous and exogenous elements that affect a tumor when the tumor is present in vivo. This environment includes interactions between tumor cells and non-tumor cells (for example, immune cells, fibroblasts, vascular endothelial cells, and the like), humoral factors such as extracellular matrix (ECM), cytokines, chemokines, and growth factors, and physical factors such as oxygen concentration, pH, nutritional state, and physical pressure. In addition, the "tumor microenvironment conditions" also include situations in which a tumor interacts with a broad physiological environment in vivo, and therefore are not limited to the local environment around the tumor. In the present invention, the "tumor microenvironment conditions" comprehensively represent broad environmental conditions encountered by a tumor in vivo, and include all factors that affect tumor progression and therapeutic responsiveness. Therefore, the tumor microenvironment conditions include the inside of a tumor, transmission of a TCR-related signal, the matrix and surroundings (outside), and conditions similar thereto.

In the present specification, the term "modification" broadly refers to any structural, functional, or expression-related change made to a gene (including a nucleic acid and a protein corresponding thereto), and includes naturally occurring or artificial changes. Specifically, the modification includes a sequence change by substitution, deletion, insertion, addition, or a combination thereof of a nucleotide sequence, a change in the amino acid sequence or properties of an encoded protein, or chemical modification for the purpose of adding or improving functionality (for example: glycosylation, phosphorylation, acetylation, and the like). In addition, the "modification" also includes gene introduction from the outside (for example: gene introduction via transfection or a viral vector), transformation, or knockout or knock-in of a specific gene using genome editing technology, suppression of gene expression (for example: suppression by RNA interference or CRISPR technology), and the like. Furthermore, epigenetic modification (for example: DNA methylation, histone modification, and the like) and modification of expression regulatory elements are also included. In the present specification, the term "modification" comprehensively refers to any form of change that affects the structure, expression, function, or biological behavior of a gene, and this includes not only endogenous changes but also changes by external introduction or manipulation.

In the present specification, the expression "in response to demand (on demand)" means that a target gene is expressed at a time when it is needed. Demand refers to, for example, a time when glucose is about to be depleted and the like (for example, when T cells infiltrate a low-glucose environment such as a tumor environment or bone marrow, when glucose consumption increases due to metabolic changes for supporting exertion of cytotoxic activity, cytokine production, division and proliferation, and the like by activation of T cells, or both), and the time period is arbitrary. For example, expression may occur only for several hours, several days, or several weeks, but is not limited thereto, and when a period in which there is demand is several days or more, such a case can also be included in the definition of on demand. The phrase "in response to demand (on demand)" may refer to a property in which expression of a glucose transporter is regulated in the T cells in response to a change in tumor microenvironment conditions, or may include a nucleic acid sequence encoding a glucose transporter, the nucleic acid sequence being operably linked to a tumor microenvironment-sensing element that senses a change in tumor microenvironment conditions. In some cases, a target gene is expressed in a cell without being incorporated into a genome of a host. In other cases, a target gene is incorporated into a genome of a host and expressed in a cell. As a method used for on-demand expression, a method known to those skilled in the art can be used, and transfection or transduction can be performed using a kit provided by a vendor. Selection may be performed to select cells capable of achieving on-demand expression, and for example, drug selection or magnetic bead selection well known to those skilled in the art may be used. When the constructed construct already has an on-demand property, the construct can also be used as it is. As an example, on demand is achieved by modification so that expression of a glucose transporter is regulated in the T cells in response to a change in tumor microenvironment conditions.

In the present specification, the term "tumor microenvironment conditions" refers to any conditions related to a tumor, and refers to conditions inside a tumor, in the matrix and surroundings (outside), and conditions similar thereto.

In the present specification, the phrase "expression of a glucose transporter is regulated (in cells such as T cells) in response to a change in tumor microenvironment conditions" means that, when tumor microenvironment conditions worsen (the cancer state worsens), expression or a level of the glucose transporter decreases or degradation is promoted, and when tumor microenvironment conditions become favorable (the healthy state improves), expression or a level of the glucose transporter is enhanced or degradation is reduced. Regulation of expression of a glucose transporter (in cells such as T cells) in response to a change in tumor microenvironment conditions can be achieved by modifying cells, such as by introducing such a gene construct.

Here, the phrase "tumor microenvironment conditions worsen" refers to a tumor microenvironment (TME) that promotes growth or progression of a tumor changing to a state more favorable for the tumor, and conditions equivalent thereto. This tumor microenvironment is composed of not only tumor cells but also surrounding blood vessels, immune cells, fibroblasts, extracellular matrix (ECM), signaling molecules, and the like. "Worsening" of this environment encompasses the following specific changes.

### 1) Progression of hypoxic state

As a tumor grows, blood vessels become insufficient and oxygen supply decreases, causing the surroundings of the tumor to become hypoxic. This state becomes a factor that promotes proliferation and metastasis of tumor cells. Therefore, by examining the oxygen state, worsening of tumor microenvironment conditions can be detected.

### 2) Activation of abnormal angiogenesis

Tumor growth promotes angiogenesis (formation of new blood vessels), but these new blood vessels are often structurally abnormal, and an imbalance in the supply of oxygen and nutrients promotes tumor progression. Therefore, by examining the state of angiogenesis, worsening of tumor microenvironment conditions can be detected.

### 3) Formation of immunosuppressive environment

A tumor suppresses antitumor immunity and reduces the ability of the immune system to eliminate the tumor by increasing immunosuppressive cells (such as regulatory T cells and myeloid-derived suppressor cells). Therefore, by examining the state of the immunosuppressive environment, worsening of tumor microenvironment conditions can be detected.

### 4) Progression of fibrosis

Activation of fibroblasts around a tumor and abnormal accumulation of extracellular matrix increase the migratory ability and drug resistance of tumor cells. Therefore, by examining the state of fibrosis, worsening of tumor microenvironment conditions can be detected.

### 5) Activation of chronic inflammation

Persistent chronic inflammatory responses in a tumor microenvironment promote proliferation, angiogenesis, and metastasis of tumor cells. Therefore, by examining the state of (chronic) inflammation, worsening of tumor microenvironment conditions can be detected.

### 6) Progression of metabolic abnormality

Tumor cells excessively consume glucose and activate anaerobic metabolism (Warburg effect). As a result, lactic acid accumulates, the microenvironment is acidified, and the functions of immune cells decrease. Therefore, by examining the state of metabolism (for example, the amount of glucose consumption, the amount of lactic acid accumulation, and the like), worsening of tumor microenvironment conditions can be detected.

The effects caused by worsening of tumor microenvironment conditions are diverse, and worsening of the tumor microenvironment affects tumor properties, including the following non-limiting examples: 1) promotion of tumor growth (tumor cells are activated, and division and proliferation are accelerated), 2) promotion of metastasis (tumor cells more easily spread to other organs via blood vessels or lymphatic vessels), 3) increase in treatment resistance (changes in the tumor microenvironment may make chemotherapy or immunotherapy less effective), and the like.

In the present specification, the phrase "modifying so that expression of a glucose transporter is regulated (in cells such as T cells) in response to a change in tumor microenvironment conditions" means that, in a cell, by newly introducing a glucose transporter or making a change to an existing glucose transporter, expression of the glucose transporter is regulated in the cell in response to a change in tumor microenvironment conditions. Such modification can be realized by operably linking, to a nucleic acid sequence encoding the glucose transporter, an element that enables expression or degradation of the glucose transporter to be regulated in response to a change in tumor microenvironment conditions in the cell. Alternatively, it may be achieved by making a change to an existing glucose transporter or an element that directly or indirectly regulates the same using genetic engineering techniques such as genetic manipulation or genome editing, so that expression or degradation of such a glucose transporter is regulated.

In the present specification, the term "TCR-related signal" refers to any signal related to a T cell receptor (TCR), and includes not only signals directly related to TCR (for example, a naturally expressed unmodified T cell receptor), but also chimeric antigen receptor (CAR)-related signals, which are complexes of a part of TCR and a costimulatory factor, expression of a tumor antigen (referring to an antigen recognized by TCR or CAR) or a costimulatory ligand, changes in cytokine conditions, and the like. For example, a TCR-related signal can be mediated by any element involved in TCR signal transduction, and may include a TCR signal or a part thereof, a chimeric signal of a TCR signal and another antigen (also referred to as a CAR signal), and the like.

In the present specification, the term "TCR signal domain" refers to any domain involved in a TCR signal, and representative examples thereof include immunoreceptor tyrosine-based activation motifs (ITAM) present in CD3 molecules (CD3ε, CD3δ, CD3γ) and the ζ chain (CD247, zeta chain) constituting a CR complex. ITAM has characteristics such as the presence of tyrosine residues (ITAM contains two tyrosine residues, and signal transduction is initiated when these are phosphorylated), recruitment of kinases (phosphorylation of tyrosine residues recruits signal transduction kinases (mainly Lck and ZAP-70), thereby activating a signaling cascade), and initiation of a signal transduction cascade (following phosphorylation of ITAM, calcium signaling, the MAPK pathway, the NF-κB pathway, and the like are activated, and immune responses such as T-cell proliferation, differentiation, and cytokine secretion are induced). Examples of the TCR signal domain include the CD3ζ chain (zeta chain; the CD3ζ chain is a major signal transduction molecule of the TCR complex and has three ITAMs, thereby enabling amplification of TCR signals), a CD3ε domain (epsilon domain; CD3ε has one ITAM and contributes to initial signal transduction via Lck after antigen recognition), a modified TCR signal domain (application in CAR-T cells), and chimeric antigen receptor (CAR) therapy (in which a TCR signal domain is artificially designed, and stronger signal transduction is achieved by combining costimulatory molecule domains such as CD28 and 4-1BB in addition to the ITAMs of the CD3ζ chain).

In the present specification, the term "tumor antigen" is also referred to as "cancer antigen", and refers to a molecule that is expressed by tumor cells and recognized by the immune system. Tumor antigens are mainly classified into those specifically expressed in tumor cells and those that are also expressed in normal cells but are abnormally highly expressed in tumor cells. This antigen is an important target in tumor immunity and is used in cancer immunotherapy (for example: vaccine therapy, T cell therapy, CAR-T therapy). Tumor antigens are mainly classified into the following two categories: tumor-specific antigens (TSA) and tumor-associated antigens (TAA). TSA is an antigen that is specifically expressed in tumor cells and is hardly expressed in normal cells. These are usually generated by the following causes: gene mutations (mutant peptides (neoantigens) generated by gene mutations such as RAS and p53), and virus-derived antigens (antigens expressed in tumors caused by viral infection (for example: E6/E7 antigens derived from human papillomavirus (HPV))). TAA is an antigen that is also expressed in normal cells but is abnormally highly expressed in tumor cells. The main characteristics are as follows: embryonic antigens (antigens that are expressed during the fetal period but are hardly expressed in adult tissues (for example: CEA, AFP)) and overexpressed antigens (antigens that are expressed at low levels in normal tissues but are expressed at high levels in tumor cells (for example: HER2, EGFR)). Examples of tumor antigens include the following. Examples of neoantigens (neoantigens, antigens generated by mutations in tumor cells): KRAS mutant peptide; examples of tumor virus-derived antigens (antigens expressed in virus-infected tumors): HPV-derived E6/E7 (cervical cancer); examples of embryonic antigens (expressed only during the fetal period in normal cells and silenced in adults): carcinoembryonic antigen (CEA), α-fetoprotein (AFP); examples of overexpressed antigens: HER2 (breast cancer), EGFR (lung cancer).

In the present specification, the term "co-stimulatory ligand" refers to a ligand that binds to a co-stimulatory molecule for complementing antigen presentation, which is a main signal in activation of T cells and other immune cells (recognition by a T cell receptor (TCR) or a B cell receptor (BCR)). Co-stimulatory ligands are expressed on the surfaces of antigen-presenting cells (APCs), tumor cells, and other cells, and play a role in enhancing or regulating immune responses. Interactions between these ligands and co-stimulatory molecules are important for T-cell activation, proliferation, cytokine secretion, and formation of immune memory, and contribute to appropriate regulation of immune responses. Co-stimulatory ligands are molecules that play important roles in immune responses and are involved in activation and regulation of immune cells, including T cells. As a characteristic thereof, first, co-stimulatory ligands provide a second signal following antigen recognition (first signal) via a T cell receptor (TCR), thereby enabling complete activation of T cells. In addition, expression of co-stimulatory ligands and interactions thereof have a role in regulating the intensity and nature of immune responses and maintain appropriate functions of the immune system. Furthermore, when co-stimulatory ligands are absent, T cells may become unresponsive (anergy) and immune tolerance may be induced, and therefore co-stimulatory ligands are also important for maintaining a balance between immune responses and immune tolerance.

Specific examples of co-stimulatory ligands include those that promote activation and those having an inhibitory role. Co-stimulatory ligands that promote activation include B7-1 (CD80) and B7-2 (CD86), and these bind to CD28 receptors on T cells to promote initial activation of T cells. Furthermore, OX40L (CD252) binds to OX40 (CD134) and promotes formation of immune memory by supporting proliferation and survival of T cells. 4-1BBL (CD137L) binds to 4-1BB (CD137) and supports proliferation and sustained activation of activated T cells, and ICOSL (CD275) binds to ICOS receptors and is involved in activation of helper T cells (Tfh cells) and B-cell help. On the other hand, inhibitory co-stimulatory ligands include Programmed Death-Ligand 1 (PD-L1) and PD-L2, and these bind to PD-1 receptors on T cells, thereby suppressing T-cell function and inducing immune tolerance. In addition, B7-H3 (CD276) and B7-H4 are also considered to have suppressive functions, and are considered to be involved in immunosuppression and induction of suppressive T cells. The application of co-stimulatory ligands has great potential in the fields of immunotherapy and disease treatment. In cancer immunotherapy, therapies using 4-1BBL, OX40L, and the like to enhance T-cell activation have been developed, and immune checkpoint inhibitors targeting inhibitory ligands such as PD-L1/PD-1 have become an important strategy in cancer treatment. Furthermore, in vaccine development, designs using adjuvants expressing co-stimulatory ligands to enhance immune responses are being advanced. In addition, in treatment of autoimmune diseases, research using inhibitory co-stimulatory ligands to control excessive immune responses is progressing.

In the present specification, the term "degree of transmission of a TCR-related signal" refers to the intensity or quality of signal transduction that occurs when a T cell receptor (TCR) recognizes a peptide-MHC complex of an antigen-presenting cell (APC). This degree greatly affects the results of immune responses such as T-cell activation, proliferation, cytokine production, differentiation, or apoptosis. The degree of transmission of a TCR-related signal is an important factor in determining T-cell activation and immune responses, and is regulated by various factors. Specifically, as the binding strength (affinity) between a peptide-MHC complex and TCR increases, the degree of signal transduction is enhanced. In addition, when the density of peptide-MHC complexes present on the surface of antigen-presenting cells is high, signal transduction via TCR is further enhanced. In addition, when ligands of co-stimulatory molecules such as CD28 and ICOS (such as B7 family molecules) are present, TCR signals are amplified, whereas when immunosuppressive molecules such as PD-1 and CTLA-4 are involved, signal transduction is suppressed. Furthermore, the phosphorylation state and expression level of signaling molecules downstream of TCR (such as Lck, ZAP-70, LAT, and SLP-76) are also important factors that affect signal intensity. In addition, metabolic environments such as cytokines, oxygen concentration, and glucose concentration also contribute to enhancement or suppression of signals. The degree of transmission of a TCR-related signal is extremely important in determining the quality of an immune response. For example, when the signal is strong, activation and cytotoxicity of effector T cells are induced, whereas a weak signal is involved in immune tolerance and induction of regulatory T cells (Treg). Using such properties, regulation of transmission of a TCR-related signal is applied to immunotherapy and disease treatment. In cancer immunotherapy, attempts have been made to induce T-cell responses that effectively attack cancer cells by increasing TCR signal intensity. In addition, in treatment of autoimmune diseases, approaches for controlling excessive immune responses by suppressing TCR signals are being studied. Furthermore, in T-cell engineering, when designing chimeric antigen receptors (CARs) or artificial TCRs, ensuring an appropriate degree of signal transduction is key to enhancing therapeutic efficacy and safety.

In the present specification, the term "degree of TCR stimulation" refers to the strength or quality of stimulation that occurs when a T cell receptor (TCR) recognizes a peptide-MHC complex on an antigen-presenting cell (APC) or target cell. The degree of this stimulation is an important factor that determines T-cell activation and the results of immune responses, and is defined by the intensity, duration, and frequency of signals.

In the present specification, the term "TCR co-stimulation" refers to additional signal transduction necessary to induce complete T-cell activation when T cells recognize a peptide-MHC complex on an antigen-presenting cell (APC) through a T cell receptor (TCR). With antigen recognition by TCR (first signal) alone, T cells are often incompletely activated or enter an unresponsive (anergy) state. Therefore, TCR co-stimulation is important for effectively inducing adaptive immune responses including T-cell proliferation, cytokine production, effector function, and formation of immune memory. TCR co-stimulation is provided by interaction between co-stimulatory molecules on the surface of T cells and co-stimulatory ligands on antigen-presenting cells. This amplifies TCR signals and regulates immune responses.

In the present specification, the term "humoral factor(s)" refers to soluble molecules that are present in body fluids and are involved in intercellular communication and maintenance of homeostasis in a living body. These factors are involved in various physiological processes, such as immune responses, inflammatory responses, tissue repair, and metabolic regulation, and mainly act in body fluids such as blood, lymph fluid, and tissue fluid. Humoral factors mainly play important roles in the immune system, and representative examples include molecules such as cytokines and complement components. These molecules bind to receptors on cell surfaces to initiate signal transduction and regulate responses of target cells.

In the present specification, the term "transcription factors that translocate into the nucleus in association with activation of T cells" refers to transcription factors that are activated and translocate into the nucleus as a result of initiation of a series of signal transduction cascades when T cells recognize a peptide-MHC complex presented by an antigen-presenting cell (APC) through a T cell receptor (TCR). These transcription factors play important roles in activation, proliferation, differentiation, and expression of effector functions of T cells. For example, major transcription factors that translocate into the nucleus in association with activation of T cells include NFAT, NF-κB, AP-1, and the STAT family. Nuclear factor of activated T-cells (NFAT) is activated by calcium signaling, translocates into the nucleus after dephosphorylation by calcineurin, and promotes expression of interleukin-2 (IL-2) and other cytokine genes. Meanwhile, NF-κB (Nuclear Factor kappa-light-chain-enhancer of activated B cells) is activated through IκB degradation by TCR signaling and controls transcription of genes related to inflammatory cytokines and cell proliferation. In addition, Activator Protein-1 (AP-1) is activated by MAPK signaling pathways such as ERK and JNK and contributes to expression of IL-2 and interferon γ (IFN-γ). Furthermore, the STAT family (Signal Transducer and Activator of Transcription) (including at least seven types: STAT1-4, STAT5A, STAT5B, and STAT56) is activated by signal transduction through cytokine receptors. For example, STAT3 is activated through IL-6 and IL-10 signals and regulates expression of genes related to inflammatory responses and immunosuppression. STAT5 is activated in response to signals from IL-2 and IL-7 and plays an important role in proliferation and survival of T cells. As described above, NFAT, NF-κB, AP-1, and the STAT family translocate into the nucleus through different pathways and function cooperatively in regulation of T cell responses.

In the present specification, the term "degree of oxygen concentration" is an index indicating the amount of oxygen present in a specific environment or tissue, and is usually measured as oxygen partial pressure (unit: mmHg) or oxygen saturation (%). In vivo, oxygen concentration is an important factor that directly affects metabolic activities of cells and tissues, and is broadly classified into four main states, including a normoxic state (normoxia), a hypoxic state (hypoxia), and a hyperoxic state (hyperoxia). Each state will be described below. First, a normoxic state (normoxia) refers to a state in which oxygen partial pressure is within a normal physiological range, and generally, the oxygen concentration is about 21%, and the oxygen partial pressure is about 100 mmHg under atmospheric pressure. This state is a typical oxygen concentration observed in healthy tissues and organs, and, for example, oxygen partial pressure in arterial blood is usually 80 to 100 mmHg. Within this range, cells efficiently produce energy, and physiological functions are normally maintained. On the other hand, a hypoxic state (hypoxia) refers to a state in which oxygen partial pressure is below the normal range. For example, in a tumor microenvironment, oxygen supply may be insufficient, and oxygen partial pressure may decrease to 20 mmHg or less. Such a hypoxic state promotes tumor growth and metastasis and also causes resistance to radiation therapy. In addition, it is known that a hypoxic state also occurs at high altitudes (3,000 meters or more above sea level) and causes acute mountain sickness. Next, a hyperoxic state (hyperoxia) is a state in which oxygen partial pressure exceeds the normal range. In hyperbaric oxygen therapy, this hyperoxic state is utilized, and oxygen supply to tissues is increased by increasing oxygen partial pressure. This method may be used for wound healing and treatment of infectious diseases, and, as a specific example, an effect is expected in treatment of diabetic foot ulcers. Finally, an extreme hypoxic state (an oxygen-free state, anoxia) refers to a state in which little or no oxygen is present. For example, in the case of cardiac arrest or severe ischemia (insufficient blood flow), oxygen supply is completely interrupted, and therefore tissues are deprived of oxygen, ultimately causing necrosis to progress. Such an extreme oxygen-deficient state has a serious effect on life. As described above, the degree of oxygen concentration is deeply involved in physiological and pathological processes, and understanding how each state affects functions in the body and progression of diseases is important for effective treatment and management.

In one embodiment, the term "tumor microenvironment condition-sensing elements" refers to elements that sense changes in tumor microenvironment conditions, and refers to molecules or structures that sense specific conditions or stimuli in a tumor microenvironment (TME) (for example: a hypoxic state, acidification, nutrient deficiency, inflammatory cytokines, changes in metabolites, and the like) and transmit that information to cells. These elements function as molecular mechanisms by which tumor cells, surrounding immune cells, fibroblasts, vascular endothelial cells, and the like adapt to the tumor microenvironment and contribute to tumor progression, metastasis, and further treatment resistance. Condition-sensing elements in a tumor microenvironment play a role in sensing specific environmental conditions and inducing adaptive responses of tumor cells in response thereto. Examples of main conditions and sensing elements corresponding thereto are shown below.

First, in an oxygen state (hypoxia), Hypoxia-Inducible Factor (HIF) functions as a sensing element. This element senses a hypoxic environment and induces tumor cell metabolism and expression of an angiogenic factor (VEGF). In particular, HIF-1α plays a central role for tumor cells to adapt to a hypoxic state. Next, in acidification (decrease in pH), pH sensors such as proton-sensitive ion channels and carbonic anhydrase IX (CAIX) function as sensing elements. These sense an acidic environment in a tumor and promote survival of tumor cells by regulating the pH balance inside and outside the cells. Under conditions of nutrient deficiency (deficiency of glucose or amino acids), the mTOR pathway and AMPK (AMP-activated protein kinase) function as sensing elements. These sense nutrient deficiency and play a role in maintaining cell survival by regulating energy metabolism. For inflammatory cytokines (for example: IL-6, TNF-α), cytokine receptors (for example: IL-6R, TNF receptor) and the NF-κB signaling pathway act as sensing elements. These sense an inflammatory environment, promote proliferation of tumor cells, and induce expression of immunosuppressive factors. In addition, in changes in metabolites (for example: accumulation of lactic acid), monocarboxylate transporters (MCTs) function as sensing elements. This enables sensing of metabolites such as lactic acid and promotes survival and spread of tumor cells. In addition, in response to oxidative stress (increase in ROS), Nuclear factor erythroid 2-related factor 2 (NRF2) functions as a sensing element. NRF2 senses reactive oxygen species (ROS) and plays a role in protecting tumor cells by activating antioxidant responses. These condition-sensing elements provide important mechanisms for tumor cells to adapt to the microenvironment and are deeply involved in tumor progression and treatment resistance.

In the present specification, a nucleic acid sequence encoding a glucose transporter, the nucleic acid sequence being operably linked to a tumor microenvironment condition-sensing element having such a configuration and sensing a change in tumor microenvironment conditions, plays a role in responding to a change in tumor microenvironment conditions and regulating glucose transport by including the following important components. First, the sequence of the sensing element has a function of responding to specific conditions of the tumor microenvironment (for example: hypoxia, acidification, nutrient deficiency). Specifically, the sequence includes a portion encoding an HIF-binding domain that responds to a hypoxic state and a protein region that functions as a pH sensor sensing acidification. This sequence makes it possible to detect specific changes in the tumor microenvironment. Next, the sequence of the glucose transporter encodes a glucose transporter (for example: GLUT1, GLUT3) that plays a role in transporting glucose across a cell membrane. This sequence enables glucose transport between the inside and outside of cells to be performed efficiently and satisfies the metabolic demand of tumor cells. Furthermore, the sequence of the linking region encodes a linker region that functionally links the sensing element and the glucose transporter. This linker region provides flexibility and stability and enables the sensing element and the glucose transporter to operate cooperatively. However, by targeting the function of the protein encoded by this nucleic acid sequence, metabolic adaptation of tumor cells can be blocked. Specifically, for inhibiting tumor adaptation, energy supply to tumor cells is cut off by sensing changes in the tumor microenvironment, such as a hypoxic state or acidification, and suppressing glucose transport based thereon. In addition, for blocking environmental responsiveness, by preventing detection of changes in the tumor microenvironment via the sensing element, tumor cells are made unable to adapt under harsh environmental conditions. By such functional inhibition, a therapeutic strategy that effectively suppresses survival and growth of tumor cells can be realized.

Examples of the tumor microenvironment-sensing element include, but are not limited to, (i) an inducible activation motif (HIH-RE, NFAT-RE, and the like) that is activated in response to a change (worsening or the like) in tumor microenvironment conditions, and (ii) a nucleic acid sequence encoding a degradation factor (ODD and the like) for the glucose transporter that is inactivated in response to the change (worsening or the like) in the tumor microenvironment conditions.

In the present specification, the term "inducible activation motif responsive to tumor microenvironmental changes" refers to a molecular motif that controls signal transduction that is selectively activated in response to changes in specific conditions of a tumor microenvironment (TME) (for example: a hypoxic state, acidification, nutrient deficiency, inflammatory signals, an increase in oxidative stress, and the like). This motif is involved in regulation of metabolism, growth, and immune responses in tumor cells and cells surrounding a tumor, and affects tumor progression, metastasis, and treatment resistance. The inducible activation motif functions in a specific process of an intracellular signal transduction pathway and changes gene expression or metabolic pathways in response to worsening of tumor microenvironment conditions (for example: progression of hypoxia or nutrient deficiency). This enhances the ability of tumor cells to adapt to harsh environmental conditions.

In the present specification, "nuclear translocation factor-binding inducible activation motif to which a nuclear translocation factor that translocates into the nucleus in response to a decrease in oxygen concentration binds (Hypoxia-Responsive Nuclear Translocation and Activation Motif)" refers to a DNA sequence or protein motif to which a specific factor (for example: HIF-1α) that is activated and translocates into the nucleus in response to a decrease in oxygen concentration (a hypoxic state, hypoxia) binds. This motif functions as a molecular switch that regulates gene expression in a hypoxic state and plays an important role for tumor cells and normal cells to adapt to a hypoxic environment. This inducible activation motif controls transcription of hypoxia-responsive genes (for example: angiogenic factors, metabolic enzymes, and oxidative stress tolerance factors), enhances the adaptive ability of cells, and is also deeply involved in cancer progression and treatment resistance.

In the present specification, the term "inducible activation motif to which a nuclear translocation factor that translocates into the nucleus in response to activation of T cells binds (Inducible Activation Motif Bound by Nuclear Translocation Factors in Response to T Cell Activation)" refers to a DNA sequence or protein domain to which a nuclear translocation factor (for example: NFAT, NF-κB, AP-1, and the like) activated by T cell receptor (TCR) stimulation or signal transduction of co-stimulatory molecules specifically binds. This motif has a role in receiving signals associated with T cell activation in the nucleus and inducing gene expression and transcriptional regulation. The inducible activation motif functions as a central molecular mechanism that controls T cell proliferation, differentiation, cytokine production, and expression of effector functions.

In the present specification, the term "tumor microenvironment-responsive inactivation or degradation factor of the glucose transporter (Degradation Factors of the Glucose Transporter Responsive to Tumor Microenvironmental Changes)" refers to a molecule or motif that plays a role in inactivating or degrading a glucose transporter (GLUT) in response to changes in specific conditions in a tumor microenvironment (TME) (for example: hypoxia, acidification, nutrient deficiency, oxidative stress, and the like). These degradation factors play a role in suppressing metabolic activity of tumor cells and inhibiting tumor growth and progression, particularly by controlling excessive glucose transport and metabolic adaptation. Specific examples include an oxygen-dependent degradation domain (ODD), and these regulate the stability of a target protein depending on environmental conditions.

In the present specification, the term "nuclear translocation factor-binding inducible activation motif to which a nuclear translocation factor that translocates into the nucleus in response to activation of T cells binds (Inducible Activation Motif Bound by Nuclear Translocation Factors in Response to T Cell Activation)" refers to a DNA sequence or protein motif to which a specific transcription factor (for example: NFAT, NF-κB, AP-1, and the like) that is activated in response to T cell receptor (TCR) stimulation or signal transduction by co-stimulatory molecules and translocates into the nucleus binds. This motif controls gene expression related to T cell activation and plays a role in regulating T cell proliferation, differentiation, cytokine production, and expression of effector functions. This inducible activation motif is a central molecular mechanism that regulates immune responses and is an important transcriptional regulatory element in T cell responses. It mainly has the following components. First, the motif includes a nuclear translocation factor-binding site. Transcription factors that translocate into the nucleus upon activation of T cells (for example: NFAT, NF-κB, AP-1) specifically bind to this site and translate the signals into gene transcription. Second, the motif includes an environment-responsive region, which is dynamically activated in response to TCR stimulation or signals from co-stimulatory molecules. This region has a property of not being activated in a resting state. Third, the motif has a transcriptional regulatory function. The motif promotes or suppresses transcription of target genes through binding of nuclear translocation factors. This motif has diverse functions. First, the motif induces gene expression. The motif regulates gene expression of cytokines such as IL-2, IFN-γ, and TNF-α and effector molecules. Next, the motif regulates immune responses, and determines the quality and intensity of adaptive immune responses by controlling T cell proliferation, differentiation, and effector functions. In addition, the motif is involved in promotion or suppression of inflammatory responses and formation of immune memory, and regulates the function of the entire immune system. Specific examples include an NFAT-binding motif present in the IL-2 promoter region, an NF-κB response element in the TNF-α promoter region, and a region to which AP-1 activated via the MAPK pathway binds. These motifs each regulate important gene expression associated with T cell activation. Various applications are contemplated for the present motif. In cancer immunotherapy and treatment of autoimmune diseases, development of drugs targeting this inducible activation motif is expected. In addition, in gene therapy and synthetic biology, it is also possible to design an artificial system that regulates gene expression under T cell-specific conditions using this motif. Furthermore, analysis of the activation state of the motif is also useful for monitoring immune responses and evaluating therapeutic effects. Examples of such motifs include, but are not limited to, an NFAT-binding domain, a κB motif (GGGACTTTCC (SEQ ID NO: 1)), a STAT-binding sequence (TTCNNNGAA), or modified sequences thereof.

In the present specification, the term "nuclear translocation factor" is a generic term for molecules that are activated in response to extracellular stimuli or intracellular signals and have a function of regulating transcription of target genes by translocating from the cytoplasm into the nucleus. These factors are activated by specific signal transduction pathways, pass through the nuclear membrane via a nuclear localization signal (NLS), and play a role in regulating gene expression by binding to DNA sequences or other transcription factors. Examples of nuclear translocation factors that may be used in the present specification include NFAT, NF-κB (nuclear factor-kappaB), and the STAT transcription factor family (seven types: STAT1-4, STAT5A, STAT5B, and STAT56).

In the present specification, the term "combination of nuclear translocation factors and nuclear translocation factor-binding inducible activation motifs (Combination of Nuclear Translocation Factors and Nuclear Translocation-Inducible Activation Motifs)" refers to a combination of transcription factors (nuclear translocation factors) that are activated in response to extracellular or intracellular stimuli (for example: activation of T cells, a hypoxic state, inflammatory signals, and the like) and translocate into the nucleus, and DNA sequences or protein motifs (nuclear translocation factor-binding inducible activation motifs) to which the transcription factors specifically bind. This combination plays a role in precisely regulating gene expression in response to specific stimuli and controlling the function and adaptation of cells.

In the present specification, this combination is responsible for coordination between signal transduction pathways and gene expression control, and is important in biological processes such as immune responses, inflammation, metabolic regulation, and cell differentiation. It is also known to play pathological roles in various pathologies including cancer and autoimmune diseases. Examples of main combinations are as follows.
NFAT and NFAT-binding motif of IL-2 promoter
Nuclear translocation factor: NFAT (Nuclear Factor of Activated T-cells)
Binding motif: NFAT response element in IL-2 promoter
Function: In response to activation of T cells, NFAT translocates into the nucleus, induces transcription of the IL-2 gene, and promotes T cell proliferation and immune responses.
NF-κB and NF-κB response motif of TNF-α promoter
Nuclear translocation factor: NF-κB (Nuclear Factor kappa-light-chain-enhancer of activated B cells)
Binding motif: NF-κB response element in TNF-α promoter (for example, κB motif (GGGACTTTCC))
Function: In response to inflammatory signals (for example: cytokine stimulation), NF-κB translocates into the nucleus, induces expression of TNF-α, and regulates inflammatory responses.
HIF-1α and HRE (hypoxia-responsive element)
Nuclear translocation factor: HIF-1α (Hypoxia-Inducible Factor-1α)
Binding motif: HRE (Hypoxia-Responsive Element)
Function: In response to a hypoxic state, HIF-1α translocates into the nucleus and induces transcription of angiogenic factors (for example: VEGF).
STAT family and cytokine response element
Nuclear translocation factor: STAT3 (Signal Transducer and Activator of Transcription 3)
Binding motif: STAT-binding site in a cytokine-responsive gene (for example, STAT-binding sequence (TTCNNNGAA))
Function: In response to cytokine (for example: IL-6) stimulation, STAT3 translocates into the nucleus and induces immunoregulation and inflammatory responses.
AP-1 and growth factor response motif
Nuclear translocation factor: AP-1 (Activator Protein-1)
Binding motif: AP-1 response element in promoter of gene related to cell proliferation or differentiation
Function: AP-1 activated via the MAPK pathway translocates into the nucleus and promotes cell proliferation and differentiation.

In the present specification, the term "factor whose activity of degrading a glucose transporter is inactivated in response to a decrease in oxygen concentration (Factors That Inactivate the Degradation Activity of Glucose Transporters in Response to Hypoxia)" refers to a factor having a role of suppressing the activity of a system that induces degradation of a glucose transporter (for example: GLUT1, GLUT3) in response to a decrease in oxygen concentration (a hypoxic state, hypoxia), and increasing the stability of the glucose transporter. This factor acts through a structure such as an oxygen-dependent degradation domain (ODD) to suppress degradation of a glucose transporter under hypoxic conditions, such that cells can maintain necessary energy supply. In addition, in a tumor microenvironment, this factor also plays an important role in promoting metabolic adaptation of tumor cells and contributing to survival and proliferation. The following is a description of components and properties of a factor whose activity of degrading a glucose transporter is inactivated in response to a decrease in oxygen concentration. First, a hypoxia-responsive sensor region can be mentioned. This region has a role of sensing a decrease in oxygen concentration and emits a degradation-suppressing signal under a hypoxic state. A representative example is Hypoxia-Inducible Factor-1α (HIF-1α), which is stabilized under a hypoxic state and plays a role in regulating the activity of a degradation factor. Next, an oxygen-dependent degradation domain (ODD) is mentioned. This region has a property of regulating degradation depending on oxygen concentration. Usually, when the oxygen concentration is high, ubiquitination is induced via this region, and degradation proceeds. On the other hand, in a hypoxic state, degradation activity is inactivated, and the stability of the glucose transporter is increased. In addition, a degradation-suppressing factor is also an important component. This factor plays a role in suppressing degradation activity by ubiquitination or the proteasome system and helping stabilize the glucose transporter. Specific examples include regulation of the activity of pVHL (von Hippel-Lindau protein). In addition, transcriptional and translational regulatory functions are also included. This factor plays a role in preventing degradation and ensuring stability at the cell membrane by regulating transcription and translation of the glucose transporter under hypoxic conditions. These components cooperate with each other and play an important role in enabling suppression of degradation activity in response to changes in oxygen concentration and allowing cells to maintain energy supply even under a hypoxic environment.

As described above, the phrase "on-demand expression and/or enhancement is achieved specifically in a tumor environment (On-demand Expression and/or Enhancement Specifically Achieved in the Tumor Microenvironment)" refers to a phenomenon in which expression of a specific gene or protein and/or enhancement of the function thereof is induced in response to characteristic conditions of a tumor microenvironment (TME) (for example: hypoxia, acidification, nutrient deficiency, presence of inflammatory cytokines, and the like), and is controlled so that expression in normal tissues other than tumors is minimal or absent. This phenomenon is important in developing tumor-specific therapeutic targets and designing therapeutic strategies with reduced side effects. A tumor environment-specific regulatory mechanism makes it possible to enhance therapeutic effects while minimizing effects on normal tissues. The main characteristics of the phenomenon in which on-demand expression and/or enhancement is achieved specifically in a tumor environment will be described below. First, one characteristic of this phenomenon is environment-dependent regulation. This refers to a mechanism in which expression of a gene or protein is induced or enhanced in response to specific conditions of a tumor microenvironment, such as a hypoxic state, acidic pH, nutrient deficiency, or inflammatory signals. A specific example includes a case in which gene expression is induced under a hypoxic environment by HIF-1 (hypoxia-responsive factor). Next, this phenomenon is characterized by tumor specificity. This indicates a property in which these genes or proteins are hardly expressed or are expressed only at an extremely low level in normal tissues other than tumors, whereas their expression or function is specifically activated in a tumor microenvironment. Furthermore, on-demand properties are also important characteristics. Expression or enhancement is regulated as necessary at a timing when specific conditions, such as a decrease in oxygen concentration or an increase in inflammatory cytokines, are met due to changes in a tumor environment. This property enables a mechanism in which functions are efficiently exerted only in a tumor environment. Finally, this phenomenon has suitability as a therapeutic target. Genes or proteins that are expressed or enhanced specifically in tumors can be used as targets for cancer therapy. By utilizing this property, it is possible to design a therapeutic strategy that enhances therapeutic effects on tumors while minimizing side effects on normal tissues. Due to the above characteristics, a mechanism in which on-demand expression and/or enhancement is achieved specifically in a tumor environment is a very promising element in new approaches to cancer therapy. By utilizing these properties, development of more effective therapies with high safety is expected.

In the present specification, with respect to a gene (nucleic acid, protein, and the like), the term "modification" refers to inserting a nucleotide sequence of a nucleic acid in a cell, changing a nucleotide sequence of a nucleic acid, partially deleting a nucleotide sequence of a nucleic acid in a cell, or a combination thereof.

In the present specification, the "disease" is interpreted in the broadest sense, refers to a state in which discomfort or inconvenience has occurred in the mind or body of a human being or an animal, and refers to any state that cannot be said to be a healthy state, such as a disease, a disorder, and various symptoms, even if not specifically defined. Diseases that can be targeted by the present disclosure include, but are not limited to, diseases to which an immune response may be related, for example, cancer, autoimmune diseases, allergies, and infectious diseases.

In the present specification, the phrase "a subject has an immune response" to a certain component or substance means that some immune reaction occurs against the component or substance. In the subject or a biological component derived from the subject (for example, cells and the like), this can be determined by observing changes in various immune cells or increases and decreases in immune-related substances (for example, cytokines and the like), can be judged by an objective index, and can also be subjectively judged based on the experience of a physician or the like.

In the present specification, whether a subject "has immune memory" with respect to a certain component or substance can be evaluated by measuring, in the subject or a biological component derived from the subject (for example, cells and the like), whether the component or substance (i) antigen-dependently enhances cytokine production in memory CD4-positive T cells or has a proliferation-promoting action, (ii) changes expression of surface antigens of memory-type regulatory T cells, (iii) changes the ratio of Treg to Th1, (iv) induces IFN-γ production from T-bet-positive Th1 cells, (v) changes IFN-γ production ability, (vi) changes IL-2 production ability, (vii) changes TNF-α production ability, or (viii) whether the subject has an antibody specific to the component or substance in blood, and confirming that at least one of these produces a positive result.

In the present specification, the "infectious disease" may be any infectious disease, including any type of infectious disease such as a viral infectious disease (including any viral form such as a single-stranded or double-stranded DNA virus or RNA virus), a bacterial infectious disease, a protozoal infectious disease, or a mycoplasma infectious disease, and may be, for example, tuberculosis, coronavirus, malaria, yellow fever virus, smallpox virus, vaccinia, measles/rubella, polio, epidemic parotitis/MUMPS, rotavirus infection, chicken pox, yellow fever, Ebola, West Nile Fever, Hib infection, pneumococcus infection, whooping cough, Japanese encephalitis, meningococcal infection, salmonella infection, pathogenic E. coli, toxoplasma, zika virus, herpes virus type 1, EBV/Epstein-Barr virus (herpes virus type 4), CMV/cytomegalovirus (herpes virus type 5), influenza, MARS, rabies, and diphtheria.

In the present specification, the term "immune abnormality" refers to any disease, disorder, or condition caused or suspected to be caused at least in part by an abnormality of the immune system. The immune abnormality refers to a state in which an abnormality occurs in immunity due to some cause, and the subject becomes susceptible to infectious diseases or an allergic reaction occurs. Examples of immune abnormalities include, but are not limited to, allergies and autoimmune diseases. When directed against self-antigens, it is generally referred to as an autoimmune disease, and when directed against external antigens, it is referred to as an allergy. When the immune response is strong, it can be said that a state of autoimmune disease occurs against self-antigens and a state of allergy occurs against non-self antigens, whereas when the immune response is weak, it can be said that a state of cancer occurs against self-antigens and an infectious disease occurs against non-self antigens.

In the present specification, the term "autoimmune disease" refers to an immune reaction occurring excessively against a specific self-antigen. An autoimmune disease can be said to be a disease caused by a breakdown of immune tolerance, in which the immune system, which has a role of recognizing and eliminating foreign substances, excessively reacts against and attacks even one's own normal cells and tissues, thereby causing symptoms. Examples of autoimmune diseases or disorders include, but are not limited to, the following: inflammatory responses such as inflammatory skin diseases including psoriasis and dermatitis (for example, atopic dermatitis); systemic scleroderma and sclerosis; reactions associated with inflammatory bowel disease (for example, Crohn's disease and ulcerative colitis); respiratory distress syndrome (including adult respiratory distress syndrome: ARDS); dermatitis; meningitis; encephalitis; uveitis; colitis; glomerulonephritis; allergic conditions such as eczema and asthma, and other conditions involving T cell infiltration and chronic inflammatory responses; atherosclerosis; leukocyte adhesion deficiency; rheumatoid arthritis; systemic lupus erythematosus (SLE) (including, but not limited to, lupus nephritis and cutaneous lupus); diabetes (for example, type I diabetes or insulin-dependent diabetes); multiple sclerosis; Raynaud's syndrome; autoimmune thyroiditis; Hashimoto's thyroiditis; allergic encephalomyelitis; Sjögren's syndrome; juvenile-onset diabetes; immune reactions associated with acute and delayed-type hypersensitivity mediated by cytokines and T lymphocytes typically observed in tuberculosis, sarcoidosis, polymyositis, granulomatosis, and vasculitis; pernicious anemia (Addison's disease); diseases involving leukocyte leakage; central nervous system (CNS) inflammatory disorders; multiple organ injury syndrome; hemolytic anemia (including, but not limited to, cryoglobulinemia or Coombs-positive anemia); myasthenia gravis; antigen-antibody complex-mediated diseases; anti-glomerular basement membrane disease; antiphospholipid syndrome; allergic neuritis; Graves' disease; Lambert-Eaton myasthenic syndrome; bullous pemphigoid; pemphigus; autoimmune polyendocrine disorder; Reiter's disease; stiff-man syndrome; Behçet's disease; giant cell arteritis; immune complex nephritis; IgA nephropathy; IgM polyneuropathy; immune thrombocytopenic purpura (ITP), autoimmune uveitis, or autoimmune thrombocytopenia.

In the present specification, the term "allergy" refers to an immune reaction occurring excessively against a specific non-self antigen, and is a disease in which an immune reaction occurs against an "allergen". An "allergen" refers to an antigen that can react with an antibody of a subject having an allergic disease. Examples thereof include, but are not limited to, allergens derived from pollen of trees (acacia, alder, velvet ash, European beech, birch, maple, mountain cedar, red cedar, poplar, Japanese cypress, American elm, Chinese elm, Douglas fir, rubber tree, eucalyptus tree, hackberry, hickory, American linden, sugar maple, mesquite, paper mulberry, Quercus, olive, pecan, pepper, pine, privet, Russian olive, American sycamore, tree of heaven, black walnut, black willow, and the like), allergens derived from pollen of grasses and plants (cotton, Bermuda grass, Kentucky bluegrass, brome, corn, meadow fescue, Johnson grass, oat, orchard grass, redtop, ryegrass, rice, sweet vernal grass, timothy, amaranth, goosefoot, cocklebur, dock, goldenrod, kochia, white goosefoot, marigold, nettle, barnyard grass, plantain, giant ragweed, ragweed, false ragweed, Russian thistle, mugwort, broom, sheep sorrel, and the like), insect-derived allergens (silkworms, mites, honeybees, wasps, ants, cockroaches, and the like), fungus-derived allergens (Alternaria, Aspergillus, Botulinus, Candida, Cephalosporium, Curvularia, Epicoccum, Epidermophyton, Fusarium, Helminthosporium, Cladosporium herbarum, Mucor, Penicillium, Phoma, Pullularia pullulans, Rhizopus, and the like), allergens derived from animal hair (dogs, cats, birds, and the like), allergen proteins derived from house dust, food-derived allergens (OVA and the like), and the like. Representative diseases of "allergy" include atopic dermatitis, allergic rhinitis (pollinosis and the like), allergic conjunctivitis, allergic gastroenteritis, bronchial asthma, childhood asthma, food allergy, drug allergy, urticaria, and the like.

### (Preferred embodiments)

Preferred embodiments of the present disclosure will be described below. The embodiments provided below are provided for a better understanding of the present disclosure, and the scope of the present disclosure should not be limited to the following description. Therefore, it is apparent that those skilled in the art can appropriately make modifications within the scope of the present disclosure in view of the description in the present specification. In addition, the following embodiments of the present disclosure can be used alone or in combination.

In one aspect, the present disclosure provides immune cells such as T cells with enhanced glucose uptake ability on demand, a cell population containing such cells, a pharmaceutical composition containing such cells or the cell population, and other related technologies. As such immune cells such as T cells with enhanced glucose uptake ability on demand and a cell population containing such cells, T cells with enhanced glucose uptake ability are typically used, the T cells having, or being modified to have, a property in which expression of a glucose transporter is regulated in the T cells in response to a change in tumor microenvironment conditions, or containing a nucleic acid sequence encoding a glucose transporter, the nucleic acid sequence being operably linked to a tumor microenvironment-sensing element that senses a change in tumor microenvironment conditions.

In another aspect, the present disclosure provides immune cells such as T cells with enhanced glucose uptake ability, in which expression of a glucose transporter is regulated in the T cells in response to a change in tumor microenvironment conditions, a cell population containing such cells, a pharmaceutical composition containing such cells or the cell population, and other related technologies.

In still another aspect, the present disclosure provides a T cell containing a nucleic acid sequence encoding a glucose transporter, the nucleic acid sequence being operably linked to a tumor microenvironment-sensing element that senses a change in tumor microenvironment conditions.

In one aspect of the present disclosure, provided is a T cell with enhanced glucose uptake ability (T_{eff}), in which the T cell is modified to express a glucose transporter on demand, and/or has enhanced expression of a glucose transporter on demand. A T cell modified so that a glucose transporter is expressed on demand and/or expression of a glucose transporter is enhanced on demand, a T cell in which expression of a glucose transporter is regulated in the T cell in response to a change in tumor microenvironment conditions, or a T cell containing a nucleic acid sequence encoding a glucose transporter, the nucleic acid sequence being operably linked to a tumor microenvironment-sensing element that senses a change in tumor microenvironment conditions, has not been conventionally contemplated. In a T cell in which glucose uptake ability is constitutively enhanced, differentiation into an effector memory-type T cell and exhaustion were observed, and marked T-cell death was induced when the T cell was activated under no- to low-glucose conditions. In contrast, a T cell in which expression of a glucose transporter is enhanced on demand is remarkable in that, while equivalent cytotoxic activity is maintained, T-cell death under no- to low-glucose conditions is avoided, and maintenance of a memory phenotype and exhaustion resistance, which are expected to maintain an effect for a longer period, are induced. It is advantageous that the cells of the present disclosure have enhanced glucose uptake ability. Such cells in which glucose uptake ability is enhanced on demand exert remarkable effects on diseases such as cancer, autoimmune diseases, allergies, and infectious diseases when applied in the form of CAR-T cells, TCR-T cells, and the like.

In addition, the cells of the present disclosure exhibited a remarkably high recurrence-preventing effect when used in CAR-T therapy and the like. In addition, sudden death of mice due to toxicity caused by non-tumor factors, which was observed in T cells in which glucose was constitutively enhanced, was avoided in cells in which a glucose transporter is expressed or expression of a glucose transporter is enhanced on demand, cells in which expression of a glucose transporter is regulated in the T cells in response to a change in tumor microenvironment conditions, or cells containing a nucleic acid sequence encoding a glucose transporter, the nucleic acid sequence being operably linked to a tumor microenvironment-sensing element that senses a change in tumor microenvironment conditions. These findings were unexpected, and without being bound by theory, they may be explained by avoidance of host toxicity caused by excessive T-cell activation or excessive glucose uptake, a marked decrease in exhaustion, nutrient supply matching nutritional demand, suppression of cell death caused by appropriate activation, induction of memory formation, and the like. In addition, the cells of the present disclosure preferably have an effector function after being introduced into a place where they are to function, particularly into the body. Examples of such cells include, but are not limited to, T_{eff}, and may also include lymphoid precursor cells, Tnv, Tscm, Temra, and the like.

In one embodiment, in an aspect of the cells of the present disclosure, the tumor microenvironment conditions include a degree of transmission of a TCR-related signal. In a preferred embodiment, examples of the TCR-related signal include a TCR signal or a part thereof, and a chimeric signal of a TCR signal and another antigen (also referred to as a CAR signal).

In a preferred embodiment, in an aspect of the cells of the present disclosure, the TCR-related signal is mediated by a molecule containing a TCR signal domain. Such a molecule may be a nucleic acid molecule.

In a preferred embodiment, the change in tumor microenvironment conditions includes presentation of a tumor antigen and/or a costimulatory ligand, and/or a change in cytokine conditions.

In an embodiment, the degree of transmission of the TCR-related signal used in the present disclosure includes at least one selected from the group consisting of a degree of TCR stimulation, TCR co-stimulation, and a humoral factor. In a specific embodiment, the degree of transmission of the TCR-related signal includes a degree of TCR stimulation and at least one selected from the group consisting of TCR co-stimulation and a humoral factor. In another embodiment, the degree of transmission of a TCR-related signal includes a degree of TCR stimulation. In another embodiment, the degree of transmission of a TCR-related signal includes a degree of TCR stimulation, TCR co-stimulation, and a humoral factor. One or more of the strength of TCR stimulation, the influence of a co-stimulatory molecule, and the action of a humoral factor may be combined. TCR stimulation is initiated when a T cell receptor recognizes an antigen peptide bound to a major histocompatibility complex (MHC) on an antigen-presenting cell (APC). The strength and duration of this recognition greatly affect the degree of signal transduction. Strong TCR stimulation promotes activation and proliferation of T cells and further exertion of effector functions, whereas weak TCR stimulation may cause partial activation or unresponsiveness (anergy). In addition, involvement of co-stimulatory molecules is essential for signaling by TCR. Co-stimulatory molecules provide a second signal necessary for T cell activation and play a role in amplifying TCR signals. For example, CD28 is a stimulatory co-stimulatory molecule and enables complete T cell activation, whereas inhibitory CTLA-4 acts to suppress immune responses. The presence of appropriate co-stimulatory signals enhances immune responses and enables formation of memory T cells and sustained immune responses. On the other hand, when co-stimulation is absent, T cells enter a state of immune tolerance, and excessive immune responses to self-antigens are suppressed. Furthermore, humoral factors, particularly cytokines, play an important role in regulating the direction of T cell activation and differentiation. For example, IL-2 promotes proliferation of T cells, and IL-10 and TGF-β act to suppress immune responses. In addition, specific cytokines secreted in an inflammatory environment, such as IL-12 and IL-4, induce differentiation into Th1 cells and Th2 cells, respectively, and determine the nature of immune responses. As described above, humoral factors finely regulate immune responses and enable effective responses adapted to pathogens. Immune responses are adaptively and flexibly regulated by TCR stimulation, co-stimulation, or humoral factors alone, or by interactions of a plurality thereof. This enables rapid and specific immune defense against pathogens, and at the same time maintains the overall balance of the immune system, such as prevention of autoimmune diseases and maintenance of immune tolerance. This mechanism is an essential element for T cells to exhibit optimal responses according to environments and stimuli, and supports the diversity and functionality of the immune system.

In a specific embodiment, TCR stimulation may be assumed to include NFAT, NF-κB (nuclear factor-kappaB), the STAT transcription factor family (including, but not limited to, seven types: STAT1-4, STAT5A, STAT5B, and STAT56), and the like, which translocate into the nucleus in association with activation of T cells.

In another embodiment, the tumor microenvironment conditions include a degree of oxygen concentration. The degree of oxygen concentration in the tumor microenvironment is an important factor that greatly affects tumor progression and therapeutic responses. In tumor tissues, oxygen supply is often insufficient compared with normal tissues due to abnormal formation and structural heterogeneity of blood vessels, and this state is referred to as a "hypoxic state (hypoxia)". A hypoxic state has various effects on metabolism, proliferation, immune evasion, and treatment resistance of tumor cells. First, tumor cells cause activation of hypoxia-inducible factor (HIF) in order to adapt to a hypoxic state. HIF promotes expression of angiogenic factors (such as VEGF) in tumor cells and induces formation of new blood vessels, but these new blood vessels have abnormal structures and become a factor that causes a further hypoxic state. HIF also changes metabolic pathways of tumor cells and promotes the "Warburg effect", in which the glycolytic pathway, which does not require oxygen, is prioritized. This metabolic change not only maintains proliferation of tumor cells but also forms an acidic environment and plays a role in suppressing activity of immune cells.

In an aspect using a glucose transporter to which a tumor microenvironment-sensing element that senses a change in tumor microenvironment conditions is operably linked, in one embodiment, a glucose transporter linked to a tumor microenvironment-sensing element that senses properties such as oxygen concentration, pH, redox state, and inflammatory cytokines in a tumor microenvironment is an innovative research target utilizing tumor-specific metabolic properties. Such a transporter may be utilized for therapy or diagnosis by responding to changes in the tumor microenvironment and being specifically involved in energy supply and metabolic pathways of tumor cells. For example, by linking a response element of hypoxia-inducible factor (HIF-1), which is specifically activated in a hypoxic environment, to a glucose transporter, a mechanism in which glucose uptake increases under a hypoxic environment is designed. Such a transporter can be applied to development of drug delivery or diagnostic probes targeting a hypoxic state. In addition, since a tumor environment is characterized by a decrease in pH, a transporter that is specifically activated in an acidic environment in which tumor cells are present can be constructed by incorporating an element that senses an acidic environment into a glucose transporter. This mechanism contributes to design of therapies specialized for an acidic environment. Furthermore, in order to respond to the redox state of the tumor microenvironment, a design in which a redox-sensing element is linked to a glucose transporter is also contemplated. Since tumor cells are often exposed to higher oxidative stress than normal cells, such a transporter functions specifically in an environment with high oxidative stress and is useful as a therapeutic or diagnostic tool targeting tumor cells. In addition, by incorporating an inflammatory cytokine (for example, IL-6 or TNF-α) response element, it is also possible to develop a transporter that senses an inflammatory state in the tumor microenvironment and regulates glucose transport in response thereto. This is expected to enable identification of inflammatory tumors and improvement of therapeutic effects. As described above, a glucose transporter linked to a tumor microenvironment-sensing element is a technology that can serve as a basis for performing diagnosis and therapy more effectively and specifically by utilizing the tumor-specific metabolic environment.

In an embodiment, the tumor microenvironment-sensing element includes (i) an inducible activation motif (for example, HIH-RE, NFAT-RE) that is activated in response to a change (worsening or the like) in tumor microenvironment conditions, and (ii) a nucleic acid sequence encoding a degradation factor (for example, ODD) for the glucose transporter that is inactivated in response to the change (worsening or the like) in the tumor microenvironment conditions.

In an embodiment, the tumor microenvironment-sensing element includes: (i) an inducible activation motif (for example, NFAT-RE) to which a nuclear translocation factor that translocates into the nucleus in response to activation of T cells binds; (ii) a nuclear translocation factor-binding inducible activation motif to which a nuclear translocation factor that translocates into the nucleus in response to a decrease in oxygen concentration binds; and (iii) a nucleic acid sequence encoding a glucose transporter-degrading enzyme whose activity of degrading a glucose transporter is inactivated in response to the decrease in oxygen concentration.

In another embodiment, the nucleic acid sequence encoding the glucose transporter includes a construct operably linked to an inducible activation motif that is activated in response to a change in tumor microenvironment conditions and/or a nucleic acid sequence encoding a degradation factor for the glucose transporter that is inactivated in response to the change in the tumor microenvironment conditions.

In another embodiment, the nucleic acid sequence encoding the glucose transporter includes a construct operably linked to a nuclear translocation factor-binding inducible activation motif to which a nuclear translocation factor that translocates into the nucleus in response to activation of T cells binds.

In still another embodiment, nuclear translocation factors that may be used include NFAT, NF-κB (nuclear factor-kappaB), the STAT transcription factor family (STAT1-4, STAT5A, STAT5B, STAT56, and the like), and the like.

In a specific embodiment, the nuclear translocation factor-binding inducible activation motif may be an NFAT-binding domain, a κB motif (GGGACTTTCC) (SEQ ID NO: 1), a STAT-binding sequence (TTCNNNGAA), or modified sequences thereof. The modified sequence may include 1, 2, or 3 or more substitutions (mutual exchange of ATGC) compared with the original sequence.

In a specific embodiment, a combination of the nuclear translocation factor and the nuclear translocation factor-binding inducible activation motif includes:
(1) a combination of NFAT and an NFAT-binding domain;
(2) a combination of NF-κB (nuclear factor-kappaB) and a κB motif (GGGACTTTCC (SEQ ID NO: 1)); or
(3) a combination of the STAT transcription factor family (seven types: STAT1-4, STAT5A, STAT5B, and STAT56) and a STAT-binding sequence (TTCNNNGAA).

In a specific embodiment, the nucleic acid sequence encoding the glucose transporter includes a construct operably linked to a nuclear translocation factor-binding inducible activation motif to which a nuclear translocation factor that translocates into the nucleus in response to a decrease in oxygen concentration binds. In this embodiment, the nuclear translocation factor may be HIF-1 or the like. In this embodiment, the nuclear translocation factor-binding inducible activation motif may be HIF-RE or the like.

In a specific embodiment, the combination of the nuclear translocation factor and the nuclear translocation factor-binding inducible activation motif may be, for example, a combination of HIF-1 and HIF-RE.

In another embodiment, the nucleic acid sequence encoding the glucose transporter includes a construct operably linked to a nucleic acid sequence encoding a factor whose activity of degrading the glucose transporter is inactivated in response to a decrease in oxygen concentration. In this embodiment, the factor to be inactivated may be, for example, an oxygen-dependent degradation factor (ODD).

In a specific embodiment, the T cell of the present disclosure includes a chimeric antigen receptor (CAR).

In another embodiment, the T cell has an effector function. Alternatively, the cell is a precursor cell of an effector T cell (T_{eff}).

In a specific embodiment, memory function of the precursor cell of an effector T cell of the present disclosure is prolonged. The property of prolonging memory function in a precursor cell of an effector T cell has a great advantage in improving the persistence and effectiveness of an immune response. This property will be described below. A precursor cell of an effector T cell has the ability to exert an effector function in response to antigen stimulation, while having the potential to differentiate into memory T cells. Accordingly, immune memory against an antigen can be maintained over a long period of time even after the initial immune response has subsided. In particular, prolongation of memory function is advantageous in that it enhances the ability to elicit a rapid and strong immune response upon reinfection. First, prolongation of memory function enhances immune defense against tumors and the like. The presence of long-term memory cells enables a rapid and effective secondary immune response when the same antigen invades again. This increases the probability that tumors and the like are eliminated before spreading in the body, and severe disease can be prevented. Furthermore, prolongation of memory function also becomes a factor that enhances the effect of vaccines. When precursor cells of effector T cells formed after vaccination retain memory function for a long period of time, the duration of immune memory is extended, and it becomes possible to reduce the frequency of additional vaccination (booster vaccination). This point also contributes to improving the efficiency of vaccine strategies and reducing the burden on patients. In the field of tumor immunity, memory function of precursor cells of effector T cells is important. Tumor cells often hide antigens or create an immunosuppressive environment, but the presence of T cells having memory function for a long period of time is expected to sustain immune surveillance against tumor cells and reduce the risk of recurrence. In particular, when combined with immune checkpoint inhibitors or CAR-T cell therapy, a more sustained and stronger antitumor effect may be obtained. Furthermore, even under conditions such as chronic infections and autoimmune diseases, precursor cells of effector T cells having memory function contribute to maintenance of an appropriate immune response and control of the pathology. Long-term surveillance and control against specific pathogens or abnormal cells become possible, while unnecessary inflammation and tissue destruction are also expected to be suppressed. Overall, the property by which precursor cells of effector T cells prolong memory function provides great advantages in treatment of infectious diseases and tumors and further in vaccine development. By utilizing this property, induction of a more sustained and effective immune response becomes possible, and contribution to the development of medicine and biotechnology is expected.

In another embodiment, the memory T cell phenotype is maintained in the precursor cell of an effector T cell. There are many advantages to a precursor cell of an effector T cell maintaining the phenotype of a memory T cell. This property is extremely important in improving long-term immune memory and the efficiency of adaptive immune responses. First, when a precursor cell of an effector T cell retains the phenotype of a memory T cell, a rapid and efficient immune response becomes possible upon reinfection. Memory T cells retain antigen-specific memory, and when an antigen enters the body again, they are rapidly activated and exert effector functions. As a result, tumors and the like are effectively eliminated before spreading, and severe disease can be prevented. Maintenance of the memory T cell phenotype improves the persistence of immune responses. Whereas effector T cells are short-lived, memory T cells continue to exist in the body over a long period of time, and therefore immune memory formed after vaccination or natural infection is retained for a long period of time. This property is particularly advantageous from the viewpoint of reducing the frequency of additional vaccination (booster vaccination) and providing sustained immune defense. In tumor immunity, maintaining the memory T cell phenotype is important. Tumor cells often evade immune system surveillance by using antigen mutations or an immunosuppressive environment, but T cells having long-term immune memory continuously exert a surveillance function against recurrence and metastasis of tumor cells. This increases the possibility of reducing the risk of recurrence after treatment and improving the prognosis of patients. Furthermore, it also contributes to control of chronic infections. For example, against pathogens such as tuberculosis and HIV, sustained immune memory is important for suppressing progression of infection and managing the pathology. Maintenance of the memory T cell phenotype enables long-term surveillance and control against pathogens. In addition, application to control of autoimmune diseases and immunotherapy is also expected. If immune responses can be appropriately regulated while retaining the memory T cell phenotype, it is possible to induce selective immune responses against pathogens or abnormal cells while suppressing excessive immune reactions. Overall, maintenance of the memory T cell phenotype by precursor cells of effector T cells provides great advantages in treatment or prevention of infectious diseases, tumors, and chronic diseases. By understanding and utilizing this property, development of more effective and sustained immunotherapies and vaccine strategies is expected.

In a specific embodiment, the effector T cell is activated or activity of the effector T cell is maintained, and memory function of the precursor cell of the effector T cell is prolonged or a memory T cell phenotype of the precursor cell of the effector T cell is maintained. Both prolongation of memory function and maintenance of the memory T cell phenotype may be achieved. In this case, the effects may be synergistically enhanced.

The present disclosure is effective in all tumors, including solid cancers. Clinical trials of CAR-T cells against solid cancers have not been able to demonstrate efficacy so far, but the present inventors have found that dysfunction of CAR-T cells in solid cancers and the like is caused by glucose deficiency in the tumor microenvironment (TME) or tumor microenvironment conditions equivalent thereto (also referred to as TMEC), and have shown that on-demand, rather than continuous, metabolic supplementation significantly improves the antitumor effect of CAR-T cells. Massive consumption of glucose by cancer cells decreases glucose levels in the TME of solid cancers and the like, and consequently impairs CAR-T cells. The present inventors found that when a glucose transporter (GLUT, for example, GLUT3), which is a high-affinity glucose transporter, is stably expressed in CAR-T cells, cytokine production and killing activity are restored. However, while CAR-T cells stably expressing GLUT induce tumor reduction in a solid cancer model, their overactivation led to adverse events and death of mice. CAR-T cells into which GLUT was introduced in an on-demand manner showed improved metabolic fitness and an increased antitumor effect, while preventing adverse events, and brought about long-term tumor control in an intracranial human GBM cell xenograft model, through a mechanism in which nuclear factor of activated T cells (NFAT), which translocates into the nucleus upon activation by stimulation with the target antigen used as an example, binds to the tumor microenvironment-sensing element, thereby inducing transcription of GLUT or suppressing degradation of GLUT. The present inventors consider that on-demand but stable metabolic fitness, such as upon exposure to a tumor antigen, is a new concept for maximizing the antitumor effect of CAR-T cells against solid cancers.

In a specific embodiment, the present disclosure shows that dysfunction of CAR-T cells in GBM is caused by glucose deficiency in a tumor microenvironment (TME), and that on-demand, rather than continuous, metabolic supplementation significantly improves the antitumor effect of CAR-T cells. Massive consumption of glucose by cancer cells decreases glucose levels in the TME of GBM, and consequently impairs CAR-T cells. When GLUT3, which is a high-affinity glucose transporter, was stably expressed in CAR-T cells, cytokine production and killing activity were restored. However, while CAR-T cells stably expressing GLUT3 induced tumor reduction in a GBM model, their overactivation led to adverse events and death of mice. In on-demand GLUT3 CAR-T cells, GLUT3 transcription was induced by nuclear translocation of nuclear factor of activated T cells (NFAT) due to target antigen stimulation, and the cells showed improved metabolic fitness and an increased antitumor effect, and brought about long-term tumor control in an intracranial human GBM cell xenograft model while preventing adverse events. The present inventors propose that on-demand but stable metabolic fitness, such as upon exposure to a tumor antigen, is a new concept for maximizing the antitumor effect of CAR-T cells against solid tumors.

In one aspect, glioblastoma (GBM), which is targeted by the present disclosure, is a devastating malignant brain tumor, with an annual incidence of 3.19 per 100,000 people. Since the introduction in 2005 of combination therapy consisting of temozolomide and radiation, and the development of tumor treating fields therapy (Novo TTF) for primary GBM, the prognosis of GBM patients in clinical practice has improved. However, the 5-year overall survival rate of GBM remains below 20% [References 1 and 2]. In light of the clinical success of chimeric antigen receptor T cell (CAR-T cell) therapy in hematologic malignancies, several preclinical and clinical trials of CAR-T cell therapy for GBM have been actively investigated [References 3 to 10]. Some patients treated in clinical trials show promising results, such as infiltration of CAR-T cells into the tumor microenvironment (TME) and reduction in tumor burden, but most patients do not respond to CAR-T cell therapy [Reference 3].

GBM has a metabolically harsh TME, impairs the function of effector T cells including CD8+ T cells, and adoptively transfers CAR-T cells into the TME. In the brain, nervous system cells such as neurons and glial cells require glucose as an energy source, and the brain consumes 25% of whole-body glucose-derived energy [Reference 11]. Furthermore, aggressive GBM cells consume three times more glucose than normal neurons due to aerobic glycolysis, which is a characteristic metabolism of cancer cells known as the Warburg effect [Reference 12]. This leads to depletion of glucose in the TME of GBM [References 13 to 16]. On the other hand, naive T cells shift energy production from oxidative phosphorylation and fatty acid oxidation to aerobic glycolysis upon activation in order to meet increased energy demand. Therefore, intense glucose-mediated metabolic competition occurs in the TME of GBM between activated T cells and cancer cells. GLUT3, which is encoded by SLC2A3 and physiologically expressed in neurons and glial cells, has a 5-fold higher affinity for glucose than GLUT1, which is the main glucose transporter expressed in T cells [References 16 to 18]. As a result, GBM cells expressing GLUT3 can survive and continuously proliferate while taking up sufficient glucose in the TME, whereas activated T cells have impaired function due to failure in metabolic competition [References 19 and 20].

Considering the substantial glucose uptake by GBM cells mediated by high-affinity GLUT3, equivalent or higher expression of GLUT3 by CAR-T cells may enable complete competition with GBM cells. In the present disclosure, a construct in which SLC2A3 was linked to CAR was developed in order to induce expression of GLUT3 in CAR-T cells. However, stable expression of GLUT3 induced overactivation of CAR-T cells, showing that effector T cells require delicate energy supplementation. Therefore, GLUT3 expression was modified so as to be driven by glucose demand using activation of CAR-T cells associated with target antigen exposure in the TME (on-demand expression). In CAR-T cells having on-demand GLUT3 expression, GLUT3 transcription was driven by nuclear translocation of nuclear factor of activated T cells (NFAT) upon target antigen stimulation, thereby restoring metabolic fitness, and as a result, enhancing sustained antitumor effects and prolonging survival in a harsh TME of a GBM model with low glucose levels. In summary, the concept of restoring T cell function in the TME by enhancing metabolic fitness needs to be investigated in an on-demand manner, rather than by stable supplementation.

### (Cell population)

In one aspect of the present disclosure, a cell population containing immune cells such as T cells with enhanced glucose uptake ability on demand is provided. Alternatively, a cell population containing T cells with enhanced glucose uptake ability, in which expression of a glucose transporter is regulated in the T cells in response to a change in tumor microenvironment conditions, is provided. Alternatively, a cell population containing T cells with enhanced glucose uptake ability, the T cells being modified so that expression of a glucose transporter is regulated in the T cells in response to a change in tumor microenvironment conditions, is provided. Alternatively, a cell population is provided, the cell population containing T cells containing a nucleic acid sequence encoding a glucose transporter, the nucleic acid sequence being operably linked to a tumor microenvironment-sensing element that senses a change in tumor microenvironment conditions. It has been unexpectedly shown that, when at least some of the T cells contained in such a cell population are modified to express a glucose transporter and/or are enhanced, remarkable effects on diseases such as cancer are achieved when the cell population is used as CAR-T cells or the like (FIG. 19). It is advantageous that the cell population of the present disclosure has enhanced glucose uptake ability. In addition, the cell population of the present disclosure preferably contains cells that have an effector function after being introduced into a place where they are to function, particularly into the body. Examples of such cells include, but are not limited to, T_{eff}, and may also include lymphoid precursor cells, Tnv, Tscm, Temra, and the like.

In a cell population containing T cells in which glucose uptake ability is constitutively enhanced, differentiation into effector memory-type T cells and exhaustion were observed. In contrast, a cell population containing those in which expression of a glucose transporter is enhanced on demand is remarkable in that, while equivalent cytotoxic activity is maintained, maintenance of a memory phenotype and exhaustion resistance, which are expected to maintain an effect for a longer period, are induced. It is advantageous that the cell population of the present disclosure is a cell population containing cells with enhanced glucose uptake ability. Such a cell population containing cells in which glucose uptake ability is enhanced on demand exerts remarkable effects on diseases such as cancer, autoimmune diseases, allergies, and infectious diseases when used as CAR-T cells, TCR-T cells, and the like.

In addition, the cell population of the present disclosure exhibited a remarkably high recurrence-preventing effect when used as CAR-T cells and the like. In addition, sudden death of mice due to toxicity caused by non-tumor factors, which was observed in T cells in which glucose was constitutively enhanced, was avoided in those in which a glucose transporter is expressed or expression of a glucose transporter is enhanced on demand, or in which expression of a glucose transporter is regulated in the T cells in response to a change in tumor microenvironment conditions and which contain a nucleic acid sequence encoding a glucose transporter, the nucleic acid sequence being operably linked to a tumor microenvironment-sensing element that senses a change in tumor microenvironment conditions. These findings were unexpected, and without being bound by theory, they may be explained by avoidance of host toxicity caused by excessive T-cell activation or excessive glucose uptake, a marked decrease in exhaustion, nutrient supply matching nutritional demand, suppression of cell death caused by appropriate activation, induction of memory formation, and the like. In addition, the cell population containing the cells of the present disclosure preferably has an effector function after being introduced into a place where it is to function, particularly into the body. Examples of such cells include, but are not limited to, T_{eff}, and may also be precursor cells of T_{eff}, for example, lymphoid progenitor cells, Tnv, Tcm, Temra, and the like.

In an embodiment of the present disclosure, in a cell population containing a cell population containing immune cells such as T cells as described above, it is advantageous that the cell population contains T cells having the features of the present disclosure (for example, enhancement or expression of GLUT on demand, an effector function, or both) in an amount of about 10% or more in the cell population. In an embodiment, in the cell population of the present disclosure, the proportion of T cells having the features of the present disclosure may be about 5% or more, about 10% or more, about 15% or more, about 20% or more, about 25% or more, about 30% or more, about 35% or more, about 40% or more, about 45% or more, about 50% or more, about 55% or more, about 60% or more, about 65% or more, about 70% or more, about 75% or more, about 80% or more, about 85% or more, about 90% or more, about 95% or more, about 97% or more, or about 99% or more.

In one embodiment, the cell population of the present disclosure contains T cells having a property of exhibiting an effector function when introduced into the body.

In one embodiment, in the cell population of the present disclosure, the T cells contain a chimeric antigen receptor (CAR).

In one embodiment, in the cell population of the present disclosure, the T cells are modified to express a glucose transporter in response to demand (on demand), and/or have enhanced expression of a glucose transporter in response to demand (on demand). Alternatively, the cells in the cell population of the present disclosure may be T cells with enhanced glucose uptake ability, in which expression of a glucose transporter is regulated in the T cells in response to a change in tumor microenvironment conditions; T cells with enhanced glucose uptake ability, the T cells being modified so that expression of a glucose transporter is regulated in the T cells in response to a change in tumor microenvironment conditions; or T cells containing a nucleic acid sequence encoding a glucose transporter, the nucleic acid sequence being operably linked to a tumor microenvironment-sensing element that senses a change in tumor microenvironment conditions.

In one embodiment, in the cell population of the present disclosure, the T cells have an effector function.

Alternatively, in the T cells in the cell population of the present disclosure, the tumor microenvironment conditions include a degree of transmission of a TCR-related signal, and in an embodiment, the TCR-related signal is mediated by a molecule selected from the group consisting of a TCR signal or a part thereof, and a chimeric signal of a TCR signal and another antigen (also referred to as a CAR signal). Preferably, the TCR-related signal is mediated by a molecule containing a TCR signal domain.

Alternatively, the change in tumor microenvironment conditions includes presentation of a tumor antigen and/or a co-stimulatory ligand, and/or a change in cytokine conditions. Preferably, the degree of transmission of a TCR-related signal includes a degree of TCR stimulation. Alternatively, in another embodiment, the degree of transmission of a TCR-related signal includes at least one selected from the group consisting of a degree of TCR stimulation, TCR co-stimulation, and a humoral factor.

In another embodiment, in the cells in the cell population of the present disclosure, the degree of transmission of a TCR-related signal includes a degree of TCR stimulation and at least one selected from the group consisting of TCR co-stimulation and a humoral factor. In one embodiment, the degree of transmission of a TCR-related signal includes a degree of TCR stimulation, TCR co-stimulation, and a humoral factor. Preferably, the TCR stimulation is selected from the group consisting of NFAT, NF-κB, and the STAT transcription factor family, which translocate into the nucleus in association with activation of T cells. Alternatively, the tumor microenvironment conditions include a degree of oxygen concentration.

In another embodiment, in a cell population containing T cells containing a nucleic acid sequence encoding a glucose transporter, the nucleic acid sequence being operably linked to a tumor microenvironment-sensing element that senses a change in tumor microenvironment conditions of the present disclosure, with respect to each T cell, the tumor microenvironment-sensing element includes: (i) an inducible activation motif that is activated in response to a change in tumor microenvironment conditions; and (ii) a nucleic acid sequence encoding a degradation factor for the glucose transporter that is inactivated in response to the change in the tumor microenvironment conditions. Here, the tumor microenvironment-sensing element preferably includes: (i) an inducible activation motif to which a nuclear translocation factor that translocates into the nucleus in response to activation of T cells binds; (ii) a nuclear translocation factor-binding inducible activation motif to which a nuclear translocation factor that translocates into the nucleus in response to a decrease in oxygen concentration binds; and (iii) a nucleic acid sequence encoding a glucose transporter-degrading enzyme whose activity of degrading a glucose transporter is inactivated in response to the decrease in oxygen concentration.

In another embodiment, with respect to the T cells in the cell population of the present disclosure, the nucleic acid sequence encoding the glucose transporter includes a construct operably linked to an inducible activation motif that is activated in response to a change in tumor microenvironment conditions and/or a nucleic acid sequence encoding a degradation factor for the glucose transporter that is inactivated in response to the change in the tumor microenvironment conditions. In an embodiment, the nucleic acid sequence encoding the glucose transporter includes a construct operably linked to a nuclear translocation factor-binding inducible activation motif to which a nuclear translocation factor that translocates into the nucleus in response to activation of T cells binds. In a preferred embodiment, the nuclear translocation factor is selected from the group consisting of NFAT, NF-κB, and the STAT transcription factor family (for example, TAT1-4, STAT5A, STAT5B, STAT5, and the like), and the nuclear translocation factor-binding inducible activation motif is selected from the group consisting of an NFAT-binding domain, a κB motif (GGGACTTTCC) (SEQ ID NO: 1), a STAT-binding sequence (TTCNNNGAA), and modified sequences thereof. In a preferred embodiment, the combination of the nuclear translocation factor and the nuclear translocation factor-binding inducible activation motif includes (1) a combination of NFAT and an NFAT-binding domain, (2) a combination of NF-κB and a κB motif (GGGACTTTCC (SEQ ID NO: 1), or (3) a combination of the STAT transcription factor family and a STAT-binding sequence (TTCNNNGAA).

In another embodiment, with respect to the T cells in the cell population of the present disclosure, the nucleic acid sequence encoding the glucose transporter includes a construct operably linked to a nuclear translocation factor-binding inducible activation motif to which a nuclear translocation factor that translocates into the nucleus in response to a decrease in oxygen concentration binds. Preferably, the nuclear translocation factor is selected from the group consisting of HIF-1, and the nuclear translocation factor-binding inducible activation motif is selected from the group consisting of HIF-RE, or the combination of the nuclear translocation factor and the nuclear translocation factor-binding inducible activation motif is selected from the group consisting of (1) a combination of HIF-1 and HIF-RE.

In another embodiment, with respect to the T cells in the cell population of the present disclosure, the nucleic acid sequence encoding the glucose transporter includes a construct operably linked to a nucleic acid sequence encoding a factor whose activity of degrading the glucose transporter is inactivated in response to a decrease in oxygen concentration, and preferably, the factor to be inactivated is selected from the group consisting of an oxygen-dependent degradation factor (ODD).

In another embodiment, with respect to the T cells in the cell population of the present disclosure, the T cells have an effector function, and the T cells may include, or consist of, precursor cells of effector T cells (T_{eff}). Here, memory function of the precursor cells of effector T cells is prolonged, and/or the memory T cell phenotype is maintained. Alternatively, the effector T cell is activated or activity of the effector T cell is maintained, and memory function of the precursor cell of the effector T cell is prolonged or a memory T cell phenotype of the precursor cell of the effector T cell is maintained.

In another embodiment, with respect to the T cells in the cell population of the present disclosure, the expression and/or enhancement is achieved specifically in a tumor environment.

In one embodiment, the cell population of the present disclosure contains T_{eff} and/or precursor cells of T_{eff}. Preferably, the cell population (or precursor cells of T_{eff}) contains at least one type of cell such as lymphoid progenitor cells, Tnv, Tscm, Tcm, or Temr.

In one embodiment, in the cell population of the present disclosure, the CAR is expressed in the T cells.

In one embodiment, in the cell population of the present disclosure, the glucose transporter includes GLUT1, GLUT2, GLUT3, and GLUT4, and is preferably GLUT3.

In one embodiment, in the cell population of the present disclosure, the T cells are human T cells.

In one embodiment, the cell population of the present disclosure is provided as a medicament or pharmaceutical composition. The use thereof may be for prevention or treatment of cancer, for cure of cancer, or for prevention of cancer metastasis or prevention of cancer recurrence. In a specific embodiment, the cell population of the present disclosure may be for preventing or treating cancer so that the cancer does not recur.

### <Glucose transporter>

Cancer cells reprogram their metabolic systems to favor their own cell proliferation, actively use the glycolytic pathway, which has low efficiency in ATP production, even in the presence of oxygen, and increase glucose uptake and lactic acid production (Warburg effect). Therefore, because cancer cells consume and deplete a large amount of glucose, T_{eff}-type tumor-specific T cells, which require glucose as an energy source, have a decreased cellular Ca²⁺ concentration and become starved and exhausted when they infiltrate into a tumor, even if they receive a signal through TCR.

Accordingly, in an embodiment of the present disclosure, T cells and cell populations genetically engineered to express a glucose transporter are provided in order to promote aerobic glycolysis and enhance competitiveness and/or fitness of immune cells in a tumor microenvironment (TME) in which glucose is depleted.

In an embodiment, examples of the glucose transporter include GLUT1, GLUT2, GLUT3, and GLUT4, and among these, the glucose transporter having high affinity for glucose is GLUT3. Therefore, in a preferred embodiment, the glucose transporter used in the cells of the present disclosure is GLUT3.

GLUT3 was first identified in the mouse brain and was initially defined as a neuronal glucose transporter. Thereafter, GLUT3 was shown to be expressed also in other cells that require glucose, such as mouse sperm, which secure energy necessary for motility, and blastocysts, which are important for post-implantation development. Furthermore, GLUT3 is expressed in immune cells such as lymphocytes, monocytes, macrophages, and platelets, and is usually stored in intracellular vesicles and, upon activation, moves to the cell surface to maintain a metabolic switch. More recently, it has been suggested that T cells, particularly CD8⁺ T cells, depend not only on GLUT1 but also on GLUT3 for glucose uptake because GLUT3 is highly expressed during differentiation and activation.

In an embodiment, the T cells of the present disclosure and the T cells contained in the cell population may be modified to express a glucose transporter on demand, and/or expression of a glucose transporter may be enhanced on demand. In a T cell in which glucose uptake ability is constitutively enhanced, differentiation into an effector memory-type T cell and exhaustion were observed, and marked T-cell death was induced when the T cell was activated under no- to low-glucose conditions. In contrast, a T cell in which expression of a glucose transporter is enhanced on demand is remarkable in that, while equivalent cytotoxic activity is maintained, T-cell death under no- to low-glucose conditions is avoided, and maintenance of a memory phenotype and exhaustion resistance, which are expected to maintain an effect for a longer period, are induced.

When a glucose transporter such as GLUT3 is activated in T cells, enhancement of effector functions such as cytotoxic activity and cytokine production ability is observed due to glucose uptake, but at the same time, when the T cells are activated for a long period of time, differentiation into short-acting effector memory-type cells and T-cell exhaustion are markedly induced. Therefore, in an embodiment of the present disclosure, it is possible to use an NFAT-induced glucose-induced expression system induced by a glucose derivative, triggered by activation in which glucose demand of T cells increases or entry into a tumor environment, which is a hypoxic environment, or a hypoxia-induced glucose-induced expression system applying the mechanism of HIF1a (for example, FIG. 12). This makes it possible to realize transient expression in accordance with an increase in demand, specifically in a tumor environment. Therefore, the cells of the present disclosure can maintain memory phenotype T cells and avoid exhaustion while maintaining cytotoxic activity.

In an embodiment of the present disclosure, the T cells of the present disclosure or the T cells contained in the cell population may include effector cells.

In an embodiment of the present disclosure, the T cells of the present disclosure and the T cells contained in the cell population may advantageously be human T cells.

### <Chimeric antigen receptor (CAR)>

In another aspect of the present disclosure, provided are a T cell with enhanced glucose uptake ability or a cell population containing such cells, in which the T cell includes a chimeric antigen receptor (CAR), and the T cell is modified to express a glucose transporter on demand and/or has enhanced expression of a glucose transporter on demand. The T cell of the present disclosure, or the T cells contained in the cell population, may contain the CAR as a protein, or may contain a nucleic acid molecule expressing the CAR, as long as the CAR can exert a function as a CAR. In one embodiment, the CAR of the present disclosure may be expressed in T cells.

The CAR disclosed in the present specification includes at least one extracellular domain capable of binding to an antigen, at least one transmembrane domain, and at least one intracellular domain.

A chimeric antigen receptor (CAR) is an artificially constructed hybrid protein or polypeptide containing an antigen-binding domain of an antibody (for example, a single-chain variable fragment (scFv)) linked to a T cell signaling domain via a transmembrane domain. A feature of CAR is that CAR redirects the specificity and reactivity of T cells toward a selected target by utilizing the antigen-binding properties of a monoclonal antibody independently of MHC. MHC-independent antigen recognition can provide T cells expressing CAR or T cells in a cell population with the ability to recognize an antigen independently of antigen processing, and can avoid immune escape of tumors.

The intracellular T cell signaling domain of CAR may include, for example, a T cell receptor signaling domain, a T cell co-stimulatory signaling domain, or both. The T cell receptor signaling domain refers to a portion of CAR including an intracellular domain of a T cell receptor, for example, an intracellular portion of a CD3 zeta protein. The co-stimulatory signaling domain refers to a portion of CAR including an intracellular domain of a co-stimulatory molecule, which is a cell surface molecule other than an antigen receptor or a ligand thereof, required for an efficient response of lymphocytes to an antigen.

### <T cell receptor (TCR)>

In another aspect of the present disclosure, provided are a T cell with enhanced glucose uptake ability or a cell population containing such cells, in which the T cell contains a T cell receptor (TCR), and the T cell is modified to express a glucose transporter on demand and/or has enhanced expression of a glucose transporter on demand. As long as the TCR can exert a function as a TCR, the T cell of the present disclosure or the T cells contained in the cell population may contain the TCR as a protein or may contain a nucleic acid molecule expressing the TCR. In a representative embodiment, the TCR of the present disclosure may be expressed in T cells.

In the present specification, TCR is a heterodimeric receptor molecule consisting of two TCR polypeptide chains, and includes αβ-type TCR expressed by ordinary T cells and γδ-type TCR having special functions. The α- and β-chain TCR molecules form a complex with a plurality of CD3 molecules (CD3ζ chain, CD3ε chain, CD3γ chain, and CD3δ chain), transmit intracellular signals after antigen recognition, and initiate various immune responses. Endogenous antigens, such as viral antigens that have proliferated intracellularly in association with viral infection and cancer antigens derived from cancer cells, are presented as antigen peptides on MHC class I molecules. In addition, antigens derived from foreign microorganisms are taken up by antigen-presenting cells by endocytosis, and after being processed, are presented on MHC class II molecules. These antigens are recognized by TCRs expressed by CD8+ T cells or CD4+ T cells, respectively. It is also known that costimulatory molecules such as CD28, ICOS, and OX40 molecules are important for stimulation mediated by TCR molecules. With respect to αβ-type TCR, the respective gene products of α and β can exhibit specificity through a unique combination. TCR can be modified as appropriate and may include a T cell receptor signaling domain, a T cell co-stimulatory signaling domain, or both. The T cell receptor signaling domain includes an intracellular domain of a T cell receptor, for example, an intracellular portion of a CD3 zeta protein. The co-stimulatory signaling domain includes an intracellular domain of a co-stimulatory molecule, which is a cell surface molecule other than an antigen receptor or a ligand thereof, required for an efficient response of lymphocytes to an antigen.

### (Extracellular domain)

In an embodiment, the CAR used in the T cell disclosed in the present specification or the T cells contained in the cell population includes an antigen-binding domain or a portion thereof. The antigen-binding domain or a portion thereof can be appropriately selected according to the type and number of ligands on the surface of target cells, and, for example, the antigen-binding domain may be selected to recognize a ligand that acts as a cell surface marker on a target cell associated with a specific disease state. Therefore, examples of cell surface markers that may act as ligands for the antigen-binding domain in the CAR of the present disclosure include tissue-specific markers, cytoma-specific markers, and those associated with viral, bacterial, and parasitic infections, autoimmune diseases, and cancer cells.

The extracellular binding domain of CAR may be constituted by a single-chain variable fragment (scFv) obtained by fusing variable heavy and light regions of a mouse or humanized monoclonal antibody. Alternatively, an scFv derived from Fab (obtained, for example, from a Fab library, not from an antibody) may be used. The scFv can be fused to a transmembrane domain and then to an intracellular signaling domain.

In an embodiment, the antigen-binding domain portion of the CAR of the present disclosure can target antigens including: (1) alloantigens including MHC class I, MHC class II, and the like; (2) extracellular autoantigens including thyroid stimulating hormone receptor (TSHR), desmoglein 3 (DSG3), Cytokeratin8, and the like; (3) foreign antigens including Gliadin, Ara h2, and the like; and (4) targeting molecules including CD4, CD8, CD19, BCMA, CD68, mesothelin (MSLN), mucosal vascular addressin cell adhesion molecule 1 (MadCam1), and the like, but antigens that can be targeted by the antigen-binding domain portion of the CAR of the present disclosure are not limited thereto.

In an embodiment, depending on a desired antigen to be targeted, the CAR of the present disclosure can be modified to include an antigen-binding domain specific to the desired antigen target. For example, when CD19 is a target antigen, an antibody against a cancer antigen such as CD19 may be used as an antigen-binding domain in the CAR. Non-limiting examples of cancer antigens include CD19, CD20, CD30, CD33, CD38, CD133, BCMA, TEM8, EpCAM, ROR1, folate receptor, CD70, MAGE-1, MAGE-2, MAGE-3, MAGE A-10, MAGE-C2, MAGE-A12, CEA, tyrosinase, midkine, BAGE, CASP-8, P-catenin, CA-125, CDK-1, ESO-1, gp75, MART-1, MUC-1, MUM-1, p53, PAP, PSA, PSMA, ras, trp-1, HER-2, TRP-1, TRP-2, IL13Ralpha, IL13Ralpha2, AIM-2, AIM-3, NY-ESO-1, C9orfl l2, SART1, SART2, SART3, BRAP, RTN4, GLEA2, TNKS2, KIAA0376, ING4, HSPH1, C13orf24, RBPSUH, C6orfl53, NKTR, NSEP1, U2AF1L, CYNL2, TPR GOLGA, BMI1, COX-2, EGFRvIII, EZH2, LICAM, Livin, LivinP, MRP-3, Nestin, OLIG2, ART1, ART4, B cycling, Grill, Cav-1, cathepsin B, CD74, E-Cadherin, EphA2/Eck, Fra-1/Fosl 1, GAGE-1, ganglioside/GD2, GnT-V, pl, 6-N, Ki67, Ku70/80, PROXI, PSCA, SOXIO, SOX11, Survivin, phCG, WT1, mesothelin, Melan A, NY-BR-1, NY-CO-58, MN (gp250), telomerase, SSX-2, PRAME, PLK1, VEGF-A, VEGFR2, and Tie-2. In some embodiments, the effector cells disclosed in the present specification are engineered to express one or more CARs in order to recognize one or more antigens.

### (Transmembrane domain)

The CAR used in the T cell disclosed in the present specification or the T cells contained in the cell population can include one or more transmembrane domains fused to the extracellular domain.

In an embodiment, a linker domain derived from the extracellular domain may be linked to the transmembrane domain. The transmembrane domain may be natural or synthetic, and a natural transmembrane domain may be derived from any membrane-bound or transmembrane protein. The transmembrane region particularly used in the present disclosure may be derived from the alpha, beta, or zeta chain of a T cell receptor, CD28, CD3 epsilon, CD45, CD4, CD5, CD8, CD9, CD16, CD22, CD33, CD37, CD64, CD80, CD86, CD134, CD137, CD154, CD271, TNFRSF19, and the like.

In an embodiment, in the CAR used in the effector T cells disclosed in the present specification, a spacer domain may also be disposed between the extracellular domain and the transmembrane domain, or between the intracellular domain and the transmembrane domain. The spacer domain can preferably have a sequence that promotes binding of the CAR to an antigen and enhances signal transduction into cells.

### (Intracellular domain)

The cytoplasmic signaling domain of CAR (or intracellular signaling domain) is involved in activation of at least one normal effector function of an immune cell expressing the CAR. The intracellular signaling domain refers to a portion of a protein that transmits an effector function signal and instructs a cell expressing the CAR to perform a specialized function. The intracellular signaling domain may include any complete, mutant, or truncated portion of an intracellular signaling domain of a given protein that is sufficient to transmit a signal that elicits or blocks an immune cell effector function.

In an embodiment, examples of the intracellular signaling domain used in CAR include cytoplasmic signaling sequences of a T cell receptor (TCR) and co-receptors that elicit signal transduction after antigen receptor binding.

### (Alloantigens, allergens, and haptens related to rejection)

The CAR used in the T cell disclosed in the present specification or in the T cells contained in the cell population can include those related to alloantigens associated with rejection, allergens, and haptens.

### <Pharmaceutical use of T cells or T cells contained in cell population>

In one aspect of the present disclosure, provided is a pharmaceutical composition containing a T cell with enhanced glucose uptake ability or a cell population containing such T cells, in which the T cell is modified to express a glucose transporter on demand and/or has enhanced expression of a glucose transporter on demand. In another aspect of the present disclosure, provided is a pharmaceutical composition containing a T cell with enhanced glucose uptake ability or a cell population containing such T cells, in which the T cell includes a chimeric antigen receptor (CAR), a T cell receptor (TCR), or the like, and the T cell is modified to express a glucose transporter on demand and/or has enhanced expression of a glucose transporter on demand. In an embodiment of the present disclosure, the T cell in the present disclosure or the T cells contained in the cell population can have one or more of the features of the effector T cells described above.

The cells, cell populations, and the like of the present disclosure can be used in immunotherapy. Immunotherapy is considered effective against diseases in which lesions having antigenicity occur (for example, cancer, autoimmune diseases, allergies, infectious diseases, and the like), and diseases in which an abnormal immune response to a specific antigen is involved in onset or progression of the pathology. For example, the cells and the like of the present disclosure can be used for treatment, therapy, or prevention of autoimmune diseases, allergic diseases, or graft-versus-host disease (GVHD), rejection, or engraftment failure at the time of transplantation. Examples of autoimmune diseases include, but are not limited to, rheumatoid arthritis (RA), Sjögren's syndrome, systemic lupus erythematosus (SLE), antiphospholipid syndrome, polymyositis/dermatomyositis, systemic sclerosis, mixed connective tissue disease, vasculitis syndrome, type I diabetes, Graves' disease, Hashimoto's disease, idiopathic Addison's disease, autoimmune hepatitis, Goodpasture's syndrome, glomerulonephritis, autoimmune hemolytic anemia (AIHA), autoimmune thrombocytopenic purpura, autoimmune neutropenia, myasthenia gravis, pemphigus, vitiligo, and idiopathic azoospermia. Examples of allergic diseases include, but are not limited to, pollinosis, allergic rhinitis, bronchial asthma, and atopic dermatitis. In addition, treatment or prevention with the cells, cell populations, and the like of the present disclosure can be used for diseases in which an abnormal immune response to a specific antigen is involved in onset or progression of the pathology.

In a specific embodiment, the present disclosure provides such immunotherapy, and the immunotherapy includes TCR-T therapy or CAR-T therapy. In the present specification, the term "TCR-T therapy" refers to cell therapy using modification of a T cell receptor (TCR), and refers to, for example, therapy used for cancer treatment. In the present specification, the term "CAR-T therapy" refers to gene and cell therapy in which a chimeric antigen receptor (CAR) (for example, one genetically engineered to overcome a tumor immune evasion mechanism) is genetically introduced into T cells of a patient, and the T cells are expanded and cultured ex vivo and then infused into the patient.

The cells, cell populations, and the like of the present disclosure may be appropriately combined with other cancer treatments and may be used as combination therapy. Typically, they may be administered in combination with one further drug or a plurality of further drugs. Alternatively, the combination therapy may be a combination with radiation therapy. The one further drug or the plurality of further drugs may be any chemotherapeutic agent or may include an immune checkpoint inhibitor. Alternatively, other cancer treatments used in the combination therapy include, but are not limited to, other cancer immunotherapies (for example, immune checkpoint inhibitors), thermotherapy, surgical procedures, and the like.

In one aspect, provided is a therapeutic agent containing the T cells or cell population of the present disclosure, in which a disease of a subject is diagnosed, and an appropriate CAR, TCR, or the like contained in the T cells or the cell population is selected based on the diagnosis.

In another aspect, the present disclosure provides a pharmaceutical composition containing any of the immune cells described in the present specification (for example, T cells such as effector T cells) or a cell population containing such cells, and a pharmaceutically acceptable carrier. When the immune cells express a polypeptide such as CAR or TCR, the pharmaceutical composition may further contain an Fc-containing therapeutic agent such as a therapeutic antibody or an Fc fusion protein. The Fc-containing therapeutic agent can bind to a target antigen, such as one on immune cells specific for a tumor antigen, a pathogen antigen, or self-antigen. The pathogen antigen may be a bacterial antigen, viral antigen, or fungal antigen.

In an embodiment, the Fc-containing therapeutic agent may be a therapeutic antibody including, but not limited to, adalimumab, ado-trastuzumab emtansine, alemtuzumab, basiliximab, bevacizumab, belimumab, brentuximab, canakinumab, cetuximab, certolizumab, daclizumab, denosumab, dinutuximab, eculizumab, efalizumab, epratuzumab, gemtuzumab, golimumab, hu14.18K322A, ibritumomab, infliximab, ipilimumab, labetuzumab, muromonab, natalizumab, obinutuzumab, ofatumumab, omalizumab, palivizumab, panitumumab, pertuzumab, ramucirumab, ranibizumab, rituximab, tocilizumab, trastuzumab, tositumomab, ustekinumab, mogamulizumab, and vedolizumab.

Furthermore, the present disclosure provides a kit containing: (i) a first pharmaceutical composition containing any of the T cells described in the present specification or a cell population containing such T cells, and a pharmaceutically acceptable carrier; and (ii) the Fc-containing therapeutic agent described in the present specification and a pharmaceutically acceptable carrier.

In another aspect, the present disclosure provides a method for inhibiting cells expressing a target antigen in a subject (for example, reducing the number of such cells, inhibiting cell proliferation, and/or suppressing cellular activity), the method including administering the cells, cell population, and/or pharmaceutical composition of the present disclosure to the subject. In an embodiment, at least some of the cells expressing the target antigen may be in a low-glucose environment.

In an embodiment, the subject treated by the method of the present disclosure may be a human patient suffering from cancer, for example, carcinoma, lymphoma, sarcoma, blastoma, or leukemia. Examples of target cancers include, but are not limited to, B-cell-derived cancer, breast cancer, gastric cancer, neuroblastoma, osteosarcoma, lung cancer, skin cancer, prostate cancer, colon cancer, renal cell carcinoma, ovarian cancer, rhabdomyosarcoma, leukemia, mesothelioma, pancreatic cancer, head and neck cancer, retinoblastoma, glioma, glioblastoma, liver cancer, and thyroid cancer. Examples of B-cell-derived cancer include B-cell acute lymphoblastic leukemia, B-cell chronic lymphocytic leukemia, and B-cell non-Hodgkin lymphoma.

In addition to treating a target disease or disorder such as cancer or an infectious disorder, use of the T cells or cell population of the present disclosure for producing a medicament for a medical treatment of interest is also within the scope of the present disclosure.

In an embodiment, a pharmaceutical composition containing T cells including the chimeric antigen receptor (CAR) or T cell receptor (TCR) and the like of the present disclosure, or a cell population containing such T cells, can be used in cell therapy. In cell therapy, T cells including the chimeric antigen receptor (CAR) or T cell receptor (TCR) and the like of the present disclosure may be infused into a subject in need thereof as a pharmaceutical composition or as a preparation of a therapeutically effective cell population expressing the CAR of the present disclosure. The infused T cells or cell population containing such T cells in the subject may treat a target disease or disorder of the subject, such as cancer or an infectious disorder. The subject may be the same subject from which the cells were obtained (autologous cell therapy), or the cells may be derived from another subject of the same species (allogeneic cell therapy).

In an embodiment, T cells including the CAR, TCR, or the like of the present disclosure, or a cell population containing such T cells, can be formulated for administration to a subject using techniques known to those skilled in the art. In an embodiment, a preparation containing therapeutically effective T cells including the CAR, TCR, or the like of the present disclosure, or a cell population containing such T cells, may contain a pharmaceutically acceptable excipient (carrier or diluent). The excipient contained in the preparation has different purposes depending on, for example, the nature of the antigen-binding domain of the CAR of the present disclosure. Non-limiting examples of commonly used excipients include the following: saline, buffered saline, dextrose, water for injection, glycerol, ethanol, and combinations thereof, stabilizers, solubilizers and surfactants, buffers and preservatives, tonicity agents, bulking agents, and lubricants.

In the present specification, when gene names and products thereof are written, unlike ordinary usage, when they are written in all capital letters, they may refer to both genes and proteins. For example, the FOXP3 gene and the FOXP3 protein may be distinguished from each other, and when written as FoxP3, the notation refers to both the concept and entity of the gene or protein (the entirety).

A preparation containing therapeutically effective T cells including the CAR, TCR, or the like of the present disclosure, or a cell population containing such T cells, may be administered to a subject using methods and techniques known to those skilled in the art. Examples thereof include, but are not limited to, intravenous injection. Other methods include, but are not limited to, intratumoral, intradermal, subcutaneous, intramuscular, intraperitoneal, intraarterial, intramedullary, intracardiac, intraarticular (joint), intrabursal (synovial fluid area), intracranial, intraspinal, and intrathecal (cerebrospinal fluid) administration.

In one embodiment, the present disclosure is provided as a pharmaceutical composition for preventing or treating cancer in a subject, in which the pharmaceutical composition is characterized by: (A) collecting a value of glucose uptake ability or a value of an index related thereto in the subject; (B) selecting T cells with enhanced glucose uptake ability, or a cell population containing the T cells, having appropriate expression characteristics according to the index; and (C) administering the T cells with enhanced glucose uptake ability or the cell population containing the T cells to the subject according to an appropriate administration method and dosage, in which the T cells contain a chimeric antigen receptor (CAR).

In one embodiment, the T cells with enhanced glucose uptake ability include T_{eff} that is not modified to express a glucose transporter in response to demand (on demand) and does not have enhanced expression of a glucose transporter in response to demand (on demand), and cells that are modified to express a glucose transporter in response to demand (on demand) and/or have enhanced expression of a glucose transporter in response to demand (on demand).

In one embodiment, the glucose transporter is GLUT3.

### (General technique)

The molecular biological techniques, biochemical techniques, and microbiological techniques used in the present specification are well known and commonly used in the art, and are described in, for example, Sambrook J. et al. (1989). Molecular Cloning: A Laboratory Manual, Cold Spring Harbor and its 3rd Ed. (2001); Ausubel, F.M. (1987). Current Protocols in Molecular Biology, Greene Pub. Associates and Wiley-Interscience; Ausubel, F.M. (1989). Short Protocols in Molecular Biology: A Compendium of Methods from Current Protocols in Molecular Biology, Greene Pub. Associates and Wiley-Interscience; Innis, M.A. (1990). PCR Protocols: A Guide to Methods and Applications, Academic Press; Ausubel, F.M. (1992). Short Protocols in Molecular Biology: A Compendium of Methods from Current Protocols in Molecular Biology, Greene Pub. Associates; Ausubel, F.M. (1995). Short Protocols in Molecular Biology: A Compendium of Methods from Current Protocols in Molecular Biology, Greene Pub. Associates; Innis, M.A. et al. (1995). PCR Strategies, Academic Press; Ausubel, F.M. (1999). Short Protocols in Molecular Biology: A Compendium of Methods from Current Protocols in Molecular Biology, Wiley, and annual updates; Sninsky, J.J. et al. (1999). PCR Applications: Protocols for Functional Genomics, Academic Press, the special volume of Experimental Medicine, "Experimental Methods for Gene Introduction & Expression Analysis", YODOSHA CO., LTD., 1997, and the like, and the relevant parts thereof (which may be the entirety thereof) are incorporated herein by reference.

For DNA synthesis techniques and nucleic acid chemistry for producing artificially synthesized genes, gene synthesis or fragment synthesis services such as GeneArt, GenScript, and Integrated DNA Technologies (IDT) can also be used. In addition, such techniques and chemistry are described in, for example, Gait, M.J. (1985). Oligonucleotide Synthesis: A Practical Approach, IRL Press; Gait, M.J. (1990). Oligonucleotide Synthesis: A Practical Approach, IRL Press; Eckstein, F. (1991). Oligonucleotides and Analogues: A Practical Approach, IRL Press; Adams, R.L. et al. (1992). The Biochemistry of the Nucleic Acids, Chapman & Hall; Shabarova, Z. et al. (1994). Advanced Organic Chemistry of Nucleic Acids, Weinheim; Blackburn, G.M. et al. (1996). Nucleic Acids in Chemistry and Biology, Oxford University Press; Hermanson, G.T. (I996). Bioconjugate Techniques, Academic Press, and the like, and the relevant parts thereof are incorporated herein by reference.

In the present specification, "or" is used when "at least one or more" of the items listed in the text can be employed. The same applies to "or". When explicitly described in the present specification as "within the range" of "two values", the range also includes the two values themselves.

References such as scientific literature, patents, and patent applications cited in the present specification are incorporated herein by reference in their entirety to the same extent as if each were specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described in the present specification, but is limited only by the claims.

### Examples

In the present Example, various functions of T cells with enhanced glucose uptake ability were examined. As reagents, the products specifically described in the Examples were used, but equivalent products from other manufacturers (Sigma-Aldrich, Wako Pure Chemical Industries, Nacalai, R&D Systems, USCN Life Science INC, and the like) may also be substituted.

### (Example 1; T cells expressing GLUT3)

### (Methods and materials)

### Preparation of GLUT3 3C10-CAR construct and preparation of viral vector

An SLC2A3 sequence (GLUT3), with a P2A sequence disposed immediately upstream thereof, was inserted into an anti-EGFRvIII CAR backbone plasmid (3C10 CAR-Katushka2S) having a structure of CD8 leader sequence-anti-EGFRvIII single-chain variable region (scFv)-CD8 hinge-CD8 transmembrane portion (TM)-CD28 intracellular domain (ICD)-4-1BB ICD-CD3z-P2A-Katushka2S, and a 3C10 CAR-GLUT3-Katushka2S lentiviral vector was constructed (FIG. 1). The lentiviral vector was transfected into 293T cells together with packaging vectors psPAX2 (Addgene #12260) and pMD2.G (Addgene #12259), and lentivirus was obtained from the supernatant. This was introduced into PBMCs containing T cells to obtain T cells expressing CAR and GLUT3.

### Construction of on-demand GLUT3-expressing 3CAR construct

### NFAT-inducible GLUT3-CAR

An NFAT-inducible GLUT3 construct including an eight-repeat NFAT biding site, a minimal promoter (minP), and an SLC2A3 (GLUT3) sequence was inserted into the GLUT3 3C10-CAR plasmid described above, and an NFAT-inducible GLUT3 CAR plasmid was constructed (NFATiGLUT3 CAR: FIG. 15; the same lentiviral plasmid: see FIG. 16). In this system, CAR is driven by an EF1a promoter and is constitutively expressed, and GLUT3 is expressed by sensing nuclear translocation of NFAT associated with activation of CAR-T cells.

### Hypoxia-inducible GLUT3-CAR

A HIF-inducible GLUT3 construct including an xx-repeat HIF1a binding site, minP, and an SLC2A3 (GLUT3) sequence is inserted into the GLUT3 3C10-CAR plasmid described above, and a hypoxia-inducible GLUT3 CAR plasmid is constructed (FIG. 15). As another method, a 2A sequence is disposed downstream of 3C10-CAR, and a conjugate of GLUT3 and an HIF-1α oxygen-dependent degradation domain (oxygen-dependent degradation domain: ODD) is inserted. In both, CAR is driven by an EF1a promoter and is constitutively expressed. In the former, GLUT3 expression is induced by sensing nuclear translocation of NFAT associated with activation of CAR-T cells, and in the latter, GLUT3 continues to be degraded under normal oxygen, but under hypoxia, degradation of GLUT3 by ODD is suppressed, and as a result, expression is induced.

### Gene transfer

Peripheral blood mononuclear cells from healthy subjects were separated from heparinized whole blood by the Ficoll method (Ficoll-Paque PLUS, GE Healthcare), stimulated with anti-CD3/28 beads (Dynabeads T-Activator CD3/CD28, Veritas), and CAR gene transfer was performed at an MOI=4 on day 1 after stimulation. The cells were cultured in the presence of IL-2 30 U/ml and were cryopreserved on days 8 to 12 or directly subjected to each evaluation.

### Immunostaining

1 × 10⁶ GLUT3 3C10 CAR-T cells were fixed with 4% PFA and permeabilized with methanol. Blocking was performed with 3% BSA for 1 hour, and the cells were incubated with an anti-GLUT3 antibody (ab15311) for 2 hours. The cells were washed with 0.05% PBST and incubated with an anti-rabbit IgG antibody (Alexa flour 488) for 1 hour. The cells were washed with 0.05% PBST, and nuclear staining was performed with DAPI. After staining, the cells were adhered to a slide glass using Cytospin (Thermo Fisher Scientific), mounted using VECTASHIELD (VECTOR LABORATORIES), and observed using Keyence XZ-800.

### Phenotype analysis (FCM)

The antibodies used for FCM analysis are shown in Table 1.

**[Table 1]**

| **Antibody** | **Company** | **Product number** |
|---|---|---|
| Anti-human CD3-BV786(SK7) | BD Biosciences | 563800 |
| Anti-human CD4-AF700(SK3) | BD Biosciences | 566909 |
| Anti-human CD4-BUV805(SK3) | BD Biosciences | 612888 |
| Anti-human CD45RA-PerCP-Cy5.5(HI100) | BD Biosciences | 563429 |
| Anti-human CD27-BV711(O323) | Biolegend | 302834 |
| Anti-human CCR7-R718(2-L1-A) | BD Biosciences | 751859 |
| Anti-human CD279(PD-1)-BV421(NIH4) | BD Biosciences | 564323 |
| Anti-human LAG3-FITC(17B4) | Enzo | ALX-804-806F-C100 |
| Anti-human TIM3-BV605(F38-2Σ2) | Biolegend | 345018 |
| Anti-human CD25-PE(M-A251) | BD Biosciences | 560989 |
| Anti-human CCR4-PeCy7(L291H4) | Biolegend | 359410 |
| Anti-human CD183(CXCR3)-APC(G025H7) | Biolegend | 353708 |
| Anti-human IFNr-AF488(4S.B3) | Biolegend | 502515 |
| Anti-human TNFa-APC(MAb11) | Invitrogen | 17-7349-82 |
| Anti-human IL2-PE/Cy7(MQ1-17H12) | Invitrogen | 25-7029-42 |
| Fixable Viability Dye eFluor 780 | Invitrogen | 65-0865-14 |

The cells were washed with 4% FBS-PBS. Dead cell staining and surface staining were performed, and after washing, data were acquired using FACSymphony A3 and analyzed using FACSDiva (v8.0.1, BD Biosciences) and FlowJo (TreeStar) software.

### In vitro CAR-T cell survival and function analysis (FIG. 21)

Long-term survival, memory formation, and maintenance of function of each CAR-T cell were evaluated. CAR-T cells were stimulated with stimulants (medium as a negative control, EGFRvIII stimulation beads, or the U87d cell line) at a ratio of 1:1. After 24 hours, the stimulants were removed by ceramic beads or sorting, and the CAR-T cells were divided into 0 mM, 0.5 mM, and 10 mM groups and continuously cultured. Before stimulation and on day 1, day 3, and day 7 after stimulation, together with cell counting, Annexin V/7AAD for evaluating cell survival and cell death, memory phenotypes such as CD27, CCR4, and CD45RA, inhibitory factors such as PD-1, TIM3, and LAG3 (up to FIGS. 23 to 28), and others (transcription factors, activation/senescence markers) were analyzed. In addition, RNA was also extracted from each sample, and detailed analysis such as RNA sequencing was also performed. After a series of analyses, some of the day 7 cells were stimulated again in the same groups, and cell survival and function analysis after repeated stimulation was performed in the same manner.

### Cytokine analysis (FCM)

1 × 10⁵ CAR-T cells and 5 × 10⁵ cells of an EGFRvIII-expressing U87 cell line (U87Δ) were co-cultured in 1 ml of medium for 12 hours, and then cultured for 6 hours in the presence of 5 µg/ml Monensin (BD 554724, BD Bioscience), thereby stimulating the CAR-T cells. As the medium, glucose-free RPMI1640 (Wako) supplemented with 10% dialyzed FBS (Cytiva) and adjusted to glucose concentrations of 10 mM and 0.5 mM with a glucose solution (Gibco) was used. Fixation was performed with Fixation/Permeabilization Diluent (Invitrogen); intracellular cytokines were stained with the antibodies shown in Table 1; data were acquired using an LSRFortessa X20; and the data were analyzed using FACSDiva (v8.0.1, BD Biosciences) and FlowJo (TreeStar) software.

### Cytotoxic activity analysis

Target cells (luciferase-expressing U87Δ cells) were seeded in a 96-well plate at 2 × 10⁵ cells per well and co-cultured with CAR-T cells at E : T ratios of 3 : 1, 1 : 1, 0.3 : 1, and 0.1 : 1. After 16 hours, viable cells were caused to luminesce using VivoGlo Luciferin (Promega); luciferase activity was measured using Cytation (Promega); and cytotoxic activity was calculated.

### Metabolic analysis

Metabolic ability of the cells was measured using an XFe24 cell flux analyzer (Bioscience) and an XF Glycolysis Stress Test Kit (Agilent Technologies) according to the product protocol. On the day before analysis, a 24-well flat-bottom plate for analysis was coated with poly-D-lysine (0.1 mg/ml), and a sensor cartridge was hydrated in a CO₂-free incubator. On the day of analysis, the cells for analysis were washed using analysis medium (Seahorse XF RPMI medium, 2 mM L-glutamine), seeded at 2 × 10⁵ cells each in the analysis plate coated with poly-D-lysine, and allowed to stand at 37°C under CO₂-free conditions for 60 minutes. After the sensor cartridge ports were loaded with glucose at a final concentration of 10 mM or 0.5 mM, oligomycin at a final concentration of 1 µM, 2-DG at a final concentration of 50 mM, and, as appropriate, oligomycin and FCCP so as to give final concentrations of 20 µM and 10 µM, respectively, analysis was performed using the flux analyzer, and metabolic function was evaluated based on OCR and ECAR.

### Evaluation of glucose uptake ability

2 × 10⁴ GLUT3 CAR-T cells were seeded in a 96-well plate and cultured in 200 µl of glucose-free RPMI1640 (Wako) for 5 hours. Five minutes before analysis, 1 µl of 2-NDBG (abcam) was added to each well, and after washing, data were acquired using LSRFortessa X20 and analyzed using FACSDiva (v8.0.1, BD Biosciences) and FlowJo (TreeStar) software.

### Intracranial tumor xenograft model

2.5 × 10⁴ cells from the luciferase-expressing U87Δ tumor cell line (U87Δ-luc) were infused intracranially into the right basal cerebrum of NSG mice, and after engraftment, Mock cells, 3C10 CAR-T cells, or GLUT3 CAR-T cells were each administered at 2 × 10⁶ cells via the tail vein. Tumor burden was monitored over time by in vivo imaging, and survival was observed. In a tumor rechallenge test for verifying long-term survival, memory formation, and recurrence-preventing effect of CAR-T cells, the same tumor cells (U87Δ-luc) 2.5 x 10⁴ cells (FIG. 13) or 1.0 x 10⁵ cells (FIG. 19) were intracranially administered again to mice in which CR had been achieved after the above treatment, and BLI and survival were monitored.

### (Method)

FIG. 1 shows the structure of a metabolically modified CAR (EGFRvIII_CAR_GLUT3). A single-chain antibody (asFv) derived from a mouse-derived antibody (clone 3C10) targeting EGFRvIII expressed in glioblastoma (GBM) was constructed and linked with CD8 hinge, a CD28 transmembrane portion (CD28TM), a CD28 intracellular domain (CD28ICD), 4-1BB ICD, and CD3z to construct a CAR. A high-affinity glucose transporter (GLUT3) was encoded downstream of the CAR via a P2A sequence.

### (Results)

The results are shown in FIG. 2 and subsequent drawings. As shown in FIG. 2, expression of GLUT3 on the T cell surface and enhancement of glucose uptake are shown. A conventional CAR (3C10 CAR) and a metabolically modified CAR (GLUT3 CAR) were introduced into healthy donor-derived T cells by gene transfer to obtain CAR-T cells. Uniform GLUT3 expression was observed on the membrane of GLUT3 CAR-T cells by fluorescence microscopy. In addition, in the GLUT3 CAR-T cells, glucose uptake was enhanced compared with T cells and conventional CAR-T cells (3C10 CAR-T cells).

Next, T cell expansion efficiency comparable to that of conventional CAR-T cells is shown in FIG. 3. As shown in FIG. 3, CAR was genetically introduced after stimulation with anti-CD3/28 beads, and T cell proliferation efficiency was analyzed. GLUT3 CAR-T cells had establishment efficiency comparable to that of conventional CAR-T cells (3C10 CAR-T cells).

Next, enhancement of T cell glycolytic ability by GLUT3 expression is shown in FIG. 4. As shown, metabolism of GLUT3 CAR-T cells and 3C10 CAR-T cells was analyzed using a Flax analyzer. GLUT3 CAR enhanced glycolytic ability in both a low-glucose environment and a normal-glucose environment.

Next, it was shown that GLUT3 CAR-T cells have a competitive advantage under low glucose (FIG. 5). As shown in FIG. 5, 3C10 CAR-T cells and GLUT3 CAR-T cells were co-cultured under normal glucose and under low glucose. Under normal glucose, survival of both was observed to be comparable, whereas under low glucose, significant survival of GLUT3 CAR-T cells was observed, suggesting that GLUT3 CAR-T cells were capable of advantageous glucose uptake.

Next, enhancement of cytokine production ability of GLUT3 CAR-T cells is shown in FIG. 6. As shown in FIG. 6, after antigen stimulation of 3C10 CAR-T cells and GLUT3 CAR-T cells, the proportion of cytokine-producing cells was analyzed by FCM. A significant increase in expression of cytokines (IFN-g, IL-2, TNF-a), which are important for T cell maintenance and activation and exertion of antitumor activity, was observed.

Next, the function of GLUT3 CAR-T cells maintained even under low glucose is shown in FIG. 7. As shown, cytokine production ability under normal glucose and under low glucose was compared. In conventional CAR-T cells (3C10 CAR-T cells), an extreme decrease in function under low glucose conditions was observed. On the other hand, in GLUT3 CAR-T cells, high cytokine production ability was observed under normal glucose conditions, and even under low glucose conditions, GLUT3 CAR-T cells exerted a function comparable to the cytokine production ability of 3C10 CAR-T cells under normal glucose conditions.

Next, enhanced cytotoxic activity of GLUT3 CAR-T cells is shown in FIG. 8. As shown, cytotoxic activity was analyzed using an EGFRvIII-expressing cell line as a target. Cytotoxic activity was analyzed under low glucose conditions and high glucose conditions, and GLUT3 CAR-T cells showed higher cytotoxic activity than 3C10 CAR-T cells under both environments.

Next, properties imparted by GLUT3 CAR to the memory phenotype are shown in FIG. 9. As shown, the memory phenotypes of 3C10 CAR-T cells and GLUT3 CAR-T cells were compared. In GLUT3 CAR-T cells, a tendency toward differentiation into effector T cells was observed.

Next, suppression of inhibitory molecule expression in GLUT3 CAR-T cells is shown in FIG. 10. Expression of inhibitory molecules was compared under unstimulated conditions and under stimulation with EGFRvIII antigen-positive cells. In GLUT3 CAR-T cells, suppression of expression of PD-1, LAG3, and Tim3 was observed.

Next, mRNA analysis of GLUT3 CAR-T cells (evaluation of metabolism, exhaustion, activation, and differentiation) is shown in FIG. 11. Details of 3C10 CAR-T and GLUT3 CAR-T cells were analyzed based on mRNA expression. In GLUT3 CAR-T cells, as with FCM, a decrease in exhaustion-related molecules and increases in lactate metabolism- and glycolysis-related factors were observed. Overall, the data show enhancement of T cell effector functions such as activation and cytokine production.

Next, FIG. 12 shows that the same effect is obtained when GLUT3 is incorporated into CD19 CAR as well as 3C10 CAR, that is, universality is demonstrated. It was confirmed that, when GLUT3 was incorporated into CD19 CAR, an effect similar to that of 3C10 CAR was obtained. In addition, CD19 was expressed in a pancreatic cancer cell line, and it was shown that an effect was also obtained against pancreatic cancer.

As shown in FIG. 13, rapid tumor eradication was achieved in an intracranial xenograft model. Antitumor activity was observed in a U87Δ intracranial xenograft model. In GLUT3 CAR-T cells, more rapid tumor eradication than in 3C10 CAR-T cells was observed. In addition, rejection was confirmed by tumor challenge.

### (Example 2: On-demand expression)

This was performed for on-demand GLUT3-expressing CAR. An outline thereof is shown in FIG. 14.

FIG. 14 shows an outline of on-demand GLUT3-expressing CAR. This is a system in which, in a steady state, only CAR is expressed and GLUT3 is not expressed, and GLUT3 is expressed when glucose demand increases, such as upon activation of CAR-T cells or in a tumor environment. In order to achieve on-demand expression of GLUT3, CAR constructs that express GLUT3 specifically in a tumor environment or specifically upon activation were designed (FIG. 15). GLUT3 expression in response to an increase in NFAT due to activation (A.) and tumor hypoxia (B.) is enabled.

Using cells into which the CAR of FIG. 15 was introduced, the function and antitumor effect thereof are analyzed.

### (Example 3: Enhanced expression of glucose transporter)

In order to achieve enhanced expression of a glucose transporter, a CAR construct that constitutively expresses GLUT3 was designed (stbl-GLUT3 in FIG. 15 = GLUT3 CAR).

Using cells into which the CAR of FIG. 15 was introduced, the function and antitumor effect thereof are analyzed.

FIG. 16 shows the structure of the NFATi-GLUT3 CAR plasmid. Details of the structure are as follows. In FIG. 16, CAR and inducible GLUT3 are incorporated into a lentiviral vector. A CAR consisting, in this order, of a single-chain antibody (3C10VH-linker-3C10VL) derived from a mouse-derived antibody (clone 3C10) targeting EGFRvIII expressed in glioblastoma (GBM) and the like, CD8 hinge, a CD28 transmembrane portion, a CD28 intracellular domain (CD28I), 4-1BB, and CD3z, is encoded. Katushka, which is a gene introduction marker, is encoded downstream of CAR via a P2A sequence. In addition, human GLUT3 (hGLUT3) is encoded under the control of an eight-repeat NFAT binding site and a minimum promoter.

FIG. 17 shows the operation and basic function of the on-demand (inducible) GLUT3 CAR system of the present disclosure. A system that expresses GLUT3 in association with NFAT nuclear translocation was constructed (NFATi GLUT3 CAR-T), and its operation was confirmed. The cytotoxic activity of NFATiGLUT3 CAR-T cells (NFATi GLUT3 CAR-T) was equivalent to that of GLUT3 CAR-T cells with constitutive expression (GLUT3 CAR-T), and cytokine production was at an intermediate level between 3C10 CAR-T (without GLUT3) and GLUT3 CAR-T cells with constitutive expression (GLUT3 CAR-T).

FIG. 18 shows the results of evaluating the antitumor effect of NFAT1GLUT3 CAR-T cells in a similar mouse model. CR was obtained in four mice with GLUT3 CAR-T and in 2 mice with NFATi GLUT3 CAR-T.

FIG. 19 shows the results of the tumor challenge and tumor rechallenge experiments. That is, the drawing shows the state of sudden death of mice in the GLUT3 CAR-T cell group. Tumor (U87d) was re-transplanted into cured mice from the experiment in the previous figure, and rejection ability was evaluated ("surrogate for memory formation"). 1 x 10⁵ tumor cells (four times the amount in the previous experiment) were re-transplanted, and tumor rejection was not achieved with 3C10 CAR-T, whereas rejection was achieved with GLUT3 CAR-T and NFATiCAR-T. However, in the GLUT3 CAR-T group, three out of four mice died (death unrelated to tumor growth).

FIG. 20 shows that apoptosis after stimulation was suppressed in NFATiGLUT3. CAR-T cells were repeatedly stimulated with U87d, and CAR-T cell apoptosis was observed using Annexin V and 7-ADD.

FIG. 21 shows that long-term survival after various stimulations was enhanced. Each CAR-T cell is observed over time under the above conditions. As a result, under low glucose, conventional CAR-T (3C10) does not exhibit effective cytokine production, whereas GLUT3 CAR-T and NFATi GLUT3 CAR-T exhibit effective cytokine production ability. However, GLUT3 CAR-T cells, due to excessive glucose uptake, differentiate over time (day 3 or day 7) into CCR7-negative Tem or Temra, undergo terminal differentiation, lose stemness, and undergo apoptosis. On the other hand, in NFATi GLUT3 CAR-T cells, which are on-demand-type cells, apoptosis is suppressed, and CCR7-positive Tcm and Tscm (memory cells, cells having stemness) are maintained.

In addition, when CAR-T cells (established under normal glucose conditions) are suddenly (day 0) placed under low-glucose conditions of 0 mM or 0.5 mM, GLUT3 CAR-T cells, which are highly glucose-dependent, are prone to apoptosis (conversely, NFATi GLUT-3 CAR-T cells show resistance to low glucose).

FIG. 22 shows that long-term survival after stimulation is enhanced. The above antitumor effect, survival, and adverse events are examined in a system similar to that of the mice described above. In Experiment 1, GLUT3 CAR-T and NFATi GLUT3 CAR-T show equivalent antitumor activity. In Experiment 2, apoptosis and over-differentiation occur due to GLUT3 CAR-T overactivation caused by glucose exposure, and antitumor activity is lost. On the other hand, in NFATiCAR-T cells, excessive activation is suppressed, and therefore an effective antitumor effect is exerted. Also in rechallenge, NFATiCAR-T cells reject tumors. In Experiment 4, in proportion to the tumor effect, predominant CAR-T cell engraftment and tumor T-cell infiltration are observed with NFATiGLUT3. In addition, Experiment 3 is an experiment in a liver tumor model in which immunosuppression due to a low-glucose, high-lactic-acid environment has been suggested, and results similar to those in Experiment 2 are expected to be obtained.

FIG. 23 shows data (in vitro data) showing enhancement of long-term survival after stimulation. At each time point on the left, staining data for 7-AAD and Annexin V, which reflect cell death and apoptosis, are shown. Cell death in each CAR-T cell was examined using Annexin V and 7-AAD. In an unstimulated state, differences depending on glucose concentration were observed, but no substantial differences were observed among the respective CAR-T cells.

FIG. 24 presents in vitro data showing enhancement of long-term survival after stimulation. As shown, survival and cell death of each CAR-T cell after stimulation were evaluated in the same manner as in the previous figure. Compared with 3C10 CAR-T cells and GLUT3 CAR-T cells, survival was enhanced (cell death was suppressed) in NFATi GLUT3 CAR-T cells.

FIG. 25 shows in vitro data showing enhancement of long-term survival after stimulation. The proportion of CAR-positive cells was analyzed for each CAR-T cell population after stimulation. After stimulation, the proportion of CAR-positive cells decreased, particularly in GLUT3 CARCAR-T cells with constitutive expression, and it was considered that this fraction had undergone cell death.

FIG. 26 shows in vitro data showing enhancement of long-term survival after stimulation. T-cell counts on day 7 are shown. As a model reflecting post-administration dynamics in a patient body (dynamics in which, after administration, cells infiltrate from blood having a sufficient glucose concentration into a tumor environment, which is a low- to no-glucose environment), cells were established and cultured under normal glucose conditions (glucose 10 mM), stimulated with EGFRvIII-binding beads, then cultured under no-glucose (0 mM), low-glucose (0.5 mM), or normal-glucose (10 mM) conditions, and the viable cell count after 7 days was evaluated. The graph shows the relative cell number, with the Mock T-cell count set to 1. In GLUT3 CAR-T, almost all cells had died under 0 mM. On the other hand, in NFATi CAR-T, many cells were maintained. It was suggested that cell death may have resulted from overactivation caused by constitutive GLUT3 expression and metabolism highly dependent on glucose.

FIG. 27 shows expression of an inhibitory molecule (PD-1). In NFATi GLUT3 CAR-T cells, expression of the inhibitory molecule (PD-1) was lower than that in GLUT3 CAR-T cells.

FIG. 28 shows suppressed expression of an inhibitory molecule (TIM3). In NFATi GLUT3 CAR-T cells, expression of the inhibitory molecule (TIM3) was lower than that in GLUT3 CAR-T cells.

### (Example 4: Example using precursor cells of other effector T cells)

Similar experiments are performed using other T cells. For example, unpurified peripheral T cells contain Tnv, Tcm, Tem, Temra, and the like, and CAR (CAR, constitutive GLUT3 CAR, or on-demand GLUT3 CAR) can be introduced into any of these fractions.

In the present Example, CAR (CAR, constitutive GLUT3 CAR, or on-demand GLUT3 CAR) is introduced into any purified and enriched T cell memory fraction. In another experiment, CAR-T cells enriched with any T cell memory fraction are induced under culture conditions.

### Experimental procedure:

From a T cell source such as peripheral blood, bone marrow, or umbilical cord blood, Tnv, Tscm, and Tcm, which are precursor cells of T_{eff} and are expected to have longer-term survival and maintenance of stemness, or T_{eff} and Temra fractions, which have already differentiated and mainly exert effector functions, are purified and enriched using FACS sorting or magnetic beads based on T cell memory-related surface antigen expression patterns, namely CD45RA⁺CCR7⁺CD95⁻, CD45RA⁺CCR7⁺CD95⁺, CD45RA⁻CCR7⁺, CD45RA⁻CCR7, and CD45RA⁺CCR7⁻, respectively.

Each fraction is stimulated with CD3/28 beads or the like, and CAR is introduced using a lentiviral vector.

In addition, as another method, after gene transfer into bulk T cells, the type and amount of cytokines added are adjusted (by using IL-7 or IL-15, or platelet lysate) to suppress T_{eff} differentiation and induce predominant proliferation of Tnv, Tscm, and Tcm, thereby obtaining precursor cell-dominant CAR-T cells.

In vitro evaluation: These cells are co-cultured with a U87d tumor cell line or EGFRvIII beads, and after 1 day, 3 days, and 7 days, division and proliferation by CFSE staining and cell counting, and activation and exhaustion states by flow cytometry are evaluated.

In vivo evaluation: These cells are administered to the above-described U87d intracranial pharmaceutical model mice, and the antitumor effect is evaluated based on antitumor activity by BLI and survival. In addition, in mice in which cure is observed, the tumor is administered again (rechallenge), and tumor rejection is evaluated.

### Results:

Compared with cases in which CAR was introduced into bulk T cells or into more differentiated T_{eff} or Temra, CAR-T cells in which CAR was introduced into precursor cells of T_{eff}, such as Tnv, Tscm, and Tcm, or in which Tnv, Tscm, and Tcm were predominantly expanded, are expected to have high division and proliferation abilities, and long-term division is expected to be observed. In addition, expression of PD1, TIM3, and LAG3 is expected to be low, and the cells are expected to be resistant to exhaustion.

In addition, these CAR-T cells are shown to have superior antitumor activity and improved survival in a mouse model. In addition, efficient tumor rejection is expected to be observed in tumor rechallenge (a surrogate for long-term effects and prevention of recurrence).

### (Example 5: Demonstration example)

In the present Example, an example demonstrated on a larger scale is provided.

### Materials and methods

### Cell lines

U-87 MG and U-251 MG GBM cell lines were purchased from American Type Culture Collection (ATCC) and Japanese Collection of Research Bioresources Cell Bank, respectively, and cultured in D-MEM (FUJIFILM Wako Pure Chemical Corporation, Osaka, Japan) supplemented with 10% fetal bovine serum (FBS) and 1% penicillin/streptomycin. An EGFRvIII-packaged lentiviral vector was introduced into U-87 MG and U-251 MG cells, and the transduced cell lines were named U-87 MGΔ and U-251 MGΔ, respectively. SUP-T1, NALM6, and AsPC-1 cells were purchased from ATCC and cultured in RPMI1640 (FUJIFILM Wako Pure Chemical Corporation) supplemented with 10% FBS and 1% penicillin/streptomycin. A CD19 truncated vector was introduced into NALM6 cells, and a CD19-expressing AsPC1 cell line was obtained.

### Flow cytometry assay

Flow cytometry assays were performed as previously described [References 40 to 43]. Briefly, cells were washed twice with FACS buffer, and Fc blocking was performed using an Fc receptor blocking solution (BioLegend, San Diego, CA) at 4°C for 10 minutes in the dark. The cells were washed twice with FACS buffer; antibodies targeting cell surface molecules were added; and the cells were incubated at 4°C for 20 minutes in the dark. Thereafter, the cells were washed twice with FACS buffer. For intracellular antigen staining, the cells were incubated in fixation/permeabilization solution (BD Biosciences, San Jose, CA) at room temperature for 1 hour, and then washed twice with wash buffer (BD Biosciences) according to the manufacturer's instructions. Antibodies targeting intracellular antigens were added, and after incubation at 4°C for 20 minutes in the dark, the cells were washed twice. For the apoptosis assay, the cells were washed once with FACS buffer and once with Annexin binding buffer. Thereafter, the cells were stained with Annexin V and 7-AAD at room temperature for 15 minutes. After washing, flow cytometry (FCM) analysis was performed using LSRFortessa X-20 cytometer (BD Biosciences), and analysis was performed using FlowJo ver.10 software (BD Biosciences). Staining solutions were prepared according to the manufacturer's instructions.

### Cytokine staining

Intracellular cytokine staining was performed as previously described [References 40 to 43]. During the last 5 hours of 6-hour T cell stimulation, monensin was added to the medium to retain cytokines inside the cells. After staining cell surface markers, Cytofix/Cytoperm reagent (BD Biosciences) was added, and the cells were incubated at 4°C for 20 minutes in the dark. Thereafter, the cells were washed twice with wash solution, and antibodies targeting cytokines were added. The cells were incubated at 4°C for 20 minutes in the dark. After washing, FCM analysis was performed using LSRFortessa X-20 cytometer (BD Biosciences), and analysis was performed using FlowJo ver.10 software (BD Biosciences). The antibodies used for cell staining are summarized in the table. Antibodies for staining were prepared according to the manufacturer's instructions.

**[Table 2]**

| **Reagent (antibody)** | **Clone** | **Supplier** | **Catalog number** |
|---|---|---|---|
| Anti-Glucose Transporter GLUT3 + GLUT14 | EPR3915 | Abcam | cat# ab315168 |
| Anti-Glucose Transporter GLUT1 | SPM498 | Abcam | cat#ab40084 |
| Goat Anti-Rabbit IgG H&L Alexa Fluor 488 | Not available | Abcam | cat#ab150077 |
| Goat Anti-Mouse IgG H&L Alexa Fluor 647 | Not available | Abcam | cat#ab150115 |
| Anti-human CD197 (CCR7) PE/Cyanine7 | G043H7 | BioLegend | cat#353226 |
| anti-human CD45RA Alexa Fluor 488 | HI100 | BioLegend | cat#304114 |
| anti-human CD366 (Tim-3) Brilliant Violet 605 | F38-2E2 | BioLegend | cat#345018 |
| Anti-human IFN-γ Alexa Fluor 488 | 4S.B3 | BioLegend | cat#502515 |
| Anti-human CD279 (PD-1) Brilliant Violet 421 | MIH4 | BD | cat#564324 |
| Anti-human CD3 Brilliant Violet 786 | SK7 | BD | cat#563800 |
| Anti-human CD4 APC-R700 | SK3 | BD | cat#566909 |
| Anti-human CD8 Brilliant Violet 521 | SK1 | BD | cat#563919 |
| Anti-human TNF alpha APC | Mab11 | Invitrogen | cat#17-7349-82 |
| Anti-human IL-2 PE/Cy7 | MQ1-17H12 | Invitrogen | cat#25-7029-82 |

### Killing assay

A total of 1 x 10⁵ luciferase-expressing cells (U-87 MGΔ, U-251 MGΔ, AsPC-1, and NALM6) were co-cultured with CAR-T cells at the indicated ratios in 200 µL of RPMI 1640 medium (manufactured by FUJIFILM Wako Pure Chemical Corporation) supplemented with low glucose (0.5 mM) or high glucose (10 mM) in flat-bottom 96-well white plates. After 24 hours of culture, Bio-Glo (Promega, Madison, WI) was added to each well, and luminescence was measured using a Cytation5 multiplate reader (Agilent Technologies, Santa Clara, CA). % specific lysis was calculated according to the following formula: % specific lysis =% specific lysis= [(experimental lysis - spontaneous lysis)/(maximum lysis - spontaneous lysis)] × 100.

### Immunofluorescence staining

After washing, the cells were fixed with 4% paraformaldehyde for 20 minutes at room temperature and permeabilized with cold methanol for 10 minutes at -20°C. The cells were blocked with 3% BSA/PBS for 60 minutes at room temperature. After blocking, the cells were incubated with a primary antibody at 4°C for 2 hours and with a secondary antibody at 4°C for 1 hour. Next, DAPI was added, and the cells were incubated at room temperature for 5 minutes. After washing, the stained cells were fixed onto slides using Cytospin and analyzed using BZ-X710 (KEYENCE Corporation, Osaka, Japan).

### Immunohistochemical (IHC) staining

IHC staining was performed on 5 µm-thick sections of formalin-fixed paraffin-embedded specimens. Antigen removal was performed using citrate buffer (pH 6.0) in a steamer, and then, deparaffinization and rehydration were performed. The slides were incubated with a primary antibody for 16 hours and with an HRP-labeled secondary antibody for 1 hour, and then developed with a diaminobenzidine substrate. An anti-CD3ε antibody (clone: SP7, catalog number: ab16669) (Abcam, Waltham, MA) was used for primary staining. The stained slides were counterstained with hematoxylin. Hematoxylin and eosin (HE) staining was performed according to a standard protocol. The deparaffinized and hydrated slides were placed in hematoxylin solution and incubated for 4 minutes. After washing, the slides were incubated with eosin solution for 2 minutes. The stained slides were scanned at magnifications of 40x and 400x using BZ-X710 (Keyence Corporation). Two pathologists independently evaluated the stained slides.

### ELISA method

A total of 2.0 × 10⁵ U-87 MGΔ cells and 2.0 × 10⁵ CAR-T cells were co-cultured in a 24-well plate. After 24 hours of culture, the supernatant was collected and subjected to ELISA to measure cytokine concentrations. ELISA kits for IFN-γ, IL-2, and TNFα (R&D Systems, Minneapolis, MN) were used according to the manufacturer's instructions. Cytokines in mouse serum were analyzed by a high-sensitivity LUMINEX assay according to the manufacturer's instructions (Merck Millipore, Burlington, MA).

### Preparation of CAR-T cells

Peripheral blood was collected from healthy subjects, and peripheral blood mononuclear cells (PBMCs) were isolated by density gradient centrifugation using Ficoll-Paque (GE Healthcare, Chicago, IL). T cells were isolated from PBMCs by negative selection using MojoSort (BioLegend). The isolated T cells were stimulated with anti-CD3/28 beads (Thermo Fisher Scientific, Waltham, MA) at a T cell-to-bead ratio of 1:1 (day 0). At 24 hours after stimulation (day 1), the T cells were transduced with CAR lentivirus at an MOI of 3 and cultured in 30 U/ml IL-2. Half of the medium was replaced after 24 hours. After 5 days, the CD3/28 beads were removed. During culture, half of the medium was replaced on days 4 and 10, and the concentration of T cells was adjusted to 0.7 × 10⁶ cells/mL. The developed CAR-T cells were collected on day 10 and subjected to subsequent analysis.

### Animal model

Female NSG mice (6 weeks old) were purchased from Jackson Laboratory. Before painful procedures, an anesthetic was intraperitoneally injected to prevent pain. Using a stereotaxic frame, tumor cells were inoculated at a position 2 mm to the right of bregma, 3 mm posterior to bregma, and 3 mm deep from the surface of the brain. A total of 2.0 × 10⁴ cells in 5 mL of PBS were injected over 1 minute, and then the needle was withdrawn by 1 mm and left for an additional 1 minute before being removed. Four days after tumor implantation, tumor growth was confirmed by bioluminescence imaging (BLI), and random assignment to each treatment group was performed. On day 5, 1.0 × 10⁶ CAR-T cells were injected via the tail vein. Tumor growth after CAR-T cell injection was monitored twice weekly by BLI. Animal care and experiments were performed in accordance with the guidelines of the Animal Committee of the National Cancer Center after approval by the Institutional Animal Care and Use Committee of the National Cancer Center.

### Glucose concentration measurement

Tissue samples (5 × 5 mm) were washed to remove attached blood. The samples were minced and centrifuged at 4°C for 10 minutes, and tumor interstitial fluid was collected. The glucose concentration in the interstitial fluid was measured using Multiskan GO (Thermo Fisher Scientific) according to the manufacturer's protocol.

### Metabolic analysis

OCR (unit: pmol/min) and ECAR (unit: mpH/min) were evaluated using a Seahorse XF-24 metabolic extracellular flux analyzer (Agilent Technologies). CAR-T cells (conv EGFRvIII CAR-T cells, GLUT3 EGFRvIII CAR-T cells, and on-demand GLUT3 EGFRvIII CAR-T cells) stimulated with EGFRvIII beads (ACRO Biosystems, Beijing, China) for 24 hours were resuspended in glucose-free unbuffered RPMI-1640 medium (Agilent Technologies) and plated on Seahorse cell plates coated with poly-L-lysine (BD Bioscience) (2.0 × 10⁵ cells per well). Perturbation profiling of metabolic pathway utilization by CAR-T cells was achieved by addition of glucose (10 mM or 0.5 mM), oligomycin (1 µM), and 2-deoxy-D-glucose (50 mM) (all manufactured by Agilent Technologies). Experiments using the Seahorse system were performed under the following assay conditions: mixing for 3 minutes, waiting for 2 minutes, and measurement for 3 minutes. Thereafter, values of metabolic parameters were calculated.

### RNA sequencing and subsequent analysis

CAR-T cells (conv EGFRvIII CAR-T cells, GLUT3 EGFRvIII CAR-T cells, and on-demand GLUT3 EGFRvIII CAR-T cells) sorted using BD FACSymphony S6 (BD Biosciences) were stimulated with EGFRvIII beads at a ratio of 1:1 under low-glucose (0.5 mM) or high-glucose (10 mM) conditions, supplemented with 30 U/ml IL-2. CAR-T cells were collected on days 3 and 7, and RNA was extracted using an RNeasy kit (QIAGEN, Hilden, Germany) according to the manufacturer's instructions. Complementary DNA (cDNA) was prepared from the isolated RNA using NEBNext Ultra Directional RNA Library Prep Kit (New England BioLabs, Ipswich, MA). RNA-seq libraries were subjected to 150-bp next-generation sequencing from both ends (paired-end reads) using Novaseq X plus (Illumina, San Diego, CA). For expression profiling using RNA-seq data, paired-end reads were aligned to the hg38 human genome assembly by STAR [Reference 44]. RNAseqChef is a web-based platform for systematic transcriptome analysis, gene expression analysis, principal component analysis, and pathway analysis [Reference 45].

### Statistical analysis

GraphPad Prism 9 (GraphPad Software, San Diego, CA) was used for statistical analysis. Relationships between groups were compared by a t-test or one-way analysis of variance. Survival was analyzed by the Kaplan-Meier method and statistically compared by the log-rank test. A P value of <0.05 was considered statistically significant.

### Results

GBM has low glucose levels in the TME, which impair the function of CAR-T cells.

Although accumulating evidence indicates that low glucose levels in the TME are a potential barrier to CAR-T cell therapy in solid cancers, the actual glucose concentration in the TME and how a low-glucose state affects the function of CAR-T cells have not yet been clarified [Reference 21]. The present inventors examined glucose concentrations in interstitial fluid from surgical specimens and in serum (FIG. 1a). The glucose concentration in the interstitial fluid of GBM specimens was about 10-fold lower than that in serum [less than 0.5 mM (mean: 0.217 mM)] (FIG. 1b). Glucose concentrations are generally low in various types of cancers, such as non-small cell lung cancer and colorectal cancer, but GBM had the lowest glucose concentration in the TME. This indicates that a low glucose concentration in the TME is a characteristic of GBM (FIG. 1c).

Next, the present inventors examined how a low-glucose environment affects functions of CAR-T cells, such as cytokine production. CAR-T cells targeting EGFRvIII, which is a GBM-related antigen (conv EGFRvIII CAR-T cells), were co-cultured with a human GBM cell line expressing EGFRvIII (U-87 MGΔ) under low-glucose conditions (0.5 mM) that reproduced the TME of GBM. Upon stimulation with U-87 MGΔ, production of cytokines (IFN-γ, IL-2, and TNFα) was significantly decreased under the low-glucose environment, even after short-term (16-hour) exposure (FIGS. 29d and 29e). To further examine functional changes in CAR-T cells under low-glucose conditions, CAR-T cells were stimulated for 16 hours under low-glucose (0.5 mM) or high-glucose (10 mM) conditions, and gene expression profiles were analyzed. The gene expression profiles were significantly altered by exposure to the low-glucose conditions (FIG. 35a). Gene sets related to the cell cycle, differentiation, and cytokine production were significantly decreased, whereas gene sets related to cellular responses to glucose starvation and intrinsic apoptosis were increased under the low-glucose conditions (FIGS. 29f and 29g). As described above, the effector functions of CAR-T cells are immediately impaired when exposed to low-glucose conditions such as the TME of GBM.

Overexpression of GLUT3 enhances metabolic fitness of CAR-T cells.

Among the SLC2 family of glucose transporters (GLUTs), GLUT3, which is encoded by SLC2A3, has the highest affinity and is characterized by predominant expression in neurons and glial cells, which have a high demand for glucose [Reference 16]. Therefore, the present inventors examined whether expression of GLUT3 by CAR-T cells could promote the activity of CAR-T cells by promoting glucose uptake in the low-glucose TME of GBM. SLC2A3 was fused to a conventional anti-EGFRvIII CAR construct via a self-cleaving P2A sequence, thereby enabling simultaneous stable expression of CAR and GLUT3 (GLUT3 EGFRvIII CAR) (FIG. 30a). GLUT3 was expressed on the membrane (FIG. 30b) of T cells into which GLUT3 EGFRvIII CAR had been introduced (GLUT3 EGFRvIII CAR-T cells), and the expression level thereof was approximately twice that of the parental anti-EGFRvIII CAR-T cells (conv EGFRvIII CAR-T cells) and comparable to that of the GBM cell line U-87 MGΔ, whereas no change was observed in the expression level of GLUT1 (FIGS. 30c and 36a). The amount of glucose uptake measured by a glucose uptake assay using the glucose analog 2-NBDG was greater in GLUT3 EGFRvIII CAR-T cells than in conv EGFRvIII CAR-T cells (FIG. 30d). Accordingly, glycolysis and glycolytic capacity of GLUT3 EGFRvIII CAR-T cells were significantly improved (FIGS. 30e and 36b). Metabolic analysis further confirmed that GLUT3 EGFRvIII CAR-T cells shifted to a more energetic state with high oxygen consumption rate (OCR)^{high} and high extracellular acidification rate (ECAR)^{high}, compared with conv EGFRvIII CAR-T cells (FIG. 30f). In a glucose competition assay in which GLUT3 EGFRvIII CAR-T cells labeled with carboxyfluorescein succinimidyl ester (CFSE) were co-cultured with conv EGFRvIII CAR-T cells at a ratio of 1 : 1, the number of GLUT3 EGFRvIII CAR-T cells relative to conventional cells increased at a low glucose level (0.5 mM) glucose level, but was comparable under high-glucose (10 mM) conditions, suggesting that GLUT3 EGFRvIII CAR-T cells are substantially competitive in terms of glucose uptake, particularly in a low-glucose environment (FIGS. 36c and 36d). Therefore, stable GLUT3 expression by CAR-T cells promotes glucose uptake, makes CAR-T cells competitive in terms of glucose uptake, and enhances metabolic fitness of CAR-T cells in a limited-glucose environment.

Since expression of GLUT3 improved the metabolic fitness of CAR-T cells, the effector function and safety profile thereof were examined. Therefore, the present inventors examined the effector function of GLUT3 EGFRvIII CAR-T cells in both in vitro and in vivo assays. Compared with conv EGFRvIII CAR-T cells, GLUT3 EGFRvIII CAR-T cells significantly increased cytokine production (IFN-γ, IL-2, and TNFα) at both low glucose levels (0.5 mM) and high glucose levels (10 mM) (FIGS. 30g and 30H, and FIG. 37a). Cytokine production was enhanced in both CD8⁺ T cell subsets and CD4⁺ T cell subsets (FIGS. 37b and 37c). GLUT3 EGFRvIII CAR-T cells also showed increased cytotoxicity against U-87 MGΔ cells and U-251 MGΔ cells, another EGFRvIII-expressing GBM cell line, under both low-glucose and high-glucose conditions (FIG. 30i and FIGS. 37d and 37e). Expression of exhaustion-related molecules including PD-1 and Tim-3 after antigen stimulation was higher in GLUT3 EGFRvIII CAR-T cells than in conv EGFRvIII CAR-T cells (FIGS. 30j and 30k, and FIGS. 37f and 37g). In addition, enhancement of effector function by stable GLUT3 expression was similarly observed in CAR-T cells targeting CD19, which is a different antigen (GLUT3 CD19 CAR-T cells). This indicates that enhancement of metabolic fitness by GLUT3 expression is universal in all CARs, regardless of the single-chain variable fragment (scFv) used or the target antigen (FIGS. 38a to 38c). Therefore, stable GLUT3 expression enables CAR-T cells to be activated in a TME with low glucose levels, although an exhausted phenotype is present.

Stable GLUT3 expression overactivates CAR-T cells and causes severe adverse effects.

Using an intracranial U-87 MGΔ xenograft model, the effects of stable GLUT3 expression in CAR-T cells on the antitumor effect and safety profile were further examined (FIG. 31a). GLUT3 EGFRvIII CAR-T cells were intravenously administered 5 days after intracranial tumor inoculation. As expected, GLUT3 EGFRvIII CAR-T cell treatment showed an enhanced antitumor effect (CR: 4/6) compared with conv EGFRvIII CAR-T cell treatment (CR: 2/6) (FIG. 31b). Nevertheless, GLUT3 EGFRvIII CAR-T cell therapy failed to improve survival because of a high incidence of treatment-related death (TRM) associated with worsening general condition and severe body weight loss (FIGS. 31c to 31e). Consistent with these findings, pathological analysis revealed overactivation of T cells in brain tissues of mice treated with GLUT3 EGFRvIII CAR-T cells, with diffuse infiltration into the brain parenchyma, particularly the ventricular wall (FIG. 31f). Furthermore, in lung tissues, diffuse alveolar damage, indicated by thickening of the alveolar wall accompanied by T cell infiltration, was detected (FIG. 31g). In fact, the frequency of effector memory T cells (CD45RA CCR7⁻⁻) was significantly higher in mice administered GLUT3 EGFRvIII CAR-T cells than in mice administered conv EGFRvIII CAR-T cells (FIG. 31h). Furthermore, gene expression assays revealed that gene sets related to cytokine production, T cell differentiation, and T cell activation were markedly upregulated in GLUT3 EGFRvIII CAR-T cells even on day 7 after stimulation (FIG. 31i). Thus, stable expression of GLUT3 by CAR-T cells causes dysregulation of the activation state of T cells due to excessive glucose uptake, indicating the need for delicate control of metabolic fitness.

On-demand expression of GLUT3 by antigen stimulation is achieved by a CAR-NFAT-GLUT3 construct.

In view of the severe toxicity caused by overactivation of GLUT3 EGFRvIII CAR-T cells, the present inventors examined whether the advantages of GLUT3 expression could be maximized and toxicity could be minimized by expressing GLUT3 in response to glucose demand, that is, at the time of T cell stimulation upon target antigen recognition in the TME. A construct in which SLC2A3 was placed downstream of an NFAT-binding domain was developed, thereby enabling on-demand GLUT3 expression (on-demand GLUT3 CAR) (FIG. 32a). Activation of T cells by antigen recognition induced nuclear translocation of NFAT and promoted expression of GLUT3. Target antigen recognition by T cells having the on-demand GLUT3 CAR construct (on-demand GLUT3 EGFRvIII CAR-T cells) induced expression of GLUT3. GLUT3 expression returned to the initial level 7 days after the last antigen stimulation (FIGS. 32b and 32c). In fact, glucose uptake and glycolysis were higher in on-demand GLUT3 EGFRvIII CAR-T cells than in conv EGFRvIII CAR-T cells (FIGS. 32d and 32e and FIG. 39a). In on-demand GLUT3 EGFRvIII CAR-T cells, metabolism shifted to a more energetic state (OCR^{high}ECAR^{high}) on day 3 and recovered to a level comparable to that of conv EGFRvIII CAR-T cells on day 7 after the last antigen stimulation (FIG. 33f). Cytokine production was significantly higher in on-demand GLUT3 EGFRvIII CAR-T cells than in conv EGFRvIII CAR-T cells at both low glucose (0.5 mM) and high glucose (10 mM) levels (FIGS. 33g and 33h and FIG. 39b). On-demand GLUT3 EGFRvIII CAR-T cells also showed increased cytotoxicity against U-87 MGΔ cells and U-251 MGΔ cells under both low-glucose and high-glucose conditions (FIG. 33i and FIG. 39c). Taken together, the on-demand GLUT3 CAR construct successfully improved metabolic fitness in an on-demand manner, and on-demand GLUT3 EGFRvIII CAR-T cells enhanced cytokine production and cytotoxicity under both low-glucose and high-glucose conditions.

On-demand GLUT3 expression enables sustained effector function of CAR-T cells.

Since on-demand GLUT3 EGFRvIII CAR-T cells with enhanced cytokine production and cytotoxicity were successfully developed, the present inventors decided to examine a comprehensive immunological profile, focusing on persistence, function, and molecular expression. First, cell proliferation after antigen stimulation was examined. On-demand GLUT3 EGFRvIII CAR-T cells continued to expand after antigen stimulation, whereas GLUT3 EGFRvIII CAR-T cells did not expand (FIGS. 40a and 40b). This failure of expansion by GLUT3 EGFRvIII CAR-T cells was mainly due to induction of apoptosis, and this apoptosis was caused by overactivation, known as activation-induced cell death (AICD), which is an important phenomenon for T cell homeostasis [Reference 22] (FIG. 34a). Gene expression analysis further confirmed that, under low-glucose conditions, expression of genes related to apoptosis was increased in GLUT3 EGFRvIII CAR-T cells compared with on-demand GLUT3 EGFRvIII CAR-T cells and conv EGFRvIII CAR-T cells (FIG. 34b). Furthermore, expression of apoptosis-related genes was closely correlated with expression of TNFα signaling genes, suggesting a role of TNFα in apoptosis of GLUT3 EGFRvIII CAR-T cells (FIG. 34c). Meanwhile, expression of T cell activation-related gene groups was increased in on-demand GLUT3 EGFRvIII CAR-T cells and GLUT3 EGFRvIII CAR-T cells compared with conv EGFRvIII CAR-T cells (FIG. 34b). Accordingly, on-demand GLUT3 EGFRvIII CAR-T cells maintained central memory (CD62L CCR7⁺⁺CD45RA⁻) and naive phenotypes (CD62L CCR7⁺⁺CD45RA⁺), whereas GLUT3 EGFRvIII CAR-T cells showed a terminally differentiated phenotype (CD62L CCR7⁻⁻CD45RA⁺) (FIG. 34d and FIG. 40c). Furthermore, expression of exhaustion-related molecules including PD-1 and Tim-3 was lower in on-demand GLUT3 EGFRvIII CAR-T cells than in GLUT3 EGFRvIII CAR-T cells and conv EGFRvIII CAR-T cells (FIG. 34e and FIG. 40d). Furthermore, comprehensive gene expression profiles of GLUT3 EGFRvIII CAR-T cells and on-demand GLUT3 EGFRvIII CAR-T cells were comparable on day 3 after stimulation, but were significantly different on day 7 after stimulation (FIG. 34f). RNA-seq analysis of each CAR-T cell population on day 7 after stimulation further confirmed that GLUT3 EGFRvIII CAR-T cells depended on glycolysis, whereas on-demand GLUT3 EGFRvIII CAR-T cells maintained oxidative phosphorylation and fatty acid oxidation, reflecting the activation state, namely the naive/stem cell memory and central memory phenotypes (FIG. 34g). These findings show that on-demand GLUT3 expression prevents overactivation of CAR-T cells and maintains functional CAR-T cells for a long period of time. From the above, particularly from the results of FIG. 34, it can be said that constitutively expressed GLUT3 enhances the antitumor effect (that is, effector function) of EGFRvIII CAR-T cells. It can also be said that it was demonstrated that on-demand GLUT3 expression not only enhances the effector function of EGFRvIII CAR-T cells, but also enhances prolongation of memory function and maintenance of a memory T cell phenotype. Furthermore, it can be concluded that adverse events such as weight loss and treatment-related death induced by constitutively expressed GLUT3 were prevented, and as a result, a remarkable improvement in survival, including cure, was achieved.

On-demand GLUT3 EGFRvIII CAR-T cells showed an increased antitumor effect without severe toxicity in an intracranial human GBM cell xenograft model.

Based on the superior phenotype of on-demand GLUT3 EGFRvIII CAR-T cells, the antitumor effect and safety profile in an intracranial human GBM cell xenograft model were examined (FIG. 35a). Compared with conv EGFRvIII CAR-T cells, on-demand GLUT3 EGFRvIII CAR-T cells and GLUT3 EGFRvIII CAR-T cells showed superior antitumor effects, and most mice achieved CR (5/10 CR with conv EGFRvIII CAR-T cells, 9/10 CR with GLUT3 EGFRvIII cells, and 9/10 CR with on-demand GLUT3 EGFRvIII CAR-T cells) (FIG. 35b and FIG. 41a). However, as observed in the previous experiment (FIGS. 32a to 32e), GLUT3 EGFRvIII CAR-T cells induced severe toxicity accompanied by marked body weight loss and failed to improve survival. In contrast, on-demand GLUT3 EGFRvIII CAR-T cells showed a significant survival-improving effect without causing severe toxicity or body weight loss (FIGS. 35c to 35e). Accordingly, tumor rechallenge was also suppressed only in mice administered on-demand GLUT3 EGFRvIII CAR-T cells (FIG. 35b). Accordingly, on day 14 after CAR-T cell injection, high frequencies of naive/stem cell memory subsets and central memory subsets were detected in the peripheral blood of mice treated with on-demand GLUT3 EGFRvIII CAR-T cells, whereas effector memory subsets were more abundant in mice treated with GLUT3 EGFRvIII CAR-T cells (FIG. 35f). This increase in the antitumor effect induced by on-demand GLUT3 EGFRvIII CAR-T cells was similarly observed in another xenograft model (U-251 MGΔ); compared with mice in the other treatment groups, mice treated with on-demand GLUT3 EGFRvIII CAR-T cells showed superior survival without severe toxicity (FIG. 35g and FIGS. 41b and 41c). Cytokine levels (TNFα and GM-CSF) remained continuously high in the serum of mice administered GLUT3 EGFRvIII CAR-T cells even on day 14, when the tumor had already been rejected, and the rate of cytokine reduction was significantly lower in mice administered GLUT3 EGFRvIII CAR-T cells than in mice administered conv EGFRvIII CAR-T cells or on-demand EGFRvIII CAR-T cells (FIGS. 35h and 35i). Furthermore, levels of cytokines (IL-5, IP-10, and MDC) reported to be involved in development of cytokine release syndrome (CRS) were higher in the serum of mice treated with GLUT3 EGFRvIII CAR-T cells than in the serum of mice treated with on-demand GLUT3 EGFRvIII CAR-T cells (FIG. 41d). Overall, on-demand GLUT3 expression by CAR-T cells is a promising strategy for achieving sustained antitumor effects in GBM without severe toxicity.

### Discussion

Enhancing antitumor effects by improving metabolic fitness of CAR-T cells in the TME is a new concept for accelerating clinical application of CAR-T cell therapy to refractory solid cancers. Some clinical trials of CAR-T cells against GBM have failed to show clinical utility, but immunological monitoring in these clinical trials revealed that abundant CAR-T cells were detected in the TME [References 3 to 5]. Considering the presence of CAR-T cells in the TME, antigen loss and migration failure are excluded as causes of failure, and it can be assumed that specific conditions in the TME of GBM are involved in inducing dysfunction of CAR-T cells. Since effector T cells undergo glycolysis for survival and activation, glucose is an essential nutrient [Reference 19]. Therefore, given that a markedly low-glucose environment is detected in the TME of GBM, low glucose levels in the TME of GBM must be a major metabolic stress for CAR-T cells. Indeed, conv EGFRvIII CAR-T cells are in a dysfunctional state (low cytokine production and high expression of exhaustion markers). Furthermore, because PD-1 signaling disrupts PI3K/Akt/mTOR signaling and the glycolytic pathway is further impaired, effector T cells may enter a negative metabolic cycle [Reference 20].

Considering that nervous system cells such as neurons and glial cells, and GBM, particularly GBM expressing EGFRvIII, satisfy their high glucose demand using GLUT3, which is a high-affinity glucose transporter [References 17, 18, and 23], causing CAR-T cells to highly express GLUT3 is likely to enable them to overcome nutrient competition. Indeed, the GLUT3 EGFRvIII CAR construct developed by the present inventors enhanced glucose uptake and effector functions such as cytokine production and cytotoxicity in GLUT3 EGFRvIII CAR-T cells. Unexpectedly, however, GLUT3 EGFRvIII CAR-T cells underwent apoptosis approximately 24 hours after antigen stimulation, and mice administered GLUT3 EGFRvIII CAR-T cells died of TRM. Comprehensive gene expression assays showed that GLUT3 EGFRvIII CAR-T cells exhibited a distinct gene expression profile in which gene sets of the TNFα signaling pathway were upregulated. This was consistent with AICD caused by overactivation of GLUT3 EGFRvIII CAR-T cells due to continuous glucose influx via GLUT3, because TNFα is an important mediator of AICD, as is the FAS-FASL pathway [References 24 and 25]. TRM, in which severe inflammation occurred in lung and brain tissues and body weight decreased, was also associated with an increase in inflammatory cytokines including TNFα, and mimicked CRS observed in cancer patients treated with CAR-T cells. CRS has been reported to be induced by IFN-γ, TNFα, and GM-CSF produced by activated CAR-T cells [Reference 26]. Therefore, in mice administered GLUT3 EGFRvIII CAR-T cells, an increase in cytokine production (IL-5, IP-10, and MDC) associated with the severity of CRS was also observed. Since the present inventors used NOD.Cg-Prkdc^{scid} Il2rg^{tm1lWjl} /SzJ (NSG) mice lacking innate immune cells including macrophages and adaptive immune cells as an in vivo animal model, inflammatory cytokines produced by macrophages could not be evaluated.

To overcome the complexity of stable GLUT3 expression by CAR-T cells, the present inventors developed an on-demand GLUT3 expression system in which CAR-T cells can express GLUT3 and take up glucose on demand according to glucose demand. This inducible GLUT3 expression was achieved by nuclear translocation of NFAT, which is a downstream signal of antigen stimulation. Nuclear translocation of NFAT occurs immediately after TCR/CAR signal transduction and peaks at 30 minutes [Reference 29]. NFAT binds to a conserved DNA-binding domain (NFAT-binding domain) and promotes a panel of genes necessary for activation of T cells. These genes include genes that induce an energy shift from oxidative phosphorylation to aerobic glycolysis in order to meet increasing energy demand [References 30 and 31]. Thus, GLUT3 expression is induced upon encounter with an antigen in the TME, and the expression kinetics of GLUT3 are comparable to those of NFAT. In addition, stable overexpression of GLUT3 in anti-CD19 CAR-T cells is not beneficial in a hematologic malignancy model [Reference 32]. Since CAR-T cells pass through various glucose conditions, such as a high-glucose environment in peripheral blood, before reaching the metabolically harsh and low-glucose TME of GBM, it is desirable and necessary to express GLUT3 on demand upon antigen exposure.

Furthermore, on-demand GLUT3 expression by CAR-T cells provides an advantageous effect on formation of memory cells through improvement of metabolic fitness, and produces a sustained antitumor effect with a remarkable survival benefit in a preclinical model. Furthermore, on-demand GLUT3 expression regulated sustained production of inflammatory cytokines such as TNFα, which was induced by stable GLUT3 expression, and significantly reduced TRM. In a clinical trial in which bicistronic CAR-T cells targeting EGFR and interleukin 13 receptor subunit α2 (IL-13Rα2) were intrathecally administered to patients with GBM, TRM was observed in all patients (n=6), and all patients (n=6) experienced early onset of immune effector cell-associated neurotoxicity syndrome (ICANs), requiring administration of high-dose dexamethasone and anakinra (anti-IL-1R antibody). This indicates that CAR-T cell toxicities such as CRS and ICANs are major concerns. In addition, high-dose steroids may reduce the antitumor effect of CAR-T cells [References 33 and 34]. Therefore, on-demand GLUT3 expression may be an ideal strategy for achieving both a sustained antitumor effect and a favorable safety profile. These findings shed light on the importance of the timing and persistence of immune cell infiltration.

The present inventors succeeded in achieving enhancement and persistence of antitumor activity without systemic adverse events caused by CAR-T cell therapy, using an on-demand system that operates upon antigen stimulation. Considering that a target molecule is ideally induced upon antigen recognition in the TME, it can be inferred that this on-demand system can be extended to other molecules, for example, other metabolism-related molecules (including glutamine transporters, fatty acid-metabolizing enzymes, and the like), cytokines, chemokines, and the like. For example, IL-12 was introduced as a promising antitumor cytokine based on animal experiments in clinical studies [References 35 to 37]. However, severe side effects have hindered its clinical application [References 38 and 39]. Supplying IL-12 to the TME on demand may overcome side effects and maximize the antitumor effect.

In summary, on-demand GLUT3 expression was induced by antigen stimulation. A novel CAR-T cell construct was developed. On-demand GLUT3 CAR-T cells showed sustained antitumor effects with ideal metabolic fitness, without exhibiting severe toxicity. Since metabolic supplementation requires delicate control, an on-demand approach is a promising strategy. Overall, this study provides deep implications for using metabolic fitness in immunotherapy for GBM and other solid cancers.

### (Example 6: Example of TCR)

Similar experiments are performed in an example in which TCR is introduced instead of CAR. For example, TCR can also be introduced into fractions such as T_{eff}, Tnv, Tcm, Tem, and Temra.

In the present Example, TCR (CAR, constitutive GLUT3 CAR, or on-demand GLUT3 CAR) is introduced into any purified and enriched T cell memory fraction. In another experiment, TCR-T cells enriched with any T cell memory fraction are induced under culture conditions.

Various in vitro and in vivo evaluations shown in Examples 1 to 5 are performed.

### (Example 7: Prevention of bacterial infection, prevention of reinfection, and treatment of bacterial infection)

In the present Example, prevention of bacterial infection, prevention of reinfection, and/or treatment of bacterial infection are verified.

### (Method)

For prevention against bacterial infection (for example, bacterial conjunctivitis), the T cells of the present disclosure modified with an infection-causing antigen of the bacterial infection are administered to a subject not having the bacterial infection.

For prevention of recurrence and/or treatment of bacterial infection (for example, bacterial conjunctivitis), the T cells of the present disclosure are administered to a subject who had and/or has the bacterial infection.

After administration, prognostic follow-up for bacterial infection in the subject is performed by a technique commonly used in the art.

### (Example 8: Prevention of parasitic infection, prevention of reinfection, and treatment of parasitic infection)

In the present Example, prevention of parasitic infection, prevention of reinfection, and/or treatment of parasitic infection are verified.

### (Method)

For prevention against parasitic infection (for example, Acanthamoeba keratitis), the T cells of the present disclosure modified with an infection-causing antigen of the parasitic infection are administered to a subject not having the parasitic infection.

For prevention of recurrence and/or treatment of parasitic infection (for example, Acanthamoeba keratitis), the T cells of the present disclosure are administered to a subject who had and/or has the parasitic infection.

After administration, prognosis follow-up for parasitic infection in the subject is performed by a technique commonly used in the art.

### (Example 9: Prevention of viral infection, prevention of onset, prevention of reinfection, and treatment of viral infection)

In the present Example, prevention of viral infection, prevention of onset, prevention of reinfection, and/or treatment of viral infection are verified.

### (Method)

For prevention against viral infection (for example, tuberculosis, malaria, yellow fever virus, smallpox virus, vaccinia, measles/rubella, polio, epidemic parotitis (mumps)/MUMPS, rotavirus infection, varicella (chickenpox), yellow fever, Ebola, West Nile fever, Hib infection, pneumococcal infection, pertussis, Japanese encephalitis, meningococcal infection, salmonella infection, pathogenic E. coli, toxoplasma, zika virus, herpesvirus type 1, EBV/Epstein-Barr virus (herpesvirus type 4), CMV/cytomegalovirus (herpesvirus type 5), influenza (virus), MARS, rabies, diphtheria, and the like, including but not limited thereto), the T cells of the present disclosure modified with an infection-causing antigen of the viral infection are administered to a subject not having the viral infection.

For prevention of recurrence and/or treatment of the above viral infection, the T cells of the present disclosure are administered to a subject who had and/or has the viral infection.

After administration, prognosis follow-up for viral infection in the subject is performed by a technique commonly used in the art.

### (Example 10: Prevention of allergy, prevention of recurrence, and treatment of allergy)

In the present Example, prevention of allergy, prevention of recurrence, and/or treatment of allergy are verified.

### (Method)

For prevention against allergy (for example, allergic conjunctivitis), the T cells of the present disclosure modified with an allergy infection-causing antigen are administered to a subject not having a causative factor antigen of the allergy.

For prevention of recurrence and/or treatment of allergy (for example, allergic conjunctivitis), the T cells of the present disclosure are administered to a subject who had and/or has a causative factor antigen of the allergy.

After administration, prognosis follow-up for allergy in the subject is performed by a technique commonly used in the art.

### (Example 11: Prevention of autoimmune disease, prevention of recurrence, and treatment of autoimmune disease)

In the present Example, prevention of autoimmune disease, prevention of recurrence, and/or treatment of autoimmune disease are verified.

### (Method)

For prevention against autoimmune disease (for example, autoimmune uveitis), the T cells of the present disclosure modified with an autoimmune disease infection-causing antigen are administered to a subject not having a causative factor antigen of allergy.

For prevention of recurrence and/or treatment of autoimmune disease (for example, autoimmune uveitis), the T cells of the present disclosure are administered to a subject who had and/or has a causative factor antigen of the autoimmune disease.

After administration, follow-up observation of the prognosis of the autoimmune disease in the subject is performed by a technique commonly used in the art.

### References

1. Stupp R, Taillibert S, Kanner A, et al (2017) Effect of Tumor-Treating Fields Plus Maintenance Temozolomide vs Maintenance Temozolomide Alone on Survival in Patients With Glioblastoma: A Randomized Clinical Trial. Jama; 318(23):2306-16.
2. Stupp R, Mason WP, van den Bent MJ, et al (2005) Radiotherapy plus concomitant and adjuvant temozolomide for glioblastoma. N Engl J Med; 352(10):987-96.
3. O'Rourke DM, Nasrallah MP, Desai A, et al (2017) A single dose of peripherally infused EGFRvIII-directed CAR T cells mediates antigen loss and induces adaptive resistance in patients with recurrent glioblastoma. Sci Transl Med; 9(399).
4. Bagley SJ, Logun M, Fraietta JA, et al (2024) Intrathecal bivalent CAR T cells targeting EGFR and IL13Rα2 in recurrent glioblastoma: phase 1 trial interim results. Nat Med; 30(5):1320-9.
5. Brown CE, Hibbard JC, Alizadeh D, et al (2024) Locoregional delivery of IL-13Rα2-targeting CAR-T cells in recurrent high-grade glioma: a phase 1 trial. Nat Med; 30(4):1001-12.
6. Bagley SJ, Binder ZA, Lamrani L, et al (2024) Repeated peripheral infusions of anti-EGFRvIII CAR T cells in combination with pembrolizumab show no efficacy in glioblastoma: a phase 1 trial. Nat Cancer; 5(3):517-31.
7. Choi BD, Gerstner ER, Frigault MJ, et al (2024) Intraventricular CARv3-TEAM-E T Cells in Recurrent Glioblastoma. N Engl J Med; 390(14):1290-8.
8. Maude SL, Frey N, Shaw PA, et al (2014) Chimeric antigen receptor T cells for sustained remissions in leukemia. N Engl J Med; 371(16):1507-17.
9. Grupp SA, Kalos M, Barrett D, et al (2013) Chimeric antigen receptor-modified T cells for acute lymphoid leukemia. N Engl J Med; 368(16):1509-18.
10. Porter DL, Levine BL, Kalos M, et al (2011) Chimeric antigen receptor-modified T cells in chronic lymphoid leukemia. N Engl J Med; 365(8):725-33.
11. Mergenthaler P, Lindauer U, Dienel GA, Meisel A (2013) Sugar for the brain: the role of glucose in physiological and pathological brain function. Trends Neurosci; 36(10):587-97.
12. Sheraj I, Guray NT, Banerjee S (2021) A pan-cancer transcriptomic study showing tumor specific alterations in central metabolism. Sci Rep; 11(1):13637.
13. Oudard S, Arvelo F, Miccoli L, et al (1996) High glycolysis in gliomas despite low hexokinase transcription and activity correlated to chromosome 10 loss. Br J Cancer; 74(6):839-45.
14. Santra A, Kumar R, Sharma P, et al (2012) F-18 FDG PET-CT in patients with recurrent glioma: comparison with contrast enhanced MRI. Eur J Radiol; 81(3):508-13.
15. Maher EA, Marin-Valencia I, Bachoo RM, et al (2012) Metabolism of [U-13 C]glucose in human brain tumors in vivo. NMR Biomed; 25(11):1234-44.
16. Flavahan WA, Wu Q, Hitomi M, et al (2013) Brain tumor initiating cells adapt to restricted nutrition through preferential glucose uptake. Nat Neurosci; 16(10):1373-82.
17. Vannucci SJ, Maher F, Simpson IA (1997) Glucose transporter proteins in brain: delivery of glucose to neurons and glia. Glia; 21(1):2-21.
18. Chen S, Yang L, Li Z, et al (2021) EGFR/EGFRvIII partly regulates the tumourigenesis of glioblastoma through the SOX9-GLUT3 axis. Am J Transl Res; 13(6):6055-65.
19. Ho PC, Bihuniak JD, Macintyre AN, et al (2015) Phosphoenolpyruvate Is a Metabolic Checkpoint of Anti-tumor T Cell Responses. Cell; 162(6):1217-28.
20. Chang CH, Qiu J, O'Sullivan D, et al (2015) Metabolic Competition in the Tumor Microenvironment Is a Driver of Cancer Progression. Cell; 162(6):1229-41.
21. Peng JJ, Wang L, Li Z, et al (2023) Metabolic challenges and interventions in CAR T cell therapy. Sci Immunol; 8(82):eabq3016.
22. Green DR, Droin N, Pinkoski M (2003) Activation-induced cell death in T cells. Immunol Rev; 193:70-81.
23. Schmidt S, Hommel A, Gawlik V, et al (2009) Essential role of glucose transporter GLUT3 for post-implantation embryonic development. J Endocrinol; 200(1):23-33.
24. Otano I, Alvarez M, Minute L, et al (2020) Human CD8 T cells are susceptible to TNF-mediated activation-induced cell death. Theranostics; 10(10):4481-9.
25. Zhang M, Wang J, Jia L, et al (2017) Transmembrane TNF-α promotes activation-induced cell death by forward and reverse signaling. Oncotarget; 8(38):63799-812.
26. Morris EC, Neelapu SS, Giavridis T, Sadelain M (2022) Cytokine release syndrome and associated neurotoxicity in cancer immunotherapy. Nat Rev Immunol; 22(2):85-96.
27. Guo XJ, Thomas PG (2017) New fronts emerge in the influenza cytokine storm. Semin Immunopathol; 39(5):541-50.
28. Mehta P, McAuley DF, Brown M, et al (2020) COVID-19: consider cytokine storm syndromes and immunosuppression. Lancet; 395(10229):1033-4.
29. Maguire O, Tornatore KM, O'Loughlin KL, et al (2013) Nuclear translocation of nuclear factor of activated T cells (NFAT) as a quantitative pharmacodynamic parameter for tacrolimus. Cytometry A; 83(12):1096-104.
30. Mognol GP, de Araujo-Souza PS, Robbs BK, et al (2012) Transcriptional regulation of the c-Myc promoter by NFAT1 involves negative and positive NFAT-responsive elements. Cell Cycle; 11(5):1014-28.
31. Siska PJ, Rathmell JC (2015) T cell metabolic fitness in antitumor immunity. Trends Immunol; 36(4):257-64.
32. Shi Y, Kotchetkov IS, Dobrin A, et al (2024) GLUT1 overexpression enhances CAR T cell metabolic fitness and anti-tumor efficacy. Mol Ther; 32(7):2393-405.
33. Liu S, Deng B, Yin Z, et al (2020) Corticosteroids do not influence the efficacy and kinetics of CAR-T cells for B-cell acute lymphoblastic leukemia. Blood Cancer J; 10(2):15.
34. Strati P, Ahmed S, Furqan F, et al (2021) Prognostic impact of corticosteroids on efficacy of chimeric antigen receptor T-cell therapy in large B-cell lymphoma. Blood; 137(23):3272-6.
35. Brunda MJ, Luistro L, Warrier RR, et al (1993) Antitumor and antimetastatic activity of interleukin 12 against murine tumors. J Exp Med; 178(4):1223-30.
36. Nguyen KG, Vrabel MR, Mantooth SM, et al (2020) Localized Interleukin-12 for Cancer Immunotherapy. Front Immunol; 11:575597.
37. Noguchi Y, Jungbluth A, Richards EC, Old LJ (1996) Effect of interleukin 12 on tumor induction by 3-methylcholanthrene. Proc Natl Acad Sci U S A; 93(21):11798-801.
38. Hurteau JA, Blessing JA, DeCesare SL, Creasman WT (2001) Evaluation of recombinant human interleukin-12 in patients with recurrent or refractory ovarian cancer: a gynecologic oncology group study. Gynecol Oncol; 82(1):7-10.
39. Motzer RJ, Rakhit A, Thompson JA, et al (2001) Randomized multicenter phase II trial of subcutaneous recombinant human interleukin-12 versus interferon-alpha 2a for patients with advanced renal cell carcinoma. J Interferon Cytokine Res; 21(4):257-63.
40. Kumagai S, Togashi Y, Kamada T, et al (2020) The PD-1 expression balance between effector and regulatory T cells predicts the clinical efficacy of PD-1 blockade therapies. Nat Immunol; 21(11):1346-58.
41. Kumagai S, Koyama S, Itahashi K, et al (2022) Lactic acid promotes PD-1 expression in regulatory T cells in highly glycolytic tumor microenvironments. Cancer Cell; 40(2):201-18.e9.
42. Tada Y, Togashi Y, Kotani D, et al (2018) Targeting VEGFR2 with Ramucirumab strongly impacts effector/ activated regulatory T cells and CD8+ T cells in the tumor microenvironment. J Immunother Cancer; 6(1):106.
43. Tanegashima T, Togashi Y, Azuma K, et al (2019) Immune Suppression by PD-L2 against Spontaneous and Treatment-Related Antitumor Immunity. Clin Cancer Res; 25(15):4808-19.
44. Dobin A, Gingeras TR (2015) Mapping RNA-seq Reads with STAR. Curr Protoc Bioinformatics; 51:11.4.1-.4.9.
45. Etoh K, Nakao M (2023) A web-based integrative transcriptome analysis, RNAseqChef, uncovers the cell/tissue type-dependent action of sulforaphane. J Biol Chem; 299(6):104810.

### (Note)

Although the present disclosure has been illustrated using preferred embodiments of the present disclosure as described above, it is understood that the scope of the present disclosure should be interpreted only by the claims. It is understood that the patents, patent applications, and other documents cited in the present specification are to be incorporated by reference in the present specification in the same manner as the contents are specifically described in the present specification. The present application claims priority to Japanese Patent Application No. 2024-001533 filed with the Japan Patent Office on January 9, 2024, the entire contents of which are incorporated herein by reference.

### Industrial Applicability

According to the present disclosure, it is possible to provide cells that are competitive with cancer cells even in a glucose-depleted environment such as tumor microenvironment (TME) conditions, without becoming starved or exhausted. Therefore, a strategy for improving the effects of cell-based immunotherapy can be developed, and application in the medical field is expected.

## Claims

1. A T cell with enhanced glucose uptake ability, wherein expression of a glucose transporter is regulated in the T cell in response to a change in tumor microenvironment conditions.

2. The T cell according to claim 1, wherein the tumor microenvironment conditions comprise a degree of transmission of a TCR-related signal.

3. The T cell according to claim 2, wherein the TCR-related signal is mediated by a molecule selected from the group consisting of a TCR signal or a part thereof, and a chimeric signal of a TCR signal and another antigen (also referred to as a CAR signal).

4. The T cell according to claim 2 or 3, wherein the TCR-related signal is mediated by a molecule comprising a TCR signal domain.

5. The T cell according to any one of claims 1 to 4, wherein the change in tumor microenvironment conditions comprises presentation of a tumor antigen and/or a costimulatory ligand, and/or a change in cytokine conditions.

6. The T cell according to any one of claims 2 to 5, wherein the degree of transmission of the TCR-related signal comprises a degree of TCR stimulation.

7. The T cell according to any one of claims 2 to 6, wherein the degree of transmission of the TCR-related signal comprises at least one selected from the group consisting of a degree of TCR stimulation, TCR co-stimulation, and a humoral factor.

8. The T cell according to any one of claims 2 to 7, wherein the degree of transmission of the TCR-related signal comprises a degree of TCR stimulation, and at least one selected from the group consisting of TCR co-stimulation and a humoral factor.

9. The T cell according to any one of claims 2 to 8, wherein the degree of transmission of the TCR-related signal comprises a degree of TCR stimulation, TCR co-stimulation, and a humoral factor.

10. The T cell according to any one of claims 6 to 9, wherein the TCR stimulation is selected from the group consisting of NFAT, NF-κB, and the STAT transcription factor family, which translocate into the nucleus in association with activation of T cells.

11. The T cell according to any one of claims 1 to 10, wherein the tumor microenvironment conditions comprise a degree of oxygen concentration.

12. A T cell comprising a nucleic acid sequence encoding a glucose transporter, the nucleic acid sequence being operably linked to a tumor microenvironment-sensing element that senses a change in tumor microenvironment conditions.

13. The T cell according to claim 12, wherein the tumor microenvironment-sensing element comprises:
(i) an inducible activation motif that is activated in response to a change in tumor microenvironment conditions; and
(ii) a nucleic acid sequence encoding a degradation factor for the glucose transporter that is inactivated in response to the change in the tumor microenvironment conditions.

14. The T cell according to claim 12 or 13, wherein the tumor microenvironment-sensing element comprises:
(i) an inducible activation motif to which a nuclear translocation factor that translocates into the nucleus in response to activation of T cells binds;
(ii) a nuclear translocation factor-binding inducible activation motif to which a nuclear translocation factor that translocates into the nucleus in response to a decrease in oxygen concentration binds; and
(iii) a nucleic acid sequence encoding a glucose transporter-degrading enzyme whose activity of degrading a glucose transporter is inactivated in response to the decrease in oxygen concentration.

15. The T cell according to any one of claims 1 to 14, wherein the nucleic acid sequence encoding the glucose transporter comprises a construct operably linked to an inducible activation motif that is activated in response to a change in tumor microenvironment conditions and/or a nucleic acid sequence encoding a degradation factor for the glucose transporter that is inactivated in response to the change in the tumor microenvironment conditions.

16. The T cell according to any one of claims 1 to 15, wherein the nucleic acid sequence encoding the glucose transporter comprises a construct operably linked to a nuclear translocation factor-binding inducible activation motif to which a nuclear translocation factor that translocates into the nucleus in response to activation of T cells binds.

17. The T cell according to any one of claims 1 to 16, wherein the nuclear translocation factor is selected from the group consisting of NFAT, NF-κB, and the STAT transcription factor family.

18. The T cell according to claim 16 or 17, wherein the nuclear translocation factor-binding inducible activation motif is selected from the group consisting of an NFAT-binding domain, a κB motif (GGGACTTTCC) (SEQ ID NO: 1), a STAT-binding sequence (TTCNNNGAA), and modified sequences thereof.

19. The T cell according to any one of claims 16 to 18, wherein a combination of the nuclear translocation factor and the nuclear translocation factor-binding inducible activation motif comprises:
(1) a combination of NFAT and an NFAT-binding domain;
(2) a combination of NF-κB and a κB motif (GGGACTTTCC) (SEQ ID NO: 1); or
(3) a combination of the STAT transcription factor family and a STAT-binding sequence (TTCNNNGAA).

20. The T cell according to any one of claims 1 to 19, wherein the nucleic acid sequence encoding the glucose transporter comprises a construct operably linked to a nuclear translocation factor-binding inducible activation motif to which a nuclear translocation factor that translocates into the nucleus in response to a decrease in oxygen concentration binds.

21. The T cell according to any one of claims 16 to 20, wherein the nuclear translocation factor is selected from the group consisting of HIF-1.

22. The T cell according to any one of claims 13 to 21, wherein the nuclear translocation factor-binding inducible activation motif is selected from the group consisting of HIF-RE.

23. The T cell according to any one of claims 13 to 22, wherein a combination of the nuclear translocation factor and the nuclear translocation factor-binding inducible activation motif is selected from the group consisting of:
(1) a combination of HIF-1 and HIF-RE.

24. The T cell according to any one of claims 1 to 23, wherein the nucleic acid sequence encoding the glucose transporter comprises a construct operably linked to a nucleic acid sequence encoding a factor whose activity of degrading the glucose transporter is inactivated in response to a decrease in oxygen concentration.

25. The T cell according to any one of claims 15 to 24, wherein the factor to be inactivated is selected from the group consisting of an oxygen-dependent degradation factor (ODD).

26. The T cell according to any one of claims 1 to 25, wherein the T cell comprises a chimeric antigen receptor (CAR).

27. The T cell according to any one of claims 1 to 26, wherein the T cell has an effector function.

28. The T cell according to any one of claims 1 to 26, wherein the T cell is a precursor cell of an effector T cell (T_{eff}).

29. The T cell according to claim 28, wherein memory function of the precursor cell of the effector T cell is prolonged.

30. The T cell according to claim 28 or 29, wherein a memory T cell phenotype of the precursor cell of the effector T cell is maintained.

31. The T cell according to any one of claims 28 to 30, wherein the effector T cell is activated or activity of the effector T cell is maintained, and memory function of the precursor cell of the effector T cell is prolonged or a memory T cell phenotype of the precursor cell of the effector T cell is maintained.

32. The T cell according to claim 1 or any one of claims 26 to 31, wherein the expression and/or enhancement is achieved specifically in a tumor environment.

33. The T cell according to any one of claims 26 to 32, wherein the CAR is expressed in the T cell.

34. The T cell according to any one of claims 1 to 33, wherein the glucose transporter comprises GLUT1, GLUT2, GLUT3, and GLUT4.

35. The T cell according to any one of claims 1 to 34, wherein the glucose transporter is GLUT3.

36. The T cell according to any one of claims 1 to 35, wherein the T cell is a human T cell.

37. A cell population comprising T cells with enhanced glucose uptake ability in response to demand (on demand).

38. A cell population comprising the T cell according to any one of claims 1 to 36.

39. The cell population according to claim 37 or 38, wherein the T cells comprise cells having a property of exhibiting an effector function when introduced into a body.

40. The cell population according to any one of claims 37 to 39, wherein the T cell comprises a chimeric antigen receptor (CAR).

41. The cell population according to any one of claims 37 to 40, wherein the T cells are modified so that expression of a glucose transporter is regulated in the T cells in response to a change in tumor microenvironment conditions, and/or comprise a nucleic acid sequence encoding a glucose transporter, the nucleic acid sequence being operably linked to a tumor microenvironment-sensing element that senses a change in tumor microenvironment conditions.

42. The cell population according to any one of claims 37 to 41, wherein the T cells have an effector function.

43. The cell population according to any one of claims 37 to 42, wherein the cell population comprises T_{eff}.

44. The cell population according to any one of claims 37 to 43, wherein the cell population comprises precursor cells of T_{eff}.

45. The cell population according to any one of claims 38 to 44, wherein the CAR is expressed in the T cells.

46. The cell population according to any one of claims 37 to 45, wherein the glucose transporter comprises GLUT1, GLUT2, GLUT3, and GLUT4.

47. The cell population according to any one of claims 37 to 46, wherein the glucose transporter is GLUT3.

48. The cell population according to any one of claims 37 to 47, wherein the T cells are human T cells.

49. A pharmaceutical composition comprising the T cell according to any one of claims 1 to 36 or the cell population according to any one of claims 37 to 48.

50. The pharmaceutical composition according to claim 49, wherein the pharmaceutical composition is for prevention or treatment of cancer.

51. The pharmaceutical composition according to claim 49 or 50, wherein the pharmaceutical composition is for curing cancer.

52. The pharmaceutical composition according to any one of claims 49 to 51, wherein the pharmaceutical composition is for prevention of cancer metastasis or prevention of cancer recurrence.

53. The pharmaceutical composition according to any one of claims 49 to 52, wherein the pharmaceutical composition is for preventing or treating cancer so that the cancer does not recur.

54. The T cell according to any one of claims 1 to 36 or the cell population according to any one of claims 37 to 48, wherein the T cell or cell population is for use as a medicament.

55. A method for preventing or treating a disease in a subject, the method comprising administering an effective amount of the T cell according to any one of claims 1 to 36 or the cell population according to any one of claims 37 to 48 to the subject.

56. Use of the T cell according to any one of claims 1 to 36 or the cell population according to any one of claims 37 to 48 for producing a medicament.
